Europäisches Patentamt

European Patent Office

Office européen des brevets

(19)

(11) EP 0 282 899 B1

(12) EUROPÄISCHE PATENTSCHRIFT

(45) Veröffentlichungstag und Bekanntmachung des Hinweises auf die Patenterteilung:
10.01.1996 Patentblatt 1996/02

(51) Int Cl.⁶: **C12N 15/53**, C12N 9/02, C12N 15/62

(21) Anmeldenummer: 88103754.3

(22) Anmeldetag: 10.03.1988

(54) **Humane Mangan-Superoxiddismutase (hMn-SOD)**

Human manganese superoxide dismutase (hMn-SoD)

La manganèse-superoxyde-dismutase humaine (hMn-SOD)

(84) Benannte Vertragsstaaten:
AT BE CH DE ES FR GB GR IT LI LU NL SE

(30) Priorität: 14.03.1987 DE 3708306
26.05.1987 DE 3717695
10.07.1987 DE 3722884
24.12.1987 DE 3744038

(43) Veröffentlichungstag der Anmeldung:
21.09.1988 Patentblatt 1988/38

(60) Teilanmeldung: 95107460.8

(73) Patentinhaber:
BOEHRINGER INGELHEIM INTERNATIONAL GmbH
D-55216 Ingelheim am Rhein (DE)

(72) Erfinder:
• Heckl, Konrad, Dr.
D-6900 Heidelberg (DE)
• Spevak, Walter, Dr.
A-2000 Stockerau (AT)
• Ostermann, Elinborg, Dr.
A-1140 Wien (AT)
• Zöphel, Andreas, Dr.
A-3040 Neulengbach (AT)

• Krystek, Edeltraud, Dr.
A-1020 Wien (AT)
• Maurer-Fogy, Ingrid, Dr.
A-1238 Wien (AT)
• Wiche-Castanon, Maria-Josefa, Dr.
A-1130 Wien (AT)
• Stratowa, Christian, Dr.
A-1010 Wien (AT)
• Hauptmann, Rudolf, Dr.
A-2483 Ebreichsdorf (AT)

(56) Entgegenhaltungen:
EP-A- 462 836          EP-A- 0 138 111
WO-A-87/01387          BE-A- 905 796
DE-A- 2 417 508        GB-A- 2 183 658

• THE JOURNAL OF BIOLOGICAL CHEMISTRY, Band 259, Nr. 20, 25. Oktober 1984, Seiten 12595-12601, The American Society of Biological Chemists, Inc., Bethesda, Md, US; D. BARRA et al.: "The primary structure of human liver manganese superoxide dismutase"
• NUCLEIC ACIDS RESEARCH, Band 16, Nr. 13, 11. Juli 1988, Seite 6224, IRL Press Ltd, Oxford, GB; K. HECKL: "Isolation of cDNAs encoding human manganese superoxide dismutase"
• SCIENCE, 219: 620-625(1983)
• BIOTECHNOLOGY, 6: 930-935(1988)
• GENE, 56: 53-59 (1987)

**Beschreibung**

Gegenstand der vorliegenden Erfindung ist ein gentechnologisches Verfahren zur Herstellung von Human Mn-Superoxiddismutase (hMn-SOD), die DNA-Sequenzen, die für dieses Enzym codieren, geeignete Vektoren, die diese DNA-Sequenzen enthalten und Wirtszellen, die diese DNA-Sequenzen exprimieren können, sowie das Enzym hMn-SOD selbst. Vorschläge zur Verwendung dieses Enzyms werden außerdem beschrieben.

Als Folge verschiedener biochemischer Prozesse in biologischen Systemen (z.B. Redoxprozesse in der Atmungskette, Oxydationen im Cytoplasma) werden bekannterweise laufend $O_2^-$-Radikale gebildet, die hochcytotoxisch sind und zu Gewebezerstörungen führen können. Bei pathologischen Situationen, z.B. im Verlauf rheumatisch bedingter Erkrankungen, werden Degradationen von Collagen und Synovialflüssigkeit durch solche Radikale diskutiert (Pasquier, C. et al., Inflammation 8, 27-32, 1984). Eukaryotische Zellen enthalten zwei Formen von Superoxiddismutasen, von denen die eine vornehmlich in Cytosol (Cu/Zn-SOD) und die andere hauptsächlich in den Mitochondrien (Mn-SOD) vorliegen. In Lebermitochondrien wurde gefunden, daß Mn-Enzym in der Matrix einschließlich der inneren Membran lokalisiert ist, obwohl die Mn-SOD auch im Cytosol der Leberzellen nachgewiesen wurde (Mc Cord J.M. et al., In: Superoxide and Superoxide Dismutases (A.M. Michelson, J.M. Mc Cord, I. Fridovich, eds.) Academic Press, N.Y., 129-138, 1977).

In Prokaryoten existiert neben einer Mn-SOD noch eine Fe-SOD. Letztere wurde auch in Algen und Protozoen sowie in einigen Pflanzenspezies nachgewiesen (Bridges, S.M., Salin, M.L., Plant Physiol. 68, 275-278, 1981). Diese hochaktiven Enzyme katalysieren die Disproportionierung $O_2^- + O_2^- + 2H^+ \rightarrow H_2O_2 + O_2$ und verhindern durch diese Dismutation der Superoxidradikale deren Anreicherung und somit deren zellschädigende Wirkung. Neben dem endoplasmatischen Retikulum der Leber können die mitochondrialen Membranen als einer der wichtigsten Orte der $O_2^-$ Entstehung in tierischen Zellen angesehen werden, sodaß es nicht verwundert, daß Mitochondrien ihrer eigene, spezielle SOD (Mn-SOD) zur Verfügung haben.

Das Strukturgen einer prokaryotischen Mn-SOD (E.coli) wurde cloniert und das chromosomale sodA-Gen lokalisiert (Touati, D., J. Bact. 155, 1078-1087, 1983).

Die 699 bp lange Nukleotidsequenz einer mitochondrialen Hefe Mn-SOD konnte aufgeklärt und die Primärstruktur sowohl des Precursors als auch des maturen Proteins davon abgeleitet werden - mit Molekulargewichten von 26123 Da für den Precursor und 23059 Da für das mature Protein (Marres, C.A.M. et al., Eur. J. Biochem. 147, 153-161 (1985). Somit unterscheiden sich die Mn - und Cu/Zn-SOD (MG=14893, EP-A 138111) in ihren Molekulargewichten deutlich.

Die vollständige Aminosäuresequenz der Mn-SOD aus Menschenleber wurde von Barra, D. publiziert, wonach die hMn-SOD aus 196 Aminosäuren zusammengesetzt sein soll(Barra, D. et al., J. Biol. Chem. 259, 12595-12601, 1984). Die Human Cu/Zn-SOD aus Erythrocyten besteht demgegenüber aus 153 Aminosäuren (Jabusch, J.R., et al. Biochemistry 19, 2310-2316, 1980) und zeigt keine Sequenzhomologien zur hMn-SOD) (Barra. D. et al. siehe oben).

Generell wird den Superoxiddismutasen eine Schutzfunktion gegenüber bestimmten Endzündungsprozessen zugesprochen. Insbesondere soll eine Defizienz an Mn-SOD bei der Entwicklung der rheumatoiden Arthritis eine Bedeutung zukommen (Pasquier, C. et al., siehe oben). Ein protektiver Effekt der SOD gegen alkoholinduzierte Leberschädigungen wird ebenfalls angenommen (Del Villano B.C. et al., Science 207, 991-993, 1980).

Die Klonierung und Expression einer Human-SOD ist nur für die Human Cu/Zn-SOD aus Menschenleber bekannt (EP-A 138111).

Anhand der vorgenannten essentiellen Eigenschaften der Superoxiddismutasen, insbesondere der hMn-SOD, ist ein Bedarf für den therapeutischen und/oder diagnostischen Einsatz zu erwarten. Dabei ist es vorteilhaft, zu diesem Zweck specieseigene, d.h. humane Mn-SOD in homogener Form und in ausreichenden Mengen zur Verfügung zu haben. Die sich daraus ableitende Zielvorstellung ist, zu erwartende immunologische Reaktionen z.B. nach therapeutischem Einsatz, zu minimieren oder zu verhindern.

Erst die Entwicklung von Technologien zur Rekombination von Fremd-DNA mit Vektor-DNA mit der Möglichkeit, auch erstere in Mikroorganismen stabil zu etablieren und zu exprimieren, gestattet es, homogene Proteine tierischer oder humaner Herkunft in großen Mengen bereitzustellen. Hierbei soll ein anderes Ziel erreicht werden, nämlich, daß das damit hergestellte Enzym - die hMn-SOD - ein gegenüber der authentischen genuinen hMn-SOD charakteristisches biologisches Aktivitätsspektrum zeigt.

Eine Aufgabe der vorliegenden Erfindung bestand daher darin, mit Hilfe gentechnologischer Verfahren erstmals die für dieses Enzym codierende DNA-Sequenz zu finden bzw. darzustellen und erstmals aufzuzeigen, nach welchen Methoden diese Sequenz erhalten werden kann.

Erfindungsgemäß wurde diese Aufgabe dadurch gelöst, daß eine aus Humanzellen plazentalen Ursprungs gewonnene cDNA-Genbank mit synthetisch hergestellten DNA-Sondenmolekülen durchsucht wurde und dadurch das für hMn-SOD codierende Gen isoliert werden konnte. Um das Gen für hMn-SOD zu erhalten, kann nach bekannten Methoden die mRNA aus solchen Zellen isoliert werden, die das gewünschte Enzym produzieren. Als Ausgangsmaterial sind verschiedene, z.B. metabolisch aktive drüsige Gewebe wie z.B. Leber oder Plazenta geeignet. Nach Herstellung der cDNA, die nach bekannten Methoden durch geprimte Synthese mit reverser Transkriptase anhand der isolierten mRNA erhalten werden kann, nachfolgendem Einbau in einen geeigneten Vektor und Amplifikation zu einer vollständigen cDNA-Genbank, kann diese mit einer definierten, radioaktiv markierten DNA-Sonde oder einer Mischung aus verschiedenen solcher Sonden durchsucht werden. Um die Degeneration des genetischen Codes zu berücksichtigen werden vorzugsweise definierte DNA-Sondenmischungen verwendet, die alle möglichen Nukleotidvariationen für ein und dieselbe Aminosäure repräsentieren bzw, die derart ausgewählt werden, daß die Anzahl der zu synthetisierenden DNA-Sonden einer Mischung möglichst gering und die Homologie zur gesuchten hMn-SOD DNA-Sequenz möglichst hoch ausfällt. Ein weiteres Auswahlkriterium für die Synthese von DNA-Sonden kann darin bestehen, daß diese komplementär sind zu mindestens zwei unabhängigen Regionen, beispielsweise nahe dem 3'- und 5'- Ende der putativen Gensequenz. Dadurch können anhand von mindestens zwei getrennten Hybridisierungen Klone identifiziert werden, die gegen beispielsweise beide unabhängige DNA-Sonden positive Signale zeigen. Diese Klone können dann vorzugsweise für die Isolierung des hMn-SOD Gens benutzt werden. da von ihnen zu erwarten ist, daß sie entweder einen wesentlichen Teil oder das komplette Gen für hMn-SOD enthalten.

Die besonderen DNA-Sequenzen, die für die erfindungsgemäßen DNA-Sonden verwendet wurden, wurden anhand der von Barra, D. et al. publizierten Aminosäuresequenz der Human Mn-SOD aus Lebergeweben (Barra, D. et al., Oxy Radicals and their scavenger Systems, Vol. 1, 336-339, 1983) abgeleitet. Insbesondere können vorzugsweise zwei Regionen der putativen hMn-SOD DNA-Sequenz verwendet werden, die für mindestens fünf Aminosäurereste, vorzugsweise für 8 Aminosäurereste codieren, wobei eine DNA-Sondenlänge von mindestens 14, vorzugsweise 23 Basen vorteilhaft ist. Besonders vorteilhaft ist es, wenn eine DNA-Sonde komplementär ist zu jener, abgeleiteten hMn-SOD DNA-Sequenz, deren genetische Information kolinear ist mit den Aminosäureresten 39 bis 46 und eine zweite DNA-Sonde komplementär ist zu der entsprechenden DNA-Region, die für die Aminosäurereste 200 bis 207 der bekannten Aminosäuresequenz codiert. Ebenso können natürlich auch DNA-Sequenzen als Sonden eingesetzt werden, die anhand anderer Mn-Superoxiddismutasen abgeleitet werden können.

Mit Hilfe einer solchen DNA-Sonde können positive Klone erhalten werden, aus denen eine cDNA-Sequenz nach folgender Formel Ia, isoliert werden kann, die einen großen Anteil einer für hMn-SOD codierenden Region enthält:

```
G ATC ATG CAG CTG CAC CAC AGC AAG CAC CAC GCG GCC TAC

  GTG AAC AAC CTG AAC GTC ACC GAG GAG AAG TAC CAG GAG

  GCG TTG GCC AAG GGA GAT GTT ACA GCC CAG ATA GCT CTT

  CAG CCT GCA CTG AAG TTC AAT GGT GGT GGT CAT ATC AAT

  CAT AGC ATT TTC TGG ACA AAC CTC AGC CCT AAC GGT GGT

  GGA GAA CCC AAA GGG GAG TTG CTG GAA GCC ATC AAA CGT

  GAC TTT GCT TCC TTT GAC AAG TTT AAG GAG AAG CTG ACG

  GCT GCA TCT GTT GGT GTC CAA GGC TCA GGT TGG GGT TGG

  CTT GGT TTC AAT AAG GAA CGG GGA CAC TTA CAA ATT GCT

  GCT TGT CCA AAT CAG GAT CCA CTG CAA GGA ACA ACA GGC

  CTT ATT CCA CTG CTG GGG ATT GAT GTG TGG GAG CAC GCT

  TAC TAC CTT CAG TAT AAA AAT GTC AGG CCT GAT TAT CTA

  AAA GCT ATT TGG AAT GTA ATC AAC TGG GAG AAT GTA ACT

  GAA AGA TAC ATG GCT TGC AAA AAG TAA
```

## Formel Ia

Überraschenderweise wurde gefunden, daß die gefundene cDNA für eine Aminosäuresequenz codiert, die sich von der publizierten Aminosäuresequenz (Barra, D. et al., J. Biol.Chem. 259, 12595-12601, 1984) in einigen Resten und in ihrer Länge voneinander unterscheidet. Die gefundenen Unterschiede dieser Sequenz zur "Barra-Sequenz" betreffen die Aminosäure-Positionen, 42, 88, 109 und 131 (jeweils Glu anstatt Gln) sowie zwei zusätzliche Aminosäuren Gly und Trp zwischen Position 123 und 124, sodaß die erfindungsgemäße DNA-Sequenz einer hMn-SOD von 198 Aminosäuren entspricht.

Völlig unerwartet war auch, daß andererseits eine für hMn-SOD codierende cDNA isoliert werden konnte, die eine Aminosäuresubstitution an Position 29 bedeutet (Codon für Gln anstatt für Lys) und somit in diesem Punkt einen weiteren Unterschied "zur Barra-Sequenz" und zur Formel Ia aufweist, entsprechend Formel Ib:

```
CAC CAC AGC CAG CAC CAC GCG GCC TAC GTG AAC AAC CTG AAC
GTC ACC GAG GAG AAG TAC CAG GAG GCG TTG GCC AAG GGA GAT
GTT ACA GCC CAG ATA GCT CTT CAG CCT GCA CTG AAG TTC AAT
GGT GGT GGT CAT ATC AAT CAT AGC ATT TTC TGG ACA AAC CTC
AGC CCT AAC GGT GGT GGA GAA CCC AAA GGG GAG TTG CTG GAA
GCC ATC AAA CGT GAC TTT GGT TCC TTT GAC AAG TTT AAG GAG
AAG CTG ACG GCT GCA TCT GTT GGT GTC CAA GGC TCA GGT TGG
GGT TGG CTT GGT TTC AAT AAG GAA CGG GGA CAC TTA CAA ATT
GCT GCT TGT CCA AAT CAG GAT CCA CTG CAA GGA ACA ACA GGC
CTT ATT CCA CTG CTG GGG ATT GAT GTG TGG GAG CAC GCT TAC
TAC CTT CAG TAT AAA AAT GTC AGG CCT GAT TAT CTA AAA GCT
ATT TGG AAT GTA ATC AAC TGG GAG AAT GTA ACT GAA AGA TAC
ATG GCT TGC AAA AAG TAA
```

## <u>Formel Ib</u>

Geht man davon aus, daß die Barra-Sequenz richtig analysiert worden ist, kann anhand der erfindungsgemäßen Nukleotid- bzw. Aminosäuresequenz die Möglichkeit eingeräumt werden, daß hiermit erstmals und überraschend die mögliche Existenz verschiedener Gene oder deren allele Ausprägungsformen oder Isoenzyme für hMn-SOD aufgezeigt wird.

Da cDNA tragende Klone erhalten werden können, denen das für das komplette hMn-SOD Gen notwendige Ende fehlt, war eine weitere Aufgabe der vorliegenden Erfindung die Bereitstellung des kompletten Gens für hMn-SOD.

Die Lösung dieser Aufgabe kann nach verschiedenen, bekannten Strategien erfolgen. Beispielsweise kann die erhaltene Sequenz selbst als DNA-Sonde eingesetzt und damit die cDNA-Bank nochmals durchsucht werden, um ein komplettes Gen bzw. eine cDNA mit dem jeweils fehlenden Ende zu finden oder die erhaltene DNA-Sequenz kann als Hybridisierungssonde gegen eine genomische Bank verwendet werden, um nach Identifizierung das komplette hMn-SOD Gen zu isolieren.

Oder man bedient sich der Möglichkeit, Oligonukleotide zu synthetisieren, deren Nuklectidsequenz dem fehlenden Ende der hMn-SOD entspricht und mit Hilfe dieser, nach geeigneter Linker-Ligation, die vollständige cDNA für hMn-SOD zu erhalten. Diese Methode besitzt den Vorteil, daß beispielsweise eine für hMn-SOD codierende DNA erhalten werden kann, deren 5'-Ende direkt mit dem Startcodon (ATG) beginnt.

Als besonders geeignet, diese Aufgabe zu lösen, erwies sich für die Komplettierung der beispielsweise ab Aminosäure 22 oder 26 codierenden, erfindungsgemäßen cDNAs die DNA-Sequenz der Formel II, beginnend mit dem 5'-Startcodon ATG und endend mit dem Codon für Aminosäure 31 (His, wobei AAG [Lys] = 1) unter Zugrundelegung der bekannten Codonpräferenzen wie sie beispielsweise für die Hefe Gültigkeit besitzen (Sharp, P.M. et al., Nucl.Acids.Res. <u>14</u> (13), 5125 - 5143, 1986)

```
5'   ATG AAG CAC TCT TTG CCA GAC TTG CCA TAC GAC TAC GGT GCT
     TAC TTC GTG AGA AAC GGT CTG AAG GGT ATG CTG ATG CCA CGA


     CTA GAA CCA CAC ATC AAT GCT CAA ATC ATG CAA TTG CAC CAC
     GAT CTT GGT GTG TAG TTG CGA GTT TAG TAC GTT AAC GTG GTG


     TCT AAG CAC CAT G
     AGA TTC GTG GTA C
```

## Formel II

Ebenso können andere bekannte synonyme Codons für die Komplettierung des hMn-SOD Gens oder zur in vitro Synthese des gesamten Gens verwendet werden, z.B. solche, die eine optimale Codon- Anticodon Wechselwirkung in Bakterien, z.B. E.coli, erleichtern und dort die Effizienz der Translation erhöhen (Grosjean, H. Fiers, W., Gene 18, 199 - 209, 1982; Ikemura, T., J. Mol. Biol. 151, 389 - 409, 1981) oder solche Codons, die den genuinen Verhältnissen in Säugetierzellen entsprechen (Grantham, R. et al., Nucleic Acid Research 9, 43-47, 1981). Letztere können vorzugsweise zur Transformation und nachfolgend zur Expression in Säugetierzellen eingesetzt werden.

Es ist prinzipiell möglich, den Methioninrest, der durch das Startcodon ATG kodiert wird und dem reifen hMn-SOD, beginnend mit der ersten Aminosäure Lysin, vorangestellt ist, nach an sich bekannten Verfahren, beispielsweise mit CNBr oder CNCl, abzuspalten. Da aber im reifen Enzym hMn-SOD weitere interne Methioninreste vorkommen können z.B. an Positionen 23 oder 192 - kann eine solche Vorgehensweise nicht durchführbar sein, sodaß in diesem Fall der zusätzliche N-terminale Methioninrest erhalten bleibt, ohne Einfluß auf die biologische Aktivität der hMn-SOD.

Es können jedoch auch enzymatische Spaltungen vorgesehen werden, wobei bekannterweise geeignete synthetische Linker eingesetzt werden können, da zu erwarten ist, daß sich Codons für entsprechende, spezifische Aminosäuren an den gewünschten Positionen auf dem Vektor, der die hMn-SOD cDNA enthält, befindet. Z. B. sind Arg- oder Lys-Reste für eine tryptische Spaltung zu nennen bzw. man bedient sich allgemein Codons, die für Protease-empfindliche Aminosäuren codieren. Diese können vor oder hinter dem Start-Codon positioniert werden oder innerhalb der codierenden Region.

Eine zusätzliche Aufgabe der vorliegenden Erfindung war es, mit Hilfe gentechnologischer Verfahren die für hMn-SOD codierende Sequenz in geeigneten Wirtszellen erstmals zur Expression zu bringen, das homogene Enzym hMn-SOD noch solchen Verfahren erstmalig herzustellen, zu isolieren und in reiner Form bereitzustellen und die dafür erforderliche Vorgehensweise erstmals zu beschreiben.

Erfindungsgemäß wurde diese Aufgabe dadurch gelöst, indem die für hMn-SOD codierenden DNA-Sequenzen, beispielsweise der Formeln IIIa, oder IIIb

```
5'  ATG AAG CAC TCT TTG CCA GAC TTG CCA TAC GAC TAC GGT

    GCT CTA GAA CCA CAC ATC AAT GCT CAA ATC ATG CAA TTG

    CAC CAC TCT AAG CAC CAC GCG GCC TAC GTG AAC AAC CTG

    AAC GTC ACC GAG GAG AAG TAC CAG GAG GCG TTG GCC AAG

    GGA GAT GTT ACA GCC CAG ATA GCT CTT CAG CCT GCA CTG

    AAG TTC AAT GGT GGT GGT CAT ATC AAT CAT AGC ATT TTC

    TGG ACA AAC CTC AGC CCT AAC GGT GGT GGA GAA CCC AAA

    GGG GAG TTG CTG GAA GCC ATC AAA CGT GAC TTT GGT TCC

    TTT GAC AAG TTT AAG GAG AAG CTG ACG GCT GCA TCT GTT

    GGT GTC CAA GGC TCA GGT TGG GGT TGG CTT GGT TTC AAT

    AAG GAA CGG GGA CAC TTA CAA ATT GCT GCT TGT CCA AAT

    CAC GAT CCA CTG CAA GGA ACA ACA GGC CTT ATT CCA CTG

    CTG GGG ATT GAT GTG TGG GAG CAC GCT TAC TAC CTT CAG

    TAT AAA AAT GTC AGG CCT GAT TAT CTA AAA GCT ATT TGG

    AAT GTA ATC AAC TGG GAG AAT GTA ACT GAA AGA TAC ATG

    GCT TGC AAA AAG TAA
```

**Formel IIIa**

```
5'  ATG AAG CAC TCT TTG CCA GAC TTG CCA TAC GAC TAC GGT

    GCT CTA GAA CCA CAC ATC AAT GCT CAA ATC ATG CAA TTG

    CAC CAC TCT CAG CAC CAC GCG GCC TAC GTG AAC AAC CTG

    AAC GTC ACC GAG GAG AAG TAC CAG GAG GCG TTG GCC AAG

    GGA GAT GTT ACA GCC CAG ATA GCT CTT CAG CCT GCA CTG

    AAG TTC AAT GGT GGT GGT CAT ATC AAT CAT AGC ATT TTC

    TGG ACA AAC CTC AGC CCT AAC GGT GGT GGA GAA CCC AAA

    GGG GAG TTG CTG GAA GCC ATC AAA CGT GAC TTT GGT TCC

    TTT GAC AAG TTT AAG GAG AAG CTG ACG GCT GCA TCT GTT

    GGT GTC CAA GGC TCA GGT TGG GGT TGG CTT GGT TTC AAT

    AAG GAA CGG GGA CAC TTA CAA ATT GCT GCT TGT CCA AAT

    CAG GAT CCA CTG CAA GGA ACA ACA GGC CTT ATT CCA CTG

    CTG GGG ATT GAT GTG TGG GAG CAC GCT TAC TAC CTT CAG

    TAT AAA AAT GTC AGG CCT GAT TAT CTA AAA GCT ATT TGG

    AAT GTA ATC AAC TGG GAG AAT GTA ACT GAA AGA TAC ATG

    GCT TGC AAA AAG TAA
```

## Formel IIb

gegebenenfalls mit entsprechenden Signal- oder Kontrollsequenzen versehen, in geeignete Vektoren inseriert und damit geeignete Wirtszellen transformiert wurden. Nach Kultivierung der transformierten Wirtszellen werden die gebildeten Polypeptide nach an sich bekannten Verfahren isoliert und gereinigt. Die erhaltenen Polypeptide entsprechen den nachfolgenden Formeln IVa und IVb.

```
    1                   5                   10                  15
    Lys His Ser Leu Pro Asp Leu Pro Tyr Asp Tyr Gly Ala Leu Glu


                        20                  25                  30
    Pro His Ile Asn Ala Gln Ile Met Gln Leu His His Ser Lys His


                        35                  40                  45
    His Ala Ala Tyr Val Asn Asn Leu Asn Val Thr Glu Glu Lys Tyr
```

```
                    50                           55                          60
Gln Glu Ala Leu Ala Lys Gly Asp Val Thr Ala Gln Ile Ala Leu

                    65                           70                          75
Gln Pro Ala Leu Lys Phe Asn Gly Gly Gly His Ile Asn His Ser

                    80                           85                          90
Ile Phe Trp Thr Asn Leu Ser Pro Asn Gly Gly Gly Glu Pro Lys

                    95                          100                         105
Gly Glu Leu Leu Glu Ala Ile Lys Arg Asp Phe Gly Ser Phe Asp

                   110                          115                         120
Lys Phe Lys Glu Lys Leu Thr Ala Ala Ser Val Gly Val Gln Gly

                   125                          130                         135
Ser Gly Trp Gly Trp Leu Gly Phe Asn Lys Glu Arg Gly His Leu

                   140                          145                         150
Gln Ile Ala Ala Cys Pro Asn Gln Asp Pro Leu Gln Gly Thr Thr

                   155                          160                         165
Gly Leu Ile Pro Leu Leu Gly Ile Asp Val Trp Glu His Ala Tyr

                   170                          175                         180
Tyr Leu Gln Tyr Lys Asn Val Arg Pro Asp Tyr Leu Lys Ala Ile

                   185                          190                         195
Trp Asn Val Ile Asn Trp Glu Asn Val Thr Glu Arg Tyr Met Ala


Cys Lys Lys
```

Formel IVa

```
        1                    5                        10                      15
        Lys His Ser Leu Pro Asp Leu Pro Tyr Asp Tyr Gly Ala Leu Glu


                            20                        25                      30
        Pro His Ile Asn Ala Gln Ile Met Gln Leu His His Ser Gln His


                            35                        40                      45
        His Ala Ala Tyr Val Asn Asn Leu Asn Val Thr Glu Glu Lys Tyr


                            50                        55                      60
        Gln Glu Ala Leu Ala Lys Gly Asp Val Thr Ala Gln Ile Ala Leu


                            65                        70                      75
        Gln Pro Ala Leu Lys Phe Asn Gly Gly Gly His Ile Asn His Ser


                            80                        85                      90
        Ile Phe Trp Thr Asn Leu Ser Pro Asn Gly Gly Gly Glu Pro Lys


                            95                        100                     105
        Gly Glu Leu Leu Glu Ala Ile Lys Arg Asp Phe Gly Ser Phe Asp


                            110                       115                     120
        Lys Phe Lys Glu Lys Leu Thr Ala Ala Ser Val Gly Val Gln Gly


                            125                       130                     135
        Ser Gly Trp Gly Trp Leu Gly Phe Asn Lys Glu Arg Gly His Leu


                            140                       145                     150
        Gln Ile Ala Ala Cys Pro Asn Gln Asp Pro Leu Gln Gly Thr Thr


                            155                       160                     165
        Gly Leu Ile Pro Leu Leu Gly Ile Asp Val Trp Glu His Ala Tyr
```

```
             170                    175                    180
 Tyr Leu Gln Tyr Lys Asn Val Arg Pro Asp Tyr Leu Lys Ala Ile

             185                    190                    195
 Trp Asn Val Ile Asn Trp Glu Asn Val Thr Glu Arg Tyr Met Ala


 Cys Lys Lys
```

## Formel IVb

Die Sequenz gemäß Formeln IIIa und IIIb eignen sich besonders zur Herstellung von nicht-glykolisierter hMn-SOD der Formeln IVa und IVb in Mikroorganismen, insbesondere in E.coli oder S. cerevisiae. Das Problem der Glykosylierung beispielsweise in Hefe kann dadurch umgangen werden, daß man sich Mutanten bedient, die Defizient sind in der Glykosylierung von Proteinen (alg Mutanten) (z.B. Huffaker, T.C., Robbins P.W. Proc. Natl. Acad. Sci. USA 80, 7466-7470, 1983).

Falls es notwendig oder sinnvoll erscheint, kann dem kompletten hMn-SOD Gen, beispielsweise gemäß Formeln IIIa oder IIIb eine Leader- bzw. Signalsequenz direkt dem ersten Codon der ersten N-terminalen Aminosäure der reifen hMn-SOD bzw. vor dem Startcodon ATG vorangestellt werden. Hiermit kann erreicht werden, daß die hMn-SOD aus der Wirtszelle transportiert und leicht aus dem Kulturmedium isoliert werden kann.

Derartige Signalsequenzen sind beschrieben worden; sie codieren für einen meist hydrophoben Proteinanteil, der durch posttranslationale Modifikationsprozesse in der Wirtszelle abgespalten wird (Davis, D.B., Tai.P.-C., Nature 283, 433-438,1980; Perlman, D., Halvorson, H.O., J.Mol. Biol. 167, 391-409, 1983). Wenn vor die erste Aminosäure der hMn-SOD ein ATG-Codon konstruiert worden ist, kann ein Genprodukt erhalten werden, das vor dem Lysin ein N-terminales Methionin enthält. Daß Signalsequenzen von Prokaryoten verwendet werden, um Proteine in das Periplasma zu sekretieren und korrekt zu prozessieren, ist bekannt. (z.B. Davis, B.D., Tai, P.-C., 1980).

Selbstverständlich ist es nach Isolierung und Klonierung der hMn-SOD DNA-Sequenz möglich, das durch diese Sequenz codierte Enzym spezifisch zu modifizieren. Enzymmodifikationen können beispielsweise über gezielte in vitro Mutationen mit syntetischen Oligonukleotiden erfolgen, wodurch die katalytischen Eigenschaften der hMn-SOD beeinflußt und neuartige enzymatische Aktivitäten geschaffen werden können. Die grundlegenden methodischen Schritte zur Durchführung derartiger Proteinmanipulationen sind bekannt (z.B. Winter, G. et al., Nature 299, 756 - 758, 1982; Dalbadie - Mc Farland, G. et al. Proc. Natl. Acad. Sci.USA, 79, 6409-6413, 1982).

Für die Klonierung, d.h. Amplifikation und Präparation, des hMn-SOD Gens kann E. coli verwendet werden, vorzugsweise E. coli C600 (Nelson et al. Virology 108,338-350, 1981) oder JM 101, bzw. E. coli-Stämme mit mindestens einem der bekannten sup-Genotypen. Die Klonierung kann aber auch in Gram-positiven Bakterien wie z.B. B.subtilis erfolgen. Derartige Systeme sind vielfach beschrieben.

Als geeignete Wirte für die Expression des erfindungsgemäßen hMn-SOD-Gens kommen sowohl Mikroorganismen als auch Kulturen multizellulärer Organismen in Frage.

Unter Mikroorganismen sind Prokaryoten, d.h. Gram-negative oder Gram-positive Bakterien, und Eukaryoten, wie Protozoen, Algen, Pilze bzw. höhere Protisten, zu verstehen. Von den Gram-negativen Bakterien sind besonders die Enterobacteriaceae, z.B. E. coli, von den Gram-positiven die Bacillaceae und apathogenen Micrococcaceae, z.B. B. subtilis und Staph. carnosus, von den Eukaryoten die Ascomyceten inbesondere die Hefen, z.B. Saccharomyces cerevisiae, bevorzugte Wirte.

Für einzellige Mikroorganismen stehen eine Vielzahl an Ausgangsvektoren zur Verfügung, die sowohl plasmidischen als auch virales Ursprungs sein können. Diese Vektoren können in einfacher Kopienzahl oder als Multicopy-Vektoren vorliegen. Derartige Vektoren, die zur Klonierung und Expression der erfindungsgemäßen hMn-SOD wie allgemein für eukaryotische DNA-Sequenzen geeignet sind, werden in vielen Publikationen und Manuals beschrieben (z.B. Maniatis, T. et al. Molecular Cloning, Cold Spring Harbor Laboratory, 1982; Glover, D.M. (ed.) DNA Cloning Vol. I, II, 1985) und sind kommerziell erhältlich.

Im allgemeinen können Plasmid-Vektoren, die in der Regel einen Replikationsursprung und Kontrollsequenzen für die Transkription, Translation und Expression enthalten, in Verbindung mit diesen Wirten verwendet werden. Diese Sequenzen sollten aus Spezies stammen, die kompatibel mit den Wirtszellen sind. Der Vektor trägt üblicherweise neben einer Replikationsstelle zusätzlich Erkennungssequenzen, die es ermöglichen, in transformierten Zelle phänotypisch zu selektionieren. Die Selektion kann entweder durch Komplementation, Suppression oder durch Inaktivierung eines Markers erfolgen. Hinsichtlich der beiden erstgenannten Verfahren sind auxotrophe Mutanten von Bakterien und Hefen, die defizient sind für ein essentielles Stoffwechselprodukt, oder Nonsense-Mutanten, in denen es bei der Translation des betroffenen Gens zum Kettenabbruch kommt. Verschiedene Suppressor-Gene, z.B.supD, E, F (supprimieren UAG), supC, G (supprimieren UAG oder UAA) sind bekannt.

Beim dritten Verfahren trägt der Vektor ein Resistenz-Gen gegen ein oder mehrere cytotoxische Agenzien, wie z.B. Antibiotika, Schwermetalle. Durch Insertion einer fremden DNA in solch ein Marker-Gen wird dieses inaktiviert, sodaß der neu entstandene Phänotyp vom ursprünglichen Phänotyp unterschieden werden kann.

Zum Beispiel kann E. coli mit pBR322 transformiert werden, ein Plasmid das aus E. coli Species stammt (Bolivar, et al., Gene $\underline{2}$, 95 (1977). pBR322 enthält Gene für Ampicillin- und Tetracyclin-Resistenz und liefert damit einfache Mittel, transformierte Zellen zu identifizieren, indem durch Klonieren in beispielsweise die PstI-Stelle im ß-Lactamase-Gen der Phänotyp Ap$^r$, Tc$^r$ zu Ap$^s$, Tc$^r$ konvertiert wird. Andere Verfahren sind gleichsam anwendbar, wofür beispielsweise die lacZ-Geninaktivierung bei λ-und M 13-Vektoren sowie bei verschiedenen Plasmiden (z.B. pUC, pUR) bedeutend ist. Diese sehr vielfältigen Selektionssysteme sind altbekannt und entprechend liegt darüber eine Fülle an Literatur vor.

Neben solchen Selektionsmarkern müssen derartige Vektoren, insbesondere Expressionsvektoren, Signalsequenzen aufweisen, die die korrekte Initiation und Termination der Transkription gewährleisten. Für die korrekte Transkription des hMn-SOD Gens können daher diese Vektoren eine bakterielle oder eukaryotische Transkriptionseinheit enthalten, bestehend aus einem Promotor, der kodierenden Region mit dem hMn-SOD Gen und des sich daran anschließenden Terminators. Entsprechend der Natur der Transkriptionseinheiten können diese konservierte Prototyp-Sequenzen wie z.B. Pribnow-Box oder TTG-Sequenz oder aber CAAT-Box, TATA-Box, die bekannten Terminationssignale (z.B. AATAAA, TATGT), sowie mindestens ein Stop-Codon enthalten, wobei vorzugsweise hinsichtlich des Wirtes homologe Promotoren und Terminatoren verwendet werden. Die gebildete mRNA enthält üblicherweise eine 3'poly(A)-Sequenz und/oder eine 5'cap-Struktur. Für die Translation des hMn-SOD Gens bedarf es einer ribosomalen Bindungsstelle (RBS), bestehend aus Shine/Dalgarno (S/D)-Sequenz und einem dazu in definiertem Abstand von allgemein 3 bis 12 Nukleotiden stehendem Initiationscodon sowie mindestens einem Stop-Codon. Alternativ können RBSs synthetisch hergestellt werden, wodurch die Homologie mit dem 3'-Ende der 16S rRNA erhöht werden kann (Jay, E. et al. Nucleic Acids Res. $\underline{10}$, 6319-6329, 1982).

Insbesondere bei eukaryotischen Expressionssystemen (z.B. S. cerevisiae) sollten vorzugsweise Regulationssysteme für die Translation wirtseigenem Ursprungs benutzt werden, da in Hefen keine den Prokaryoten analogen Verhältnisse (Homologie der S/D-Sequenz mit dem 3'-Ende der 16S rRNA) vorliegen und die Signale bzw. die RBS für die Initiation der Translation auf eine etwas andere Weise als bei Prokaryoten definiert sind (z.B. Kozak, M., Nucleic Acids Res. $\underline{9}$, 5233-5252, 1981; Kozak, M. J. Mol., Biol. $\underline{156}$, 807-820, 1982).

Vorzugsweise besitzt der Klonierungs - oder Expressionsvektor nur eine Restriktionsendonuklease-Erkennungsstelle, die entweder im Ausgangsvektor a priori vorliegt oder nachträglich über entsprechende Linker eingeführt werden kann. Linker können entweder chemisch leicht synthetisiert werden oder sind kommerziell erhältlich.

Häufig benutzte Hefe-Promotoren bei der Herstellung von entprechenden Expressionsplasmiden beinhalten solche Promotoren, die im Hefesystem die Expression besonders effizient steuern, wie z.B. PGK Promotor (Tuite, M.F. et al., The EMBO Journal 1, 603-608, 1982; Hitzeman, R.A. et al., Science $\underline{219}$, 620-625, 1983), PH05 Promotor (Hinnen, A.,& Meyhack, B., Current Topics in Microbiology and Immunology $\underline{96}$, 101-117, 1982; Kramer, R.A. et al., Proc. Natl. Acad. Sci. USA $\underline{81}$, 367-370, 1984), GAPDH Promotor (Urdea, M.S. et al. Proc. Natl. Acad. Sci. USA $\underline{80}$, 7461-7465, 1983), GAL10 Promotor (Broach et al. Experimental Manipulation of Gene Expression 83-117, 1983), Enolase (ENO)-Promotor (Holland, M.J. et al, J. Biol. Chem. $\underline{256}$, 1385-1395, 1981), A-Faktor Promotor (Bitter, G.-A. et al. Proc. Natl. Acad. Sci. USA $\underline{81}$, 5330-5334; Yakota, T. et al. Miami Winter Symp. 17. Meet. Adv. Gene Technol.2, 49-52, 1985)) oder der ADHI Promotor (Ammerer, G., Methods in Enzymology $\underline{101}$, 192-201, 1983; Hitzeman, R.A. et al. Nature $\underline{293}$, 717-722, 1981).

Auch sind Promotoren anderer glykolytischer Enzyme (Kawasaki und Fraenkel, Biochem. Biophys. Res. Comm. $\underline{108}$, I107-1112, 1982) geeignet, wie Hexokinase, Pyruvatdecarboxylase, Phosphofructokinase, Glucose-6-Phosphat Isomerase, Phosphoglucose Isomerase und Glucokinase. Bei der Konstruktion geeigneter Expressionsplasmide können die mit diesen Genen assoziierten Terminationssequenzen ebenfalls in den Expressions-Vektor am 3'Ende der zu exprimierenden Sequenz eingesetzt werden, um Polyadenylierung und Termination der mRNA vorzusehen. Andere Promotoren, die zudem noch den Vorteil der durch Wachstumsbedingungen kontrollierten Transkription besitzen, sind die Promotor-Regionen der Alkohol-Dehydrogenase-2, des Isocytochrom C, der abbauenden Enzyme, die mit dem Stickstoff-Metabolismus gekoppelt sind, die oben erwähnte Glycerinaldehyd-3-Phosphat Dehydrogenase (GAPDH) und der Enzyme, die für die Metabolisierung von Maltose und Galaktose verantwortlich sind. Promotoren, die durch den Hefe Mating Typ Locus reguliert werden, beispielsweise promotoren der Gene BARI, MECI, STE2, STE3, STE5 können bei temperaturregulierten Systemen durch die Verwendung von temperaturabhängigen sir Mutationen eingesetzt werden. (Rhine, Ph. D. Thesis, University of Oregon, Eugene, Oregon (1979), Herskowitz and Oshima, The Molecular Biology of the Yeast _Saccharomyces_, part I, 181-209 (1981), Cold Spring Harbor Laboratory).

Diese Mutationen beeinflussen die Expression der ruhenden Mating Typ Kassetten von Hefen und dadurch indirekt die Mating Typ abhängigen Promotoren. Generell ist jedoch jeder Plasmid-Vektor, der einen Hefe-kompatiblen Promotor, originäre Replikations- und Terminationssequenzen enthält, geeignet.

Für den Fall, daß die Expression von hMn-SOD in Bakterien erfolgen soll, sind vorzugsweise solche Promotoren einzusetzen, die eine hohe Syntheserate an mRNA bedingen und die darüberhinaus induzierbar sind. Bekannte und verwendete Promotoren beinhalten die Beta-Lactamase-(Penicillinase) und Lactose-Promotor-Systeme (Chang et al., Nature 275, 615 (1978); Itakura et al., Science 198, 1056 (1977); Goeddel et al., Nature 281, 544 (1979) inclusive des UV5-Promotors (Silverstone, A.E et al. Proc. Natl. Acad. Sci. USA 66, 773-779, 1970) und Tryptophan (trp) Promotor-Systeme (Goeddel et al., Nucleic Acids Res. 8 4057 (1980); Europa-Anmeldung, Offenlegungs-Nr. 0036 776). Darüberhinaus sind auch andere mikrobielle Promotoren entwickelt und benutzt worden. Die Gen-Sequenz für hMn-SOD kann beispielsweise unter der Kontrolle des Lambda-$P_L$Promotors transkribiert werden. Dieser Promotor ist bekannt als einer der besonders starken, steuerbaren Promotoren. Die Steuerung wird möglich durch einen thermolabilen Repressor cl (z.B. cl857), von dem benachbarte Restriktionsschnittstellen bekannt sind. Daneben können auch der Promotor der alkalischen Phosphatase aus E. coli (Ohsuye, K. et al., Nucleic Acids Res. 11, 1283-1294, 1983) und hybride Promotoren, wie z.B. den tac-Promotor (Amann, E. et al. Gene 25, 167-178, 1983; De Boer, H.A., et al. Proc. Natl. Acad. Sci. USA 80, 21-25, 1983). Über die Anwendung solcher tragbaren Promotoren (lacuv5, lacZ SD, tac) bzw. über Vektoren zur Herstellung fusionierter und nicht-fusionierter eukaryotischer Proteine in E. coli, kann auch in T. Maniatis et al., Molecular Cloning, Cold Spring Harbor Laboratory, 1982, insbesondere S. 412f nachgelesen werden. Die Expression und Translation einer hMn-SOD Sequenz in Bakterien kann auch unter Kontrolle anderer Regulationssysteme, die als "homolog" zu dem Organismus in seiner untransformierten Form gelten können, ablaufen. Beispielsweise können auch Promotor-Operator-Systeme wie Arabinose-Operator, Colicin El-Operator, Galactose-Operator, alkalische Phosphatase-Operator, trp-Operator, Xylose A Operator, u.ä. oder Teile davon verwendet werden.

Zur Klonierung oder Expression von hMn-SOD in Bakterien, beispielsweise in E. coli, oder in Hefen, beispielsweise in S. cerevisiae, stehen gutbekannte Vektoren zur Verfügung, von denen für die erstgenannten Wirtssysteme verteilhaft die pBR-(Bolivar, F. et al., Gene 2, 95-113,1977), pUC-(Vieira,I., Messing.I., Gene 19, 259-268,1982) pOP- (Fuller, F., Gene 19, 43-54, 1982), pAT-(Windass, J.D. et al., Nucleic Acids Res. 10, 6639-6657, 1982) pHV-Plasmide (Ehrlich, S.D., Proc. Natl. Acad. Sci. USA 75, 1433-1436, 1977), Lambda-Vektoren inclusive Phasmide (Brenner, S. et al., Gene 17, 27-44,1982), Cosmide (Collins, J., Hohn. B., Proc. Natl, Acad. Sci. USA 75, 4242-4246, 1979) und die anderen literaturbekannten Vektoren (z.B. Maniatis, T. et al., Molecular Cloning, Cold Spring Habor Laboratory, 1982), insbesondere pBR - und pUC-Derivate, beispielsweise pBR322, pUC18 verwendet werden können.

Als Expressionsvektoren in Hefen bieten sich integrierende (YIp), replizierende (YRp) und episomale (YEp) Vektoren (Struhl, K. et al. Proc. Natl. Acad. Sci. USA 76, 1035-1039, 1979; Stinchcomb, D.T. et al., Nature 282, 39-43, 1979; Hollenberg, C.P., Current Topics in Microbiology and Immunology 96, 119-144, 1982) an, vorzugsweise YEp13 (Broach, J.R. et al. Gene 8, 121-133, 1979), YIp5 (Struhl, K. et al., 1979 s.o., ATCC 37061) sowie pJDB207 (DSM 3181) oder pEAS102. Der Vektor pEAS102 kann erhalten werden, indem YIp5 mit PstI teilweise und mit BamHI vollständig verdaut und das isolierte 4.3 kb Fragment (enthält das URA3 Gen) mit dem 4.4kb BamHI/PstI-fragment von pJDB207 ligiert werden. Zusätzlich zu Mikroorganismen sind Kulturen multizellulärer Organismen ebenfalls geeignete Wirte für die Expression von hMn-SOD. Im Prinzip ist jede dieser Kulturen einsetzbar, ob von Wirbeltier- oder wirbellosen Tierzellkulturen. Größtes Interesse besteht jedoch an Wirbeltier-Zellen, so daß die Vermehrung von Wirbeltierzellen in Kultur (Gewebe-Kultur) in den letzten Jahren zu einer routinemäßiger Methode wurde. (Tissue, Culture, Academic Press, Kruse and Patterson, Editors, 1973). Beispiele solche nützlichen Wirtszellinien sind VERO- und HeLa-Zellen, Goldhamster-Eierstock (CHO)-Zellen und W138, BHK, COS-7 und MDCK-Zellinien. Expressionvektoren für diese Zellen enthalten üblicherweise einen Replikationsursprung, einen Promotor, der vor der zu exprimierenden hMn-SOD lokalisiert ist, eine notwendige Ribosomenbindungsstelle, RNA-Spleißstelle, Polyadenylierungsstelle und transkriptionelle Terminations-Sequenzen.

Bei der Verwendung von Säugetierzellen werden die Kontrollfunktionen auf den Expressions-Vektoren oftmals aus viralem Material vorgesehen. Beispielsweise stammen die üblicherweise verwendeten Promotoren aus Papovaviren wie Polyoma-, Papilloma Viren, Simian Virus 40 (SV 40)und aus Retroviren, Adenovirus Typ 2,. Die frühen und späten Promotoren des SV 40 und deren Anwendungen sind vielfältig beschrieben. Außerdem ist es möglich und oft empfehlenswert, Promotor- oder Kontroll-Sequenzen oder Spleißsignale zu verwenden, die mit den gewünschten Gensequenzen ursprünglich verknüpft sind, vorausgesetzt, diese Kontrollsequenzen sind kompatibel zu den Wirtzellsystemen. So sind SV40-Vektoren bekannt, in denen eine exogene eukaryotische DNA mit ihren eigenen Promotorsequenzen und Spleißsignalen neben dem späten SV40-Promotor ein stabiles Transkript liefern.

Ein Replikationsursprung kann entweder durch eine entsprechende Vektorkonstruktion erhalten werden, sodaß diese einen exogenen Startpunkt, beispielsweise aus SV 40 oder anderen viralen Quellen (z.B. Polyoma, Adeno, VSV, PBV, etc.) enthält oder dieser kann durch die chromosomalen Replikationsmechanismen der Wirtszelle geliefert werden.

Wird der Vektor in das Wirtszellenchromosom integriert, reicht die zuletztgenannte Maßnahme meistens aus.

Die Transformation der Zellen mit den Vehikeln kann durch eine Vielzahl an Verfahren erreicht werden. Beispielsweise kann sie durch Kalzium erfolgen, wobei entweder die Zellen in Magnesium gewaschen und die DNA den in Kalzium suspendierten Zellen zugegeben wird oder die Zellen werden einem Kopräzipitat von DNA und Kalziumphosphat ausgesetzt. Bei nachfolgender Genexpression werden die Zellen auf Medien übertragen, die für transformierte Zellen selektieren.

Bei intrazellulärer Produktion von hMn-SOD kann das Enzym dadurch isoliert werden, daß die Zellen nach Erreichen einer entsprechend hohen Zelldichte abzentrifugiert und danach enzymatisch oder mechanisch aufgeschlossen werden. Die Reinigung der erfindungsgemäßen hMn-SOD kann über bekannte biochemische Verfahren zur Protein- bzw. Enzymreinigung wie z.B. Dialyse, Elektrophorese, Präzipitation, Chromatographie oder Kombinationen davon erfolgen. Wenn das Enzym aus der Zelle sekretiert wird, werden analoge Verfahren zur Proteinreinigung durchgeführt, um hMn-SOD aus dem Kulturmedium in reiner Form zu erhalten.

Das mit diesen Methoden gereinigte, erfindungsgemäße hMn-SOD zeigt ein dem genuinem Enzym identisches biologisches Aktivitätspektrum in vivo und in vitro.

Unter diesen Aktivitäten sind sowohl immunologische Eigenschaften (z.B. Kreuzreaktion mit Antikörpern von genuiner hMn-SOD gegen die erfindungsgemäße hMn-SOD) als auch biochemische und enzymatische Aktivitäten zu verstehen. Für die biochemische und enzymatische Charakterisierung der hMn-SOD kann z.B. der Lehre von Marklund, S. (Marklund, S.& Marklund, G., Eur J. Biochem. 47, 469-474, 1974) gefolgt werden, wonach auch eine strikte Unterscheidung zwischen den Cu/Zn und Mn enthaltenden Enzymen, z.B. durch Zusatz von KCN (inhibiert Cu/Zn-SOD nicht aber Mn-SOD) oder anhand der unterschiedlichen pH-Abhängigkeit ihrer Aktivitäten gegeben ist (siehe insbesondere: Ysebaert-Vanneste, M., Vanneste, W.H., Anal. Biochem. 107, 86-95, 1980).

Bei dem erfindungsgemäßen Polypeptid handelt es sich nicht ausschließlich nur um das reife hMn-SOD, das im einzelnen beschrieben ist, sondern ebenfalls um jedwede Modifikation dieses Enzyms. Diese Modifikationen beinhalten z.B. Verkürzung des Moleküls am N- oder C-terminalen Ende, Austausch von Aminosäuren durch andere Reste, wodurch die Enzymaktivität nicht wesentlich verändert wird.

Gegenstand der Erfindung sind nicht nur Gensequenzen, die spezifisch für die beschriebene und beispielhaft belegte hMn-SOD codieren, sondern ebenfalls Modifikationen, die leicht und routinemäßig über Mutation, Abbau, Transposition oder Addition erhalten werden können. Jede Sequenz, die für die erfindungsgemäße hMn-SOD codiert (d.h. die das entsprechende und bekannte biologische Aktivitätsspektrum aufweist) und im Vergleich mit der gezeigten degeneriert ist, ist ebenfalls eingeschlossen; Fachleute auf diesem Gebiet sind in der Lage, DNA-Sequenzen insbesondere der codierenden Regionen zu degenerieren. Ebenso ist jede Sequenz, die für ein Polypeptid mit dem Aktivitätsspektrum der authentischen hMn-SOD codiert, und die mit den gezeigten Sequenzen (oder Teilen davon) unter stringenten Bedingungen hybridisiert beinhaltet.

Es gehört zum Stand der Technik, unter welchen definierten Bedingungen eine Hybridisierung (inclusive Vorwaschen, Prähybridisierung, Hybridisierung, Waschen) durchzuführen ist, um stringente Bedingungen zu erreichen. Für die Hybridisierung von Oligonukleotiden gegen eine Genbank ("gene bank screening") ist vorzugsweise unter den Bedingungen nach Wood, I.M. et al. (Proc. Natl. Acad. Sci. USA 82, 1582-1588, 1985) zu verfahren. Um zu testen, ob eine bestimmte DNA-Sequenz mit einer der erfindungsgemäßen, für hMn-SOD codierenden DNA-Sequenzen hybridisiert - entweder via in situ Hybridisierung gegen Plaques oder Bakterienkolonien oder via Southern-Blotting - sind die von Maniatis, T. et al. detailliert beschriebenen Methoden und Bedingungen zu übernehmen (Maniatis. T.et al., Molecular Cloning, Cold Sping Harber Laboratory, 1982, insbesondere S. 326-328 und S. 387-389). Alle vom Hintergrund sich deutlich abhebenden Signale zeigen demgemäß ein positives Hybridisierungssignal an.

Spezifischer werden die oben näher bezeichneten Aufgaben dadurch gelöst, daß aus Menschengewebe, vorzugsweise aus Gewebe der Plazenta des Menschen, die RNA präpariert wird. Während der Aufschluß von Gewebekulturzellen direkt mit heißem Phenol erfolgen kann, müssen Gewebe für diese Art der Extraktion erst in tiefgefrorenem Zustand, vorteilhafterweise in Gegenwart von pulverisiertem oder körnigen Trockeneis oder in flüssigem Stickstoff, zerkleinert werden (z.B. Starmix). Durch Phenol gebildete Aggregate zwischen mRNA und anderen RNAs können durch Formamid oder durch Erwärmen (z.B. 65°C) wieder aufgelöst werden. Ein bevorzugtes Verfahren zur RNA-Isolierung ist dasjenige nach Chirgwin (Chirgwin, J.M. et al.. Biochemistry 18, 5294-5299, 1979).Die poly(A)+ RNA kann aus dem von Protein und DNA gereinigtem Präparat zweckmäßigerweise durch Affinitätschromatographie, z.B. Poly(U)-Sepharose oder Oligo(dT)-Cellulose isoliert werden, da in der Regel eukaryotische mRNA-Populationen einen Poly(A)-Schwanz an ihrem 3'-Ende aufweisen (Aviv, H., Leder, P., Proc.Natl.Acad. Sci. USA 69, 1409 - 1412, 1972; Lindberg, U., Persson, T., Europ. J. Biochem. 31, 246 - 254, 1972).Zur Isolierung der poly(A)+-RNA kann bevorzugt nach Auffray (Auffray, C., Rougeon, F., Eur. J. Biochem. 107, 303-314, 1980) vorgegangen werden.

Eine Anreicherung der gereinigten mRNA kann dadurch erfolgen, daß die gesamte mRNA-Fraktion nach ihrer Größe aufgetrennt wird -z.B. durch Zentrifugation in einem Saccharosegradient. Die gewünschte mRNA kann z.B. über bekannte in-vitro-Proteinbiosynthese-Systeme (Retikulotyten, Oozyten von Xenopus laevis) nachgewiesen werden.

Die gereinigte mRNA oder die angereicherte Fraktion dient als Template für die Synthese des ersten Stranges der cDNA, die mit Hilfe der reversen Transkriptase und einem Primer erfolgt. Als Primer sind entweder Oligo (dT) oder synthetische Primer einsetzbar, letztere können anhand der bekannten Aminosäuresequenz der hMn-SOD abgeleitet werden und gestatten das wiederholte Primen der reversen Transkription (Uhlen, M. et al. EMBO Journal 1, 249 - 254, 1982).

Bei der vorliegenden Erfindung wurde die Synthese des ersten Stranges der cDNA mit Oligo(dT)12-18 als Primer in Gegenwart von dNTPs gestartet.

Für die Synthese des zweiten Stranges der cDNA können verschiedene bekannte Methoden angewendet werden, von denen auswahlweise das Primen mit einem komplementären Primer (Rougeon, F., Mach, B., J.Biol. Chem. 252, 2209 - 2217, 1977) das Selbstprimen anhand einer am 3'-Ende der cDNA liegenden "hairpin"-Struktur (Efstratiadis, A. et al, Cell 7, 279, 1976) oder mit einem durch RNaseH gebildeten Okazaki Fragment-ähnlichen Primer (Gubler, U., Hoffmann, B.J., Gene 25, 263, 1982) genannt sein sollen. In der vorliegenden Erfindung wurde die Methode bevorzugt, wie sie von Huynh, T.V. beschrieben wird (Huynh, T.V. et al., in DNA Cloning Vol I, (D.M. Glover ed.), chap. 2, S. 49-78, 1985). Die danach erhaltene doppelsträngige cDNA kann direkt in einem geeigneten Vektor, beispielsweise in einem Cosmid, Insertions- oder Substitionsvektor, insbesondere in einem Lambda-Vektor, vorzugsweise in λgt10 (Huynh, T.V. et al.,1985) kloniert bzw. verpackt werden. Für die Klonierung in Lambda stehen mehrere bekannte Methoden zur Verfügung, von denen beispielsweise das "Homopolymertailing über dA-dT bzw, dC-dG oder die Linker-Methode mit synthetischen Linkern genannt sein sollen (Maniatis, T. et al., Molecular Cloning, Cold Spring Harbor Laboratory, 1982; Huynh, T.V. et al. DNA cloning Vol I (D.M., Glover ed.) 1985, 1980; Watson, C.J., Jackson, F. dto, 1985, chapter 3). Im Falle des Klonierens der erfindungsgemäßen cDNA wurde diese in die EcoRI-Stelle von λgt10 inseriert. Die in-vitro-Verpakkung und Klonierung der erfindungsgemäßen cDNA bzw. die Konstruktion der cDNA Genbank erfolgte nach Huynh, T.V. et al. 1985, S. 49-78.

Mit der erhaltenen, eine cDNA-Genbank aus Plazentagewebe repräsentierenden Phagenpopulation wurde die Amplifikation und Plaque-Reinigung durch Infektion eines geeigneten Wirts, insbesondere von E.coli, vorzugsweise von E. coli C 600, bzw. unter Sicherstellung des lytischen Vermehrungszyklus von Lambda, durchgeführt.

Die cDNA-Genbank wurde mit radioaktiv markierten, synthetischen Oligonukleotiden, die anhand der publizierten Aminosäuresequenz (Barra, D. et al. Oxy Radicals and their scavenger Systems, Vol. 1, 336-339, 1983) abgeleitet wurde, unter stringenten Hybridisierungsbedingungen durchsucht. Bei der vorliegenden Erfindung wurde das in situ Hybridisierungsverfahren nach Benton und Davis (Benton, W.D., Davis, R.W., Science 196, 180 - 182, 1977) angewendet. Bevorzugt wurden zwei Gemische aus je acht synthetischen 23-mer Oligonukleotiden der Formel Va und Vb, die kolinear sind mit den Aminosäuren 39 bis 46 bzw. 200 - 207 der von Barra, D. et al. (s.o.) publizierten Aminosäuresequenz und die die Degeneration des genetischen Codes berücksichtigen. Der jeweils letzten Base am 5'-Ende dieser DNA-Sonden fehlt die Wobble-Base für das vollständige Codon für Gln (Aminosäure 46) bzw. Glu (Aminosäure 207).

A, G, C und T stehen für die entsprechenden Nukleotide, I für Inosin.

```
                       C   C   C
         5'       TGITA TT TC TCIGTIACITT
                   T   T   T
```

**Formel Va**

```
                   A   C       A
         5'     TCIGTIAC TT TCCCA TTIAT
                 G   T       G
```

**Formel Vb**

Die Oligonukleotid-Sonden können nach an sich bekannten chemischen Syntheseverfahren hergestellt werden. Für die vorliegende Erfindung wurde ein DNA-Synthesizer Modell 381A (Applied Biosystems) verwendet.

Die Synthese aller möglichen Kombinationen dieser beiden DNA-Sonden sichert, daß mindestens eines der vorhandenen Oligonukleotide optimal mit der für die Sonde komplementären einzelsträngigen DNA.Region des gesuchten hMn-SOD Gens paart. Der Einsatz zweier unabhängiger Pools von 23-mer Oligenukleotiden vermindert die Möglichkeit, daß die "falschen" Positiven ausgewählt werden.

Nach der Isolierung in sich homogener Plaques, die anhand positiver Signale nach Hybridisierung mit den beiden 23-mer DNA-Sonden identifiziert wurden, konnten sieben rekombinante Phagen isoliert und von deren DNA 500 bis 1000 bp lange EcoRi-Fragmente sequenziert werden. Nach Sequenzanalyse dieser EcoRI-Fragmente nach der Methode von Sanger (Sanger et al., Proc. Natl. Acad. Sci. USA 74, 5463 - 5467, 1977; Sanger F. et al. FEBS Letters 87, 107 - 111, 1978) und nach Subklonierung in die EcoRI-Stelle des M13-Vektors (Bluescribe, Vector Cloning Systems) und Transformation in E.coli, beispielsweise E.coli JM101, konnte gefunden werden, daß die EcoRI-Fragmente cDNA-Inserts enthalten, die für hMn-SOD ab Aminosäure 22 (Klone BS5, BS8, BS9, BS13 BSXIII) bzw. ab Aminosäure 26 (Klone BS3, BS12) codieren.

Es wurde jedoch überraschenderweise auch gefunden, daß sich aus den ermittelten DNA-Sequenzen einige Abweichungen zu der Aminosäuresequenz von Barra, D. et al (1984, s.o.) ergeben:

| Klon | Amino-Säure | Codon | abgeleitete Aminosäuren | Aminosäure nach Barra, D. et al, 1984 |
|---|---|---|---|---|
| BS3, BS12, | 29 | GAG | Gln | Lys (29) |
| BS5, BS9, BS13 BSXIII | 29 | AAG | Lys | Lys (29) |
| BS3, BS12, BS13, BS5, BS9 | 42 | GAG | Glu | Gln (42) |
| BSXIII | 88 | GAG | Glu | Gln (88) |
| BS8 | 29 | AAG | Lys | Lys (29) |
| | 42 | GAG | Glu | Gln (42) |
| | 88 | GAG | Glu | Gln (88) |
| | 109 | GAG | Glu | Gln (109) |
| | 124 | GGT | Gly | Δ |
| | 125 | TGG | Trp | Δ |
| | 139 | GAA | Glu | Gln (129) |

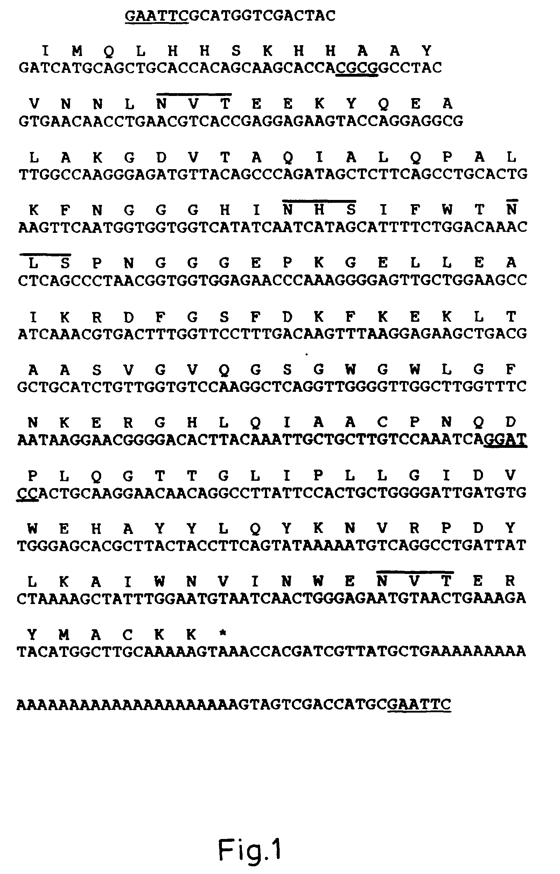

Die DNA Sequenz eines 617 bp langen EcoRI-Fragments, welches aus einem der erhaltenen Klonen, z.B. BS8 isoliert werden konnte, ist in Fig. 1 dargestellt. Das EcoRI-Fragment enthält eine 532 bp lange für hMn-SOD codierende Sequenz sowie eine 51 bp lange nicht-translatierte Region, inclusive eines poly(A)$_{30}$ Schwanzes. Anteile von Linker-Sequenzen sind bis zu den (vollständigen) EcoRI-Stellen ebenfalls dargestellt.

Die Positionen 30 bis 33 zeigen eine Thal-Schnittstelle, an Position 367 bis 372 ist eine BamHI-Stelle zu erkennen. Überraschenderweise finden sich Codons an Positionen 53 bis 61, 155 bis 163, 176 bis 184 sowie 500 bis 508, die kolinear sind für potentielle N-Glykosylierungsstellen der entsprechenden Aminosäuren gemäß der dafür charakteristischen allgemeinen Aminosäurenanordnungen Asn-X-Thr bzw. Asn-X-Ser, wobei X beispielsweise für Valin, Histidin oder Leucin steht, wohin gegen die Cu/Zn-SOD des Cytosols nur eine solche Aminosäurekombination aufweist.

Auf die Aminosäureunterschiede gegenüber der Aminosäure-Sequenz von Barra, D. et al. (Barra, D. et al., J. Biol. Chem. 259, 12595 - 12601, 1984), die von dem erhaltenen EcoRI-Fragment abgeleitet wurden, wurde schon an früherer Stelle eingegangen.

Um die fehlenden Basen an den 3' und/oder 5' Termini der hMn-SOD DNA-Teilsequenz aus der cDNA-Genbank zur Herstellung eines vollständigen hMn-SOD Gens zu erhalten, können verschiedene Strategien verfolgt werden. Beispielsweise kann die erhaltene cDNA als Hybridisierungssonde gegen eine genomische Genbank eingesetzt werden, um die für das ganze Enzym kodierende Sequenz zu erhalten, oder man verfährt z.B. nach Kakidani, H. unter Verwendung synthetischer, zur mRNA komplementärer Oligonkleotide als spezifische Primer für die reverse Transkription (Kakidani, H. et al. Nature 298, 245 - 249, 1982). Es ist jedoch auch möglich, das fehlende Ende der cDNA--Sequenz anhand der bekannten Aminosäuresequenz (Barra, D. et al., J. Biol. Chem. 259, 12595 - 12601, 1984) chemisch zu synthetisieren und mit der gefundenen cDNA zu verbinden, wobei ein definiertes Ende erhalten wird.

Für den zuletzt genannten Weg wurde zur Herstellung der vollständigen, erfindungsgemäßen DNA-Sequenz für hMn-SOD das 5'-Ende durch zwei Oligonukleotide der Formeln VIa und VIb komplettiert, die vorteilhafterweise XhoI/XbaI - bzw XbaI/NcoI - überstehende Enden aufweisen. Erfindungsgemäß ist am 5' - Ende des kodierenden Stranges das 3'-Ende des ADHI-Promotors berücksichtigt worden (Formel VIa)

```
5 TCGAG TATACA ATG AAG CAC TCT TTG CCA GAC TTG
3      C ATATGT TAC TTC GTG AGA AAC GGT CTG AAC
  XhoI


  CCA TAC GAC TAC GGT GCT
  GGT ATG CTG ATG CCA CGA GATC
                              XbaI
```

Formel VIa

```
5 CTAGAA CCA CAC ATC AAT GCT CAA ATC ATG CAA
3      TT GGT GTG TAG TTA CGA GTT TAG TAC GTT
  XbaI


  TTG CAC CAC TCT AAG CAC
  AAC GTG GTG AGA TTC GTG GTAC
                          NcoI
```

Formel VIb

Nach Kombination beider synthetischer Oligonukleotide der Formeln VIa und VIb, Klonierung in einen geeigneten Vektor, beispielsweise in ein entsprechend verändertes pUC18-Derivat und Hinzufügen des ThaI/EcoRI-Fragments der erfindungsgemäßen cDNA aus einem der erhaltenen Klone, dessen 5'-Ende mindestens die ThaI-Stelle aufweist, kann ein Plasmid erhalten werden, das eine vollständige cDNA des hMn-SOD Gens im richtigen Leserahmen entsprechend Formeln VIIa und VIIb enthält, ohne die ThaI-Stellen.

```
5'  ATG AAG CAC TCT TTG CCA GAC TTG CCA TAC GAC TAC GGT
    GCT CTA GAA CCA CAC ATC AAT GCT CAA ATC ATG CAA TTG
    CAC CAC TCT AAG CAC CAT GCG GCC TAC GTG AAC AAC CTG
    AAC GTC ACC GAG GAG AAG TAC CAG GAG GCG TTG GCC AAG
    GGA GAT GTT ACA GCC CAG ATA GCT CTT CAG CCT GCA CTG
    AAG TTC AAT GGT GGT GGT CAT ATC AAT CAT AGC ATT TTC
    TGG ACA AAC CTC AGC CCT AAC GGT GGT GGA GAA CCC AAA
    GGG GAG TTG CTG GAA GCC ATC AAA CGT GAC TTT GGT TCC
    TTT GAC AAG TTT AAG GAG AAG CTG ACG GCT GCA TCT GTT
    GGT GTC CAA GGC TCA GGT TGG GGT TGG CTT GGT TTC AAT
    AAG GAA CGG GGA CAC TTA CAA ATT GCT GCT TGT CCA AAT
    CAG GAT CCA CTG CAA GGA ACA ACA GGC CTT ATT CCA CTG
    CTG GGG ATT GAT GTG TGG GAG CAC GCT TAC TAC CTT CAG
    TAT AAA AAT GTC AGG CCT GAT TAT CTA AAA GCT ATT TGG
    AAT GTA ATC AAC TGG GAG AAT GTA ACT GAA AGA TAC ATG
    GCT TGC AAA AAG TAA
```

## Formel VIIa

```
5'      ATG AAG CAC TCT TTG CCA GAC TTG CCA TAC GAC TAC GGT

        GCT CTA GAA CCA CAC ATC AAT GCT CAA ATC ATG CAA TTG

        CAC CAC TCT CAG CAC CAT GCG GCC TAC GTG AAC AAC CTG

        AAC GTC ACC GAG GAG AAG TAC CAG GAG GCG TTG GCC AAG

        GGA GAT GTT ACA GCC CAG ATA GCT CTT CAG CCT GCA CTG

        AAG TTC AAT GGT GGT GGT CAT ATC AAT CAT AGC ATT TTC

        TGG ACA AAC CTC AGC CCT AAC GGT GGT GGA GAA CCC AAA

        GGG GAG TTG CTG GAA GCC ATC AAA CGT GAC TTT GGT TCC

        TTT GAC AAG TTT AAG GAG AAG CTG ACG GCT GCA TCT GTT

        GGT GTC CAA GGC TCA GGT TGG GGT TGG CTT GGT TTC AAT

        AAG GAA CGG GGA CAC TTA CAA ATT GCT GCT TGT CCA AAT

        CAG GAT CCA CTG CAA GGA ACA ACA GGC CTT ATT CCA CTG

        CTG GGG ATT GAT GTG TGG GAG CAC GCT TAC TAC CTT CAG

        TAT AAA AAT GTC AGG CCT GAT TAT CTA AAA GCT ATT TGG

        AAT GTA ATC AAC TGG GAG AAT GTA ACT GAA AGA TAC ATG

        GCT TGC AAA AAG TAA
```

## Formel VIIb

Die Durchsequenzierung der Klone BS5, BS9, BS13, BSXIII sowie der Klone BS3 und BS12 ergab, daß die Sequenzen der Klone BS5, BS9, BS13 und BSXIII identisch mit Klon BS8 sind. Die Klone BS3 und BS 12 zeigen, wie bereits angegeben, gegenüber Klon BS8, einen Unterschied in Aminosäure 29 (CAG statt AAG bzw. Gln statt Lys, Formeln Ib, IIIb und IVb). Ansonsten besteht 100% Homologie zu Klon BS8 bis zur Base 573 des in Fig 1 dargestellten EcoRI-Fragments (...TA*A ACC ACG ATC GTT ATG CTG$^{573}$). Ab dieser Base sind beide Klone BS3 und BS12 hinsichtlich der in Formel VIII angegeben 5'-ut(untranslatierte)-Region identisch.

```
5'AAG CAC TCT ... [Formel IIb] ... AAA AAG TAA ACC ACG

ATC GTT ATG CTG AGTAT GTTAA GCTCT TTATG ACTGT TTTTG TAGTG

GTATA GAGTA CTGCA GAATA CAGTA AGCTG CTCTA TTGTA GCATT TCTTG

ATGTT GCTTA GTCAC TTATT TCATA AACAA CTTAA TGTTC TGAAT AATTT

CTTAC TAAAC ATTTT GTTAT TGGGC AAGTG ATTGA AAATA GTAAA TGCTT

TGTGT GATTG AATCT GATTG GACAT TTTCT TCAGA GAGCT AAATT ACAAT

TGTCA TTTAT AAAAC CATCA AAAAT ATTCC ATCCA TATAC TTTGG GGACT

TGTAG GGATG CCTTT CTAGT CCTAT TCTAT TGCAG TTATA GAAAA GTAGT

CGACCATGCGGAATTC
Linker      EcoRI
```

## Formel VIII

Weiterhin wurden mehrere cDNA-Klone aus einer cDNA-Genbank (Plazenta) über λgt11 isoliert. Die Herstellung dieser cDNA-GenbanK erfolgte in gleicher Weise zu der in den Beispielen beschriebenen Herstellung der cDNA-Genbank aus Placenta-DNA in λgt10. Einer dieser aus λgt 11 isolierten Klone, Klon 4, wurde ebenfalls wie beschrieben, iii Bluescribe M13+ subkloniert. Die Sequenzierung erfolgte durch wiederholtes Priming mit den synthetischen 17mer

Oligonukleotiden

```
EBI 760 : 5´ AGATACATGGCTTGCAA 3´
EBI 765 : 5´ CTCTGAAGAAAATGTCC 3´
EBI 782 : 5´ GGAGATGTTACAGCCCA 3´
EBI 785 : 5´ AAGGAACGGGGACACTT 3´
```

Klon 4 ist bis auf Aminosäure 29 (AAG bzw. Lys) und einer ...TCTA ...-Sequenz am 3'Ende im Anschluß an die Multiklonierungstelle mit den Klonen BS3 und BS12 aus λgt10 identisch. Obwohl die analysierte DNA-Sequenz der verbliebenen 61 Basen des 5'-Endes (vor Formel Ia, Klon BS8, entsprechend Codons +1 bis +21 entsprechend Lys bis Glu) einige Basenänderungen gegenüber der abgeleiteten DNA-Sequenz (Formel II, enthalten in Formel IIIb) aufweist, ergibt die Übersetzung dieses DNA-Abschnittes keine Unterschiede zu Barra et al. 1984. Eine Leader-Sequenz vor dem ATG konnte ebenfalls analysiert werden. Die Formel IX zeigt die gefundene Sequenz von Klon 4.

EcoRI

(GGGCGAATTCCAGC)

```
-24                -20                     -15
 M    L    S    R    A    V    C    G    T    S    R    Q    L    P

ATG  TTG  AGC  CGG  GCA  GTG  TGC  GGC  ACC  AGC  AGG  CAG  CTG  CCT
```

```
-10                      -5                   -1   +1
 P    V*   L    G    Y    L    G    S    R    Q    K    H    S    L

CCG  GTT  TTG  GGG  TAT  CTG  GGC  TCC  AGG  CAG  AAG  CAC  AGC  CTC
```

```
+5                      +10                    +15
 P    D    L    P    Y    D    Y    G    A    L    E    P    H    I

CCC  GAC  CTG  CCC  TAC  GAC  TAC  GGC  GCC  CTG  GAA  CCT  CAC  ATC
```

```
        +20  +21
 N    A    Q    I

AAC  GCG  CAG  ATC ... [Formel Ia] ... AAA  AAG  TAA  ACC
```

```
ACG ATC GTT ATG CTG AGTAT GTTAA GCTCT TTATG ACTGT TTTTG

TAGTG GTATA GAGTA CTGCA GAATA CAGTA AGCTG CTCTA TTGTA

GCATT TCTTG ATGTT GCTTA GTCAC TTATT TCATA AACAA CTTAA

TGTTC TGAAT AATTT CTTAC TAAAC ATTTT GTTAT TGGGC AAGTG

ATTGA AAATA GTAAA TGCTT TGTGT GATTG AATCT GATTG GACAT

TTTCT TCAGA GAGCT AAATT ACAAT TGTCA TTTAT AAAAC CATCA

AAAAT ATTCC ATCCA TATAC TTTGG GGACT TGTAG GGATG CCTTT

CTAGT CCTAT TCTAT TGCAG TTATA GAAAA TCTA GGAATTCGCCC
                                              EcoRI-Linker
```

Formel IX

*Andere durchsequenzierte Klone zeigen an Position -9 Alanin (GCT).

Andere Klone haben unterschiedlich lange 5'ut - Regionen.

Die erfindungsgemäßen DNA-Sequenzen können in verschiedenen Expressionsvektoren eingebaut und mit Hilfe der beschriebenen Kontrollelemente exprimiert werden, vorzugsweise in pES103 mit dem ADHI-Promotor (DSM 4013). pES103 wird dadurch erhalten, daß in das pUC18-Derivat pES102, welches in der HincII-Schnittstelle einen Xho-Linker enthält, das 1500 bp lange BamHI/XhoI-Fragment des ADHI-Promotors (z.B. Ammerer, G., Methods in Enzymology 101, 192 - 201, 1983) eingebaut wird.

Anstelle dieser ADHI-Promotor-Sequenz von ursprünglich 1500 bp kann auch ein auf ca. 400 bp Länge verkürzter ADHI-Promotor als BamHI/XhoI-Fragment verwendet werden. Den verkürzten ADHI-Promotor (ADHIk) erhält man dadurch, daß Plasmid pWS323E (DSM 4016) mit BamHI/XhoI verdaut und der ADHIk Promotor isoliert wird.

Für die korrekte Termination wird eine geeignete Terminatorsequenz, zweckmäßigerweise ein ADH-Terminator, vorzugsweise der ADHII-Terminator hinter dem hMn-SOD ligiert. Der ADHII-Terminator (Beier, D.R., Young, E.T., Nature 300, 724 - 728, 1982) kann über SphI-Verdauung von pMW5-ADHII (Washington Research Fundation) als 1530 bp langes Fragment und nachfolgender HincII-Verdauung als endgültiger ADHII-Terminator (329 bp) erhalten werden, oder aus Plasmid pGD2 (DSM 4014) als 336 bp langes HindIII/XbaI-Fragment.

Für die Expression in Hefe stehen verschiedene Hefevektoren zur Verfügung, in die die Expressionskassetten mit dem erfindungsgemäßen hMn-SOD-Gen eingebaut werden können, vorzugsweise YEp13 (Broach, J.R. et al., Gene 8, 121 - 133, 1979; ATCC 37115), pJDB 207 (DSM 3181, hinterlegt am 28.12.1984), YIp5 (Struhl, K. et al., Proc. Natl.Acad. Sci. USA 76, 1035 - 1039, 1979; ATCC 37061), pEAS102 (pEAS102 kann dadurch erhalten werden, daß YIp5 mit PstI teilweise und mit BamHI vollständig verdaut wird und das das URA3 Gen enthaltende, isolierte 4.3 kb Fragment mit dem 4.4 kb BamHI/PstI-Fragment von pJDB207 ligiert wird.)

Mit diesen, eine Expressionskassette mit dem erfindungsgemäßen hMn-SOD Gen tragenden Hefevektoren können geeignete Hefezellen nach bekannten Verfahren transformiert werden. Als geeignete Hefezellen für die Expression können vorzugsweise alle diejenigen angesehen werden, die defizient sind für ihre eigene, hefespezifische Mn-SOD und die ein selektierbares Hefe-Gen, wie z.B. HIS3, URA3, LEU2, SUP, um nur einige zu nennen, enthalten. Derartige Mutanten, die beispielsweise durch in vitro oder in vivo konstruierte mutierte Gene und diese über ein "Transplacement" enthalten, sind über integrative Transformation zu erhalten (z.B. Winston, F. et al., Methods in Enzymclogy 101, 211-228, 1983). Das zu mutierende Mn-SOD Gen der Hefe ist beispielsweise in Plasmid pL41 als BamHI-Fragment enthalten (van Loon et al., Gene 26, 261-272, 1983). Da die vollständige Sequenz dieses BamHI-Fragments bekannt ist (Marres, C.A.M. et al., Eur.J. Biochem. 147, 153-161, 1985), ist das Mn-SOD Gen der Hefe auch ohne pL41 zugänglich.

Die durch solche Transformanten produzierte hMn-SOD kann nach bekannten Methoden der Proteinisolierung und Proteinreinigung gewonnen werden. Der Zellaufschluß kann z.B. nach van Loon et al. (Proc. Natl. Acad. Sci.USA 83, 3820 - 3824, 1986) erfolgen.

Für die Expression von hMn-SOD in Bakterien, vorzugsweise E.coli, beispielsweise E.coli HB101, C600, JM101, stehen die schon erwähnten und etablierten Expressionssysteme zur Verfügung. Die erfindungsgemäßen DNA-Sequenzen müssen zu diesem Zweck unter Kontrolle eines starken E.coli Promotors (s.o.), nicht unter einen eukaryotischen Promotor gebracht werden. Beispiele solcher bekannten Promotoren sind lac, lacuv5, trp, tac, trp-lacuv5, $\lambda P_L$, ompF,

bla. Die obligate Anwendung einer ribosomalen Bindungsstelle, um eine effiziente Translation in E.coli zu gewährleisten, wurde schon an früherer Stelle ausführlich beschrieben.

Zum Nachweis der Expression der hMn-SOD- Aktivität durch E.coli werden die Bakterien nach der Inkubation in einem geeigneten, herkömmlichen Kulturmedium aufgeschlossen und der Überstand auf hMn-SOD Aktivität, wie beschrieben (z.B. Marklund, S., Marklund, G., 1974; Ch. Beauchamp und I. Fridovich, Anal. Biochem. 44, 276 - 287, 1971; H.P. Misra und I. Fridovich, Arch.Biochem.Biophys. 183, 511 - 515, 1977; B.J. Davis, Annals of the NY Academy of Sciences 121, 404 - 427, 1964; M. Ysebaert-Vanneste and W.H. Vanneste, Anal.Biochem. 107, 86 - 95, 1980) getestet.

Der Nachweis des exprimierten hMn-SOD-Gens kann auch durch Markierung der Proteine in Maxizellen erbracht werden. Plasmidcodierte Proteine können durch Verwendung der Maxizelltechnik (Sancar, A. et al., J. Bacteriol, 137, 692 - 693, 1979) selektiv in vivo markiert werden. Der E.coli Stamm CSR603 (CGSC 5830) besitzt keine DNA-Repair-Mechanismen. Durch eine geeignete UV-Strahlendosis wird das bakterielle Chromosom zerstört, einige der wesentlich kleineren und in mehreren Kopien pro Zelle vorhandene Plasmid-DNAs bleiben dagegen funktionell erhalten. Nach Abtöten aller nicht geschädigten, sich vermehrenden Zellen durch das Antibiotikum D-Cycloserin und Aufbrauchen der endogenen mRNA werden in den verbleibenden Zellen nur noch Plasmid-codierte Gene transkribiert und translatiert. Die gebildeten Proteine können durch Einbau von $^{35}$S-Methionin radioaktiv markiert und nachgewiesen werden. E.coli CSR603 wird nach üblichen Verfahren mit den Expressionsplasmiden transformiert und auf ampicillinhaltigen Agarplatten auf transformierte Bakterien selektioniert. Die Präparation der Maxizellen und das Markieren der Proteine erfolgt nach der Vorschrift von A. Sancar (1979, s.o.). Als Molekulargewichtsstandard wird ein $^{14}$C-methyliertes Proteingemisch (Amersham) verwendet. Als Kontrolle wird das Plasmid, das nur den Promotor ohne das hMn-SOD-Gen enthält, eingesetzt.

Nach erfolgter Transformation des Wirtes, Expression des Gens und Fermentation oder Zellkultivierung unter Bedingungen, bei denen die erfindungsgemäßen Proteine exprimiert werden, kann das Produkt üblicherweise durch bekannte chromatographische Trennmethoden extrahiert werden, um so ein Material zu erhalten, das die Proteine mit oder ohne Leader und Tailing-Sequenzen enthält. Die erfindungsgemäße hMn-SOD kann mit einer Leader-Sequenz am N-Terminus exprimiert werden, die wie schon beschrieben, von einigen Wirtszellen entfernt werden kann. Wenn nicht, so ist, wie ebenfalls schon beschrieben, eine Abspaltung des Leader-Polypeptids (wenn vorhanden) erforderlich, um reifes hMn-SOD zu erhalten. Alternativ kann die Sequenz so modifiziert werden, daß das reife Enzym im Mikroorganismus direkt produziert wird. Für diesen Fall kann die Precursor-Sequenz des Hefe-Mating-Pheromons MF-alpha-1 verwendet werden, um eine korrekte "Reifung" des fusionierten Proteins und die Ausscheidung der Produkte in das Wachstumsmedium oder den periplasmischen Raum zu gewährleisten. Die "Sezernierung" der hMn-SOD in Hefemitochondrien kann dadurch erfolgen, indem direkt vor das hMn-SOD Gen die Leadersequenz für das Hefe-Mn.SOD Gen gestellt wird.

Geignete Leader-Sequenzen, beispielsweise die von Marres C.A.M. et al., Eur. J. Biochem. 147, 153-161 (1985) beschriebene oder Derivate davon, können entweder natürlichen Urprungs sein und aus entsprechenden eukaryotischen Zellen (z.B. S. cerevisiae) isoliert werden oder synthetisch hergestellt werden. Beispielsweise kann eine hefespezifische DNA-Präsequenz, die für den Import der hMn-SOD in das Hefe-Mitochondrium notwendig ist, durch Ligieren einzelner synthetischer Oligonukleotide erhalten werden. Erfindungsgemäß kann die komplette Präsequenz zwischen dem Startcodon ATG und dem ersten Codon für die erste Aminosäure der maturen hMn-SOD (Lys, z.B. AAG) oder einer beliebigen Portion eines N-terminalen Endes davon, bespielsweise in Formeln II, IIIa, IIIb, VIa, VIIa, VIIb, VIII oder IX, eingefügt werden. Ebenso kann eine derartige Präsequenz direkt nach dem ATG-Startcodon und direkt vor dem ersten Codon einer gegenüber der genuinen DNA-Sequenz der hMn-SOD durch Sequenzveränderungen mutierten und für ein Protein mit hMn-SOD Aktivität codierenden DNA eingebaut werden.

Eine Leader-Sequenz, die für den Zweck eines Imports einer hMn-SOD in das Hefe-Mitochondrium erfindungsgemäß verwendet werden kann, zeigt nachstehende Formel X, in der die bekannte Sequenz GCA GCT (Marres, C.A.M. et al, 1985, s.o.) zu GCT GCA ausgetauscht (beide Tripletts kodieren für Alanin) und eine PvuII-Erkennungstelle geschaffen wurde.

**PvuII**

TTCGCGAAAACAGCTGCAGCTAATTTAACCAAGAAGGGTGGTTTGTCATTGCTCT

CCACCACAGCAAGGAGAACC

**Formel X**

Vorzugsweise kann die Leader-Sequenz, beispielsweise gemäß Formel X, in dem XhoI/XbaI Fragment der Formel VIa enthalten sein. Damit wird erreicht, daß dieser 128 bp Linker mit dem Leader über die XhoI- und XbaI-Stellen derart mit dem restlichen hMn-SOD Gen verbunden werden kann, daß die Leadersequenz direkt nach dem Start ATG und direkt vor der ersten Aminosäure (Lysin) der hMn-SOD liegt (Formel XI).

XhoI                        PvuII

Start

5' TCGAGTATACAATGTTCGCGAAAACAGCTGCAGCTAA

CATATGTTACAAGCGCTTTTGTCGACGTCGATT


TTTAACCAAGAAGGGTGGTTTGTCATTGCTC

AAATTGGTTCTTCCCACCAAACAGTAACGAG


Lysin

TCCACCACAGCAAGGAGAACCAAGCACTCTTT

AGGTGGTGTCGTTCCTCTTGGTTCGTGAGAAA


GCCAGACTTGCCATACGACTACGGTGCT 3'

CGGTCTGAACGGTATGCTGATGCCACGAGATC

XbaI


Formel XI

Die Reinigung der hMn-SOD aus Zellen kann nach bekannten Verfahren erfolgen.

Die gentechnisch hergestellten, erfindungsgemäßen hMn-SOD sind aufgrund des biologisch-enzymatischen Wirkungsspektrums einerseits und wegen der jetzt verfügbaren Menge an hochreinem Enzym mit höchstmöglicher immunologischer Identität gegenüber der genuinen hMn-SoD andererseits für jede Art der Prävention, Behandlung und/oder Diagnostik im Bereich von entzündlichen, degenerativen, neoplastischen oder rheumatischen Erkrankungen zur Wundheilung, bei Autoimmunkrankheiten und bei Transplantationen geeignet -bzw. zur Prävention und Therapie von Krankheiten, die mit einer Defizienz an hMn-SOD einhergehen oder kausal damit verbunden sind. Beispielsweise umfassen die klinischen Anwendungsmöglichkeiten jene, wie sie aus Bannister W. H. und Bannister J. V. (Biological ans Clinical Aspects of Superoxide and Superoxide Dismutase, Vol. 11B, Elsevier/North-Holland, 1980) und aus Michelson, A. M., McCord, J. M., Fridovich (Superoxide and Superoxiddismutases, Academic Press, 1977) zu entnehmen sind. Ferner sind folgende klinische Anwendungsmöglichkeiten in Betracht zu ziehen: bei Perfusionswunden, Schlaganfall, alkoholgeschädigter Leber, Frühgeborenen, evtl. Pankreatitis, akuten Atemwegserkrankungen (ARDs), Emphysem, dialysegeschädigten Nieren, Osteoarthritis, rheumatoider Arthritis, strahleninduzierten Schäden, Sichelzellanämie.

Ebenso sind die erfindungsgemäßen hMn-SODs zur Erhöhung der Haltbarkeit von festen oder flüssigen Nahrungsmitteln geeignet.

Die erfindungsgemäßen hMn-SODs können entweder systemisch oder topisch verabreicht werden, wobei sich im ersten Fall herkömmliche parenterale Applikationsrouten (z.B. i.v., i.m. s.c., i.a.) und für den zweiten Fall die entsprechend bekannten Darreichungsformen (z.B. Pasten, Salben, Gele, Lutsch- oder Kautabletten, Pulver, und andere die lokale Resorption des hMn-SOD-Präparates ermöglichende galenische Formulierungen und pharmazeutisch akzeptable Trägerstoffe) eignen. Als therapeutisch wirksamer Dosisbereich können nach individuellen Kriterien (z.B. Patienten, Krankheitsschwere etc) beispielsweise ca.4mg täglich eingesetzt werden.

Es soll verdeutlicht werden, daß die vorliegende Erfindung solche humane Mn-Superoxiddismutasen einschließt, die in im Wesentlichen reiner Form aus menschlichen Zellen bzw. Zellverbänden isoliert und jene, die gentechnisch hergestellt werden. Ausgeschlossen von der Erfindung wird jedoch ein nicht gentechnisch gewonnenes Polypeptid, das aus humanem Lebergewebe stammt und am N-Terminus mit der Aminosäure Lysin (Lys) beginnt, welches im Vergleich zu Formeln IVa und/oder IVb an Position 29 ein Lysinrest (Lys), an Positionen 42, 88, 109 und 129 ein Glutaminrest (Gln), an Positionen 124 und 125 keinen Glycinrest (Gly) und Tryptophanrest (Trp) in der Reihenfolge Gly(124)-Trp(125) enthält.

Legenden zu den Figuren:

Fig. 1:      EcoRI-Fragment aus Klon BS8 mit der 532 bp langen codierenden Region ab Aminosäure 22 der reifen hMn-SOD, der 51 bp 3' ut-Region und den Sequenzanteilen des Linkers. Die potentiellen N-Glykosylie-

rungsstellen (überstrichen), die einzige Thal-Stelle und die BamHI-Stelle (unterstrichen) sind angegeben

Fig. 2: schematische Strategie zur Konstruktion von Plasmid HSOD4, welches das synthetische 5'Ende des hMn-SOD Gens als XhoI/NcoI-Fragment enthält.

Fig. 3 Restriktionskarten der Plasmide HSOD2 und HSOD3, sowie von dem daraus konstruierten Plasmid HSOD4.

Fig. 4 Konstruktion eines Plasmid (HSOD6) mit der vollständigen cDNA für hMn-SOD, als XhoI/EcoRI-Fragment.

Fig. 5 Präparation des Thal/EcoRI Fragments der hMn-SOD cDNA aus Klon BS8.

Fig. 6 Konstruktion von Plasmid p154/2, das den ADHI-Promotor als 1500 bp BamHI/XhoI-Fragment enthält.

Fig. 7 Konstruktion von Plasmid p150/2 mit den zur Expression von hMn-SOD notwendigen Einheiten ADHI-Promotor und ADHII-Terminator (336 bp XbaI/HindIII-Fragment)

Fig. 8 Fertigstellung der endgültigen Plasmide (pKHI und pKH2) mit dem ADHI-Promotor bzw. dem ADHIk-Promotor und dem ADHII-Terminator, zur weiteren Insertion der hMn-SOD cDNA über die XhoI/EcoRI-Stelle. Das Plasmid pKH2 entspricht pKHI, außer, daß pKH2 anstelle des ADHI-Promotors den ADHIk-Promotor enthält.

Fig. 9 Konstruktion der Expressionskassette HSOD7 mit dem verkürzten ca. 400 bp langen ADHI-Promotor (ADHIk). Die Konstruktion mit dem ADHI-Promotor der ursprünglichen Länge erfolgt ausgehend von pKHI in analoger Weise.

Fig. 10 Konstruktion von Plasmiden mit dem innerhalb des Hefe Mn-SOD Gens liegenden URA3-Gens als Marker in unterschiedlichen Orientierungen zum MN-SOD Gen (SODY7, SODY8), zur Herstellung einer für die Expression geeigneten Hefe Mn-SOD Mutante. Das Gen-Transplacement im entsprechenden Hefe-Stamm (DBY747) wurde mit SODY7 und SODY8 durchgeführt.

Fig. 11 Gelelektrophoretischer Nachweis der Expression von hMn-SOD über die Plasmide pWS490A und pWS491A im Hefestamm WS30-5g. Bahn 1: WS30-5g/pWS490AI, Bahn 2: WS30-5g/pWS490A2, Bahn 3: WS30-5g/pWS491AI, Bahn 4: WS30-5g/pWS491A2, Bahn 5: WS21-1(SOD1), enthält Hefe-MnSOD, Bahn 6: WS30-5g, Bahnen 7 bis 10: hMn-SOD aus Leber (O,3 ug Bahn 8, 1.2 ug Bahn 9, 3.0 ug Bahn 10). Mit den Ziffern 1 bzw. 2 hinter den Plasmidbezeichnungen sind unterschiedliche Transformanten mit denselben Plasmiden gemeint.

Fig.12 Analyse der MnSOD Aktivität in Hefeextrakten, welche die Expressionsplasmide pEO24-AB, pEO25-AC bzw. pEO26-AC enthalten, durch Auftrennung der Proteine im Polyacrylamidgel und anschließender Aktivitätsfärbung mit o-Dianisidin nach bekannter Methode:
a= WS30-5g, b=WS30-5g/pE024-AB,
c=WS30-5g/pE025-AC, d=WS30-5g/pEO26AD,
e=Marker(0.15μg human-Leber MnSOD).

Fig.13 Analyse der Aktivität von rekombinanter humaner MnSOD in den Mitochondrien bzw. im Cytoplasma von 6 verschiedenen Hefetransformanten, durch gelelektrophoretische Auftrennung des Proteins und anschließende Aktivitätsfärbung mit o-Dianisidin nach bekannter Methode (CP-Extr. = Cytoplasma Extrakt, MC-Extr.=Mitochondrien Extrakt):

| a=Marker, | 0,15μg humane Leber MnSOD | | |
|---|---|---|---|
| b=CP-Extr. | WS30-5g | pWS490A | ohne MC-Leader |
| c=MC-Extr. | WS30-5g | pWS490A | ohne MC-Leader |
| d=CP-Extr. | WS30-5g | pEO24-AB | mit MC-Leader |
| e=MC-Extr. | WS30-5g | pEO24-AB | mit MC-Leader |

(fortgesetzt)

| a=Marker, | 0,15µg humane Leber MnSOD | | |
|---|---|---|---|
| f=CP-Extr. | WS30-5g | pWS491A | ohne MC-Leader |
| g=MC-Extr. | WS30-5g | pWS491A | ohne MC-Leader |
| h=CP-Extr. | WS30-5g | pEO25-AC | mit MC-Leader |
| i=MC-Extr. | WS30-5g | pEO25-AC | mit MC-Leader |
| j=CP-Extr. | WS30-5g | pWS550A | ohne MC-Leader |
| k=MC-Extr. | WS30-5g | pWS550A | ohne MC-Leader |
| l=CP-Extr. | WS30-5g | pEO26-AD | mit MC-Leader |
| m=MC-Extr. | WS30-5g | pEO26-AD | mit MC-Leader |
| n=freie Spur | | | |
| o-Marker, | 0,075µg humane Leber MnSOD | | |

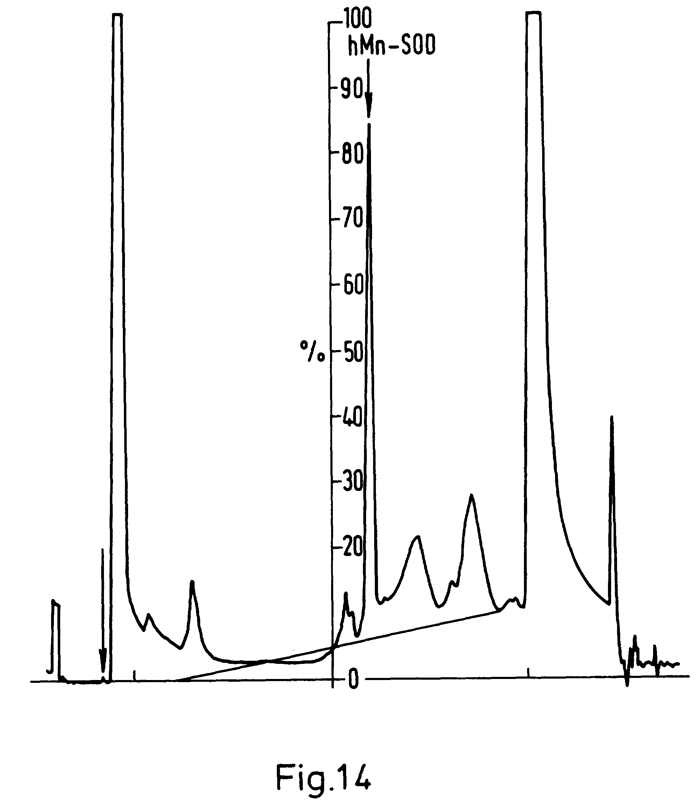

Fig. 14.  Elutionsdiagramm (Beispiel 15, Schritt 5) der Chromatographie der erfindungsgemäßen hMn-SOD nach $(NH_4)_2SO_4$-Fällung (Beispiel 15, Schritt 4) durch eine Mono S Kationenaustauschersäule (Pharmacia).

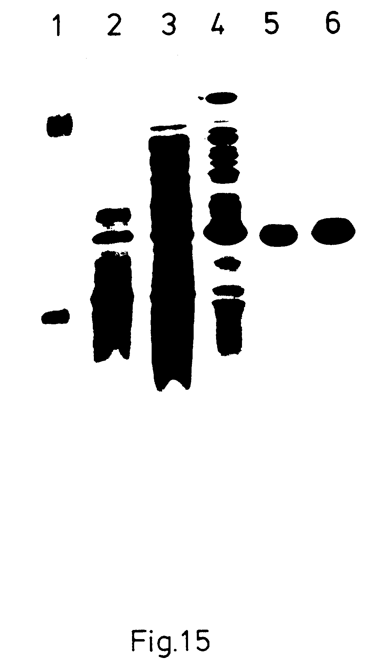

Fig. 15.  SDS-Polyacrylamidgel (15%, Silberfärbung) von hMn-SOD Proben nach verschiedenen Reinigungsstufen.

```
1= 4µl Marker (LMW-Pharmacia)1:50
2= 10µg Rohextrakt
3= 10µg nach Ammoniumsulfatfällung
4= 9µg nach Chromatographie an Mono S
5= 2.5µg ⎫ nach Chromatographie
6= 5µg   ⎭ an Hydroxylapatit
```

Die folgenden Beispiele, die die Erfindung nicht eingrenzen sollen, veranschaulichen die Erfindung im Detail.

Verwendete Materialien:

Wenn in den folgenden Beispielen nicht besonders angegeben, wurden folgende Materialien, Lösungen, Plasmide, Vektoren und Mikroorganismen benutzt:

ADHI-Promotor:
(1500bp BamHI/XhoI)

DSM 4013 (pES103),
hinterlegt am 27.2.87

ADHI-Promotor, verkürzt:
(400bp BamHI/XhoI)

DSM 4016 (pWS323E),
hinterlegt am
27.2.87

ADHII-Terminator:
(336 bp XbaI/HindIII)

DSM 4014 (pGD2),
hinterlegt am
27.2.87

BamHI-Puffer:

150mM NaCl, 6mM Tris-HCl
pH 7.9, 6 mM $MgCl_2$,
100µg/ml BSA

Core-Puffer:

50mM Tris-HCl pH 8.0, 10mM
$MgCl_2$, 50 mM NaCl

Denaturierlösung:

0,5M NaOH, 1.5M NaCl

Denhardt-Lösung (50x):     1g Polyvinylpyrrolidon
                           MG 360.000, 1g Ficoll, 1g
                           Rinderserumalbumin (BSA) ad
                           100ml $H_2O$

E.coli C600:               $F^-$, supE44, thil, thrl, leuB6,
                           lacYl, tonA21, $\lambda^-$
                           (ATCC 23724)

E.coli JM101:              supE, thi, $\Delta$(lac-pro AB), [F´,
                           traD36, proAB, lacI$^q$Z,$\Delta$M15]

High-Puffer:               100mM NaCl, 50mM Tris-HCl pH
                           7.5, 10mM $MgCl_2$, 1mM
                           Dithiothreitol (DTT)

HincII-Puffer:             10mM Tris-HCl pH 7.5, 60mM NaCl,
                           10mM $MgCl_2$, 1mM
                           2-Merkaptoäthanol, 100µg/ml BSA

Hybridisierlösung:         wie Prähybridisierlösung, ohne
                           Lachssperma-DNA

Klenow-Reaktionslösung:    22µl DNA/$H_2O$, 2,5µl 10x
                           NTR-Puffer (0,5M Tris-HCl pH
                           7.2, 0.1M $MgSO_4$, 1mM DTT,
                           500 µg/ml BSA) je 1µl 2mM
                           dATP, dGTP, dCTP, dTTP, 2.5 U
                           Klenow-Fragment (0.5 µl)

Lambda-Puffer:             100mM Tris-HCl pH 7.5, 10mM
                           $MgCl_2$, 1mM EDTA

LB-Agar:                   LB-Flüssigmedium, 15g/1
                           Bacto-Agar (Difco)

27

LB-Flüssigmedium:                10g/l Bacto-Trypton (Difco),
                                 5g/l Hefe-Extrakt (Difco), 5g/l
                                 NaCl, 10M NaOH ad pH 7.4

Ligationslösung:                 66mM Tris-HCl pH 7.6, 10mM
                                 $MgCl_2$, 5mM DTT, 1mM ATP, 1 U
                                 T4-DNA Ligase

Neutralisationslösung:           0,5M Tris-HCl pH 7.5, 1,5M NaCl

Nitrozellulosefilter:            Schleicher & Schuell,
                                 Membranfilter BA 85

NruI-Puffer:                     50mM KCl, 50mM NaCl, 50mM
                                 Tris-HCl pH 8.0, 10mM $MgCl_2$

Prähybridisierlösung:            5 x SSC, 5 x Denhardtlösung,
                                 50mM Na-Phosphatpuffer pH 6.8,
                                 1mM $Na_2P_4O_7$, 100µM ATP,
                                 0,1% SDS, 30 - 100 (50)µg/ml
                                 denaturierte, ultraschall-
                                 behandelte Lachssperma-DNA

puC18:                           Pharmacia

pURA3:                           DSM 4015,          hinterlegt am
                                 27.2.87

S. cerevisiae DBY747:            a, leu2, his3, trpl, ura3 (Yeast
                                 Genetic Stock Centre, Berkeley)

SC-URA-Medium:                   0,67% BYNB (Difco), 2% Glukose,
                                 2% 50x AS-Mix (pro Liter: 1g
                                 Histidin, 6g Leucin, 2,5g
                                 Tryptophan, 4g Lysin, 1.2g
                                 Adenin, 2g Arginin, 1g
                                 Methionin, 6g Phenylalanin,
                                 5g Threonin, 6g Isoleucin)

| | |
|---|---|
| **SmaI-Puffer:** | **10mM Tris-HCl pH 8.0, 20mM KCl, 10mM MgCl$_2$, 10mM 2-Merkaptoäthanol, 100µg/ml BSA** |
| **SphI-Puffer:** | **10mM Tris-HCl pH 7.5, 100mM NaCl, 10mM MgCl$_2$, 10mM 2-Merkaptoäthanol, 100µg/ml BSA** |
| **SSC (20x):** | **3.0M NaCl, 0.3M Na$_3$-Citrat, pH 7.0** |
| **SSPE (20x):** | **3.6M NaCl, 0,2M Na$_2$HPO$_4$, 20mM EDTA, mit NaOH (10N) ad pH 7.4** |
| **TE-Puffer:** | **10mM Tris-HCl pH 8.0, 1mM EDTA** |
| **ThaI-Puffer:** | **50mM Tris-HCl pH 8.0, 10mM MgCl$_2$** |
| **Top-Agarose:** | **LB-Flüssigmedium, 0,7% Agarose (Seaken FM-Agarose)** |
| **Vorwaschlösung:** | **1 M NaCl, 50mM Tris-HCl pH 8.0, 1mM EDTA, 0,1% SDS** |

Beispiel 1. <u>Konstruktion einer cDNA-Genbank</u>

Aus einer frischen Human-Placenta wurden würfelgroße Gewebestücke in flüssigem Stickstoff schockgefroren und das Gewebe bei unter -80°C pulverisiert. Aus dem pulverisiertem Gewebematerial wurde anschließend die RNA nach der von Chirgwin, J.M. et al beschriebenen Prozedur extrahiert und präpariert (Chirgwin, J.M. et al, Biochemistry <u>18</u>, 5294 - 5299, 1979).

Aus der danach erhaltenen RNA wurde die poly(A)[+]RNA nach Aviv, H. und Leder, P. (Proc. Natl. Acad. Sci. USA <u>69</u>, 1409 - 1412, 1972) präpariert.

Die Synthese der cDNA wurde mit "cDNA synthesis system" (Amersham RPN 1256) durchgeführt. Das Packaging erfolgte mit Gigapack (Vector cloning systems). Alle weiteren methodischen Schritte zur Klonierung in die EcoRI-Stelle von λgt10 wurden nach der Vorschrift von Huynh T.V. et al. (DNA Cloning Vol 1, D.M. Glover ed., IRL Press, Chapter 2, 1985) durchgeführt, außer daß als "platinq bacteria" E.coli C 600 verwendet wurde. Der Titer der die cDNA-Genbank repräsentierenden λgt10 Phagen betrug 1.2x10[10]pfu/ml, die Zahl der unabhängien Klone 1x10[6].

EP 0 282 899 B1

Beispiel 2. <u>Amplifikation der λgt10-Genbank</u>

Ein geeigneter E.coli Wirtstamm (C600, Genotyp F-, supE44, thi1, thr1 leuB6 lacY1 tonA21 lambda- (M.A. Hoyt et al., 1982, Cell 31, 5656) wurde über Nacht in LB-Medium, supplementiert mit 0,2% Maltose, bei 37° vorgezüchtet.

Diese Übernachtkultur wurde 5 min bei 3000 upm abzentrifugiert und in eiskalter 10 mM $MgSO_4$- Lösung suspendiert, sodaß die 0D600nm 4,0 betrug. Die so bereiteten Mg-Zellen wurden bei 4°C gelagert und konnten eine Woche verwendet werden.

12x200 ul Mg-Zellen wurden in sterilen Röhrchen mit einer Phagensuspension (je 50000pfu der cDNA-Genbank/Platte) gemischt und 20 min bei 37°C inkubiert.

Anschließend wurden zu jedem Röhrchen 6 - 7 ml geschmolzene, auf 42°C temperierte Top-Agarose (enthält 10 mM $MgSO_4$, Endkonzentration) pipettiert, gemischt und auf 12 bei 37°C vorgewärmte LB-Agarplatten (13,5 cm Durchmesser) mit 10 mM $MgSO_4$ ausgegossen und 6 - 12 Stunden bei 37°C inkubiert.

Beispiel 3. <u>Primärscreening zur Identifikation</u> rekombinanter λ-Phagen

a. Präparation der Nitrozellulosefilter

Die so bereiteten Platten wurden nach erfolgter Inkubation auf 4°C abgekühlt. Mit Bleistift numerierte Nitrozellulosefilter wurden auf die Plattenoberfläche gelegt und die Positionen auf den Platten durch Nadelstiche markiert. Etwa 1 min, nachdem sie vollkommen durchfeuchtet waren, wurden die Filter wieder vorsichtig abgezogen, in Denaturierlösung gelegt und 1 min bei Raumtemperatur (RT) inkubiert. Anschließend erfolgte die Neutralisation in Neutralisationslösung für 5 min bei RT und eine Inkubation für 30 sec in 2xSSPE ebenfalls bei RT.

Bis zu 3 weitere Abzüge wurden von jeder Platte angefertigt, wobei die Filter jeweils 30 sec länger auf der Platte belassen wurden. Die Positionen der Nadelstiche wurden auf die folgenden Filter genau übertragen.

Die Filter wurden auf Filterpapier liegend an der Luft getrocknet und die DNA durch zweistündiges Backen bei 80°C fixiert. Die Platten bewahrte man bis zum Ergebnis der nachfolgenden Hybridisierung auf.

b. Präparation der [32]P-markierten DNA-Sonden

Die synthetischen Oligonukleotidgemische wurden am 381A DNA-Synthesizer (Applied Biosystems) hergestellt, durch Polyacrylamidgelelektrophorese (20% in 8 M Harnstoff, T. Maniatis et al., Molecular Cloning, Cold Spring Harbor Laboratory, 1982, p173ff) gereinigt und über Sephadex G50 (Pharmacia) entsalzt. Die so synthetisierten DNA-Sonden sind komplementär zu RNA-Basensequenzen, die a) für die Aminosäuren 39-46 bzw. b) 200-207 kodieren (D. Barra et al., Oxy Radicals and their scavenger Systems, Vol. 1, 336-339, 1983) und haben folgende Basensequenzen:

```
                    C    C     C
a) 5'    TG ITA   TT   TC   TC IGT IAC ITT    3'
                  T    T     T


                    A    C     A
b) 5'    TC IGT IAC   TT   TC CCA   TT IAT    3'
                  G    T     G
```

wobei A, G, C und T für die entsprechenden Nukleotide, I für Inosin steht.

Die chemisch synthetisierten DNA-Probengemische wurden je in einer Konzentration von 20 pM/ul in Wasser gelöst.

Reaktionssatz:

20-100 pM gamma[32]- PATP (>3000Ci/mmol, Amersham), lyophilisiert aus äthanolischer Lösung, 20-100 pmol Oligonukleotid, 1 ul 10xKinasepuffer (0,7 M Tris-HCl pH 7,6, 0,1 m $MgCl_2$, 50 mM Dithiothreit, 10 Einheiten T4 Polynukleotidkinase (BRL), Wasser ad 10 ul.

Die Reaktion erfolgte 60 min bei 37°C und wurde durch Zugabe von 25 mM EDTA gestoppt. Nicht eingebaute Radioaktivität entfernte man durch Ausschlußchromatographie über eine 1 ml Biogel P6-DG (Biorad) Säule, hergestellt in einer 1 ml Einwegspritze. Als Elutionsmittel wurde TE-Puffer verwendet.

c. In situ Hybridisierung

Um Reste von Agarose und Bakterien von der Nitrozellulose zu entfernen, die bei der Hybridisierung zu starker Hintergrundstrahlung führen, wurden die Filter in einem nicht zu kleinem Volumen Vorwaschlösung einige Stunden bis zu über Nacht unter Schwenken bei 65°C inkubiert. Um unspezifische Bindungsstellen für DNA auf den Nitrozellulosefiltern abzusättigen, wurden diese 1-12 Stunden bei 37°C in der zuvor im Vakuum entgasten Prähybridisierlösung inkubiert.

Die zum Hybridisieren eingesetzten, radioaktiv markierten DNAs (ca. 1 x $10^9$ cpm/$\mu$g) wurden zur erforderlichen Menge an auf 37°C vorgewärmter und entgaster Hybridisierlösung zugegeben. Um die Konzentration der jeweiligen DNA-Probe in der Hybridisierlösung möglichst hoch zu halten, wurde nur so viel Hybridisierflüssigkeit verwendet, daß die Filter gerade von Flüssigkeit bedeckt waren. Die Hybridisierung erfolgte 12-18 Stunden bei 37°C.

Die Nitrozellulosefilter wurden anschließend entsprechend der Methode von Wood et al. (Proc.Natl.Acad.Sci. Vol 82, 1585-1588, 1985) 3mal in 6xSSC und 0,05% SDS gespült (4°C) und 2x30 min bei 4°C ebenso gewaschen. Anschließend wurden die Filter in einer frisch bereiteten Lösung, die 3 M Tetramethylammoniumchlorid (Me4NCl), 50 mM Tris-HCl pH8, 2 mM EDTA und 0,05% SDS enthält, 3mal bei Raumtemperatur (RT) gespült, 2x30 min (RT) gewaschen und schließlich 3x30 min bei 49°C (Oligonukleotidgemisch a)) bzw. bei 52°C (Oligonukleotidgemisch b))gewaschen, an der Luft getrocknet (Oligonukleotidgemisch b) und auf Papier geklebt. Röntgenfilme wurden 2-8 Tage bei -70°C unter Verwendung eines "intensifiying-screen" exponiert.

Beispiel 4. Plaquereiningung

Da bei der angewandten hohen Dichte der Plaques im ersten Suchvorgang keine einzelnen Plaques isoliert werden konnten, wurden die rekombinanten Lambda-Phagen durch mehrere aufeinanderfolgende Suchvorgänge bei gleichzeitig verringerter Plaquedichte gereinigt.Nach Entwicklung der Autoradiogramme wurden Regionen von der Agarplatte isoliert, (von drei durchgeführten Primärscreenings waren von 28 Regionen 2, von 35 Regionen 1 und von 15 Regionen 5 positiv), die auf beiden im Duplikat hybridisierten Nitrozellulosefiltern ein positives Hybridisiersignal ergaben. Dazu wurde die gewünschte Stelle mit dem engen Ende einer sterilen Pasteurpipette aus dem Agar gestochen und in 0,3 - 0,6 ml Lambda-Puffer (100 mM Tris HCl pH 7,5, 10 mM MgCl$_2$ und 1 mM EDTA) überführt. Es kann aber auch SM-Puffer (Maniatis T., Molecular-Cloning, 1982, S. 70) genommen werden. Nach Zugabe von einem Tropfen Chloroform ließ man die Phagen über Nacht bei 4°C aus dem Agar herausdiffundieren und plattierte jede einzelne Phagensuspension in mehreren Verdünnungen erneut aus. Von Platten, die 300-1000 Plaques aufwiesen, wurde erneut ein Nitrozellulosefilterabzug hergestellt und jeweils gegen beide DNA-Sonden hybridisiert. Dieser Vorgang wurde wiederholt, wobei Einzelplaques weiterverfolgt wurden, bis alle Plaques einer Platte ein positives Hybridisiersignal ergaben.

Beispiel 5. Analyse der erhaltenen Phagenklone

a. Titration der $\lambda$-Phagen

Die Phagensuspensionen wurden in Verdünnungsschritten von 1 : 10 mit Lambda-Puffer verdünnt, durch mehrmaliges Umschwenken gemischt und ausplattiert. Nach Inkubation bei 37°C konnten die auf dem Bakterienrasen entstandenen Plaques gezählt und der Titer (plaque forming units (pfu)) bestimmt werden. Der Titer betrug für die gereinigten Phagensuspensionen 2,2-8,6 x $10^{10}$pfu/ml.

b) Präparation der Lambdaphagen-DNA

Nach Isolation und Titration der in sich homogenen Phagenklone wurden diese in einer Dichte von 2 x $10^6$ pfu/13.5 cm Petrischale (mit dem Kulturmedien der Zusammensetzung: 1,5% Agarose, 10 g/l Trypton, 5 g/l Hefeextrakt, 5 g/l NaCl, 10 mM MgSO$_4$ und 0,2% Glukose) mit 200 ul C600 Mg-Zellen (4 OD$_{600}$) plattiert, 5 Stunden bei 37°C inkubiert und anschließend auf 4°C abgekühlt. Die Elution der Phagen erfolgte durch Übecschichten der Platten mit je 8 ml Lambdapuffer und einigen Tropfen Chloroform und leichtes Schwenken bei 4°C über Nacht. Der durch Zentrifugation (15000 upm, 15 min, 4°C) gereinigte Überstand wurde schließlich abgenommen und die Phagen durch Zentrifugation bei 50000 upm (Beckman Ti50 Rotor) 30 min bei RT pelletiert. Nach Zugabe von je 500 $\mu$l Lambdapuffer und Inkubation mit Ribonuklease A (RNase A, 10 $\mu$g/ml) und Desoxiribonuklease (DNase, 1 $\mu$g/ml), 30 min bei 37°C, wurde die Salzkonzentration durch Zugabe von je 25 ul 0,5 M EDTA, 12 ul 1 M Tris-HCl pH 8.0 und 6.5 ul 20% SDS erhöht und die vorhandenen Enzyme durch Inkubation bei 70°C für 15 min inaktiviert. Nach einer Extraktion mit Phenol und zweimaliger Extraktion mit Chloroform/Isoamylalkohol (24:1) zu jeweils gleichen Volumina wurde die DNA durch Zugabe von 0,1 Vol. 3 M Natriumazetat pH 5,2 und 2 Vol Alkohol ausgefällt, abzentrifugiert, mit 70% Alkohol gewaschen, getrocknet und in 50 ul TE-Puffer aufgenommen.

c. Restriktionsanalyse

Je 2 ul DNA-Lösung wurden mit 5 Einheiten EcoRI in HIGH-Puffer 2 Stunden bei 37°C inkubiert, die erhaltenen Fragmente auf einem 1 % Agarosegel (T. Maniatis et al., 1982, p149ff) unter einer Spannung von 1-5 Volt/cm aufgetrennt, die Fragmente mit Längen zwischen 500 und 1000 Basenpaaren vom Gel eluiert (G.M. Dretzen et al., Anal. Biochem.112, 295-298, 1981) und schließlich einer Sequenzanalyse unterworfen.

d. Sequenzanalyse

Die Subklonierung der Restriktionsfragmente in einen für die Sequenzbestimmung nach Sanger (F. Sanger et al., Proc.Natl. Acad.Sci. 74, 5463-5467, 1977; F. Sanger et al. FEBS-Letters 87, 107-111, 1978) geeigneten Vektor (Bluescribe M13+ bzw. M13-, Vektor Cloning Systems (C. Yanisch-Perron et al., Gene 33, 103-119, 1985)) erfolgte nach den üblichen Methoden zur Durchführung von Restriktion und Ligation von DNA-Fragmenten und Transformation von E.coli Wirtszellen (T. Maniatis et al., 1982, Molecular Cloning, Cold Spring Harbor Press, p104, 146ff, 396; DNA-Cloning, IRL-Press 1985, Vol 1, chapter 6). So wurden 100 ng isolierte EcoRI-cDNA-Fragmente über EcoRI-Stellen in die entsprechend vorbereitete dsDNA-Form (replikative Form, 50 ng) des Vektors insertiert (durch Inkubation von 2-12 Stunden bei 14°C in 10 ul Ligationslösung) und mit dieser rekombinanten Konstruktion (mit den Bezeichnungen BS3, BS5, BS8, BS9, BS12, BS13, BSXIII) kompetente E.coli (Stamm JM 101) Zellen transformiert.

Es wurde die Einzelstrang-DNA der rekombinanten Phagen isoliert und nach Sanger sequenziert. Die gelesenen Sequenzen wurden mittels geeigneter Computerprogramme (R. Staden, Nucl.Acid.Res. 10, 4731-4751, 1982) verarbeitet. Der isolierte Klon 8 (BS8) enthält die kodierende Sequenz ab Aminosäure 22 des reifen Enzyms (Fig. 1)

Beispiel 6. Konstruktion einer Expressionskassette

Zur Expression der hMn-SOD in Hefe sind Vervollständigung der isolierten cDNA, sowie die Konstruktion einer Expressionskassette notwendig, wobei der ADHI-Promotor in seiner ursprünglichen Länge (ca. 1500 bp, Methods in Enzymology, Vol. 101, Part C, 192-201, 1983), derselbe in verkürzter Form (ADHIK ca. 400 bp) und der ADHII-Terminator (Dr. R. Beier and E.T. Young, Nature 300, 724-728, 1982) zur Anwendung kommt.

a. Vervollständigung des Gens

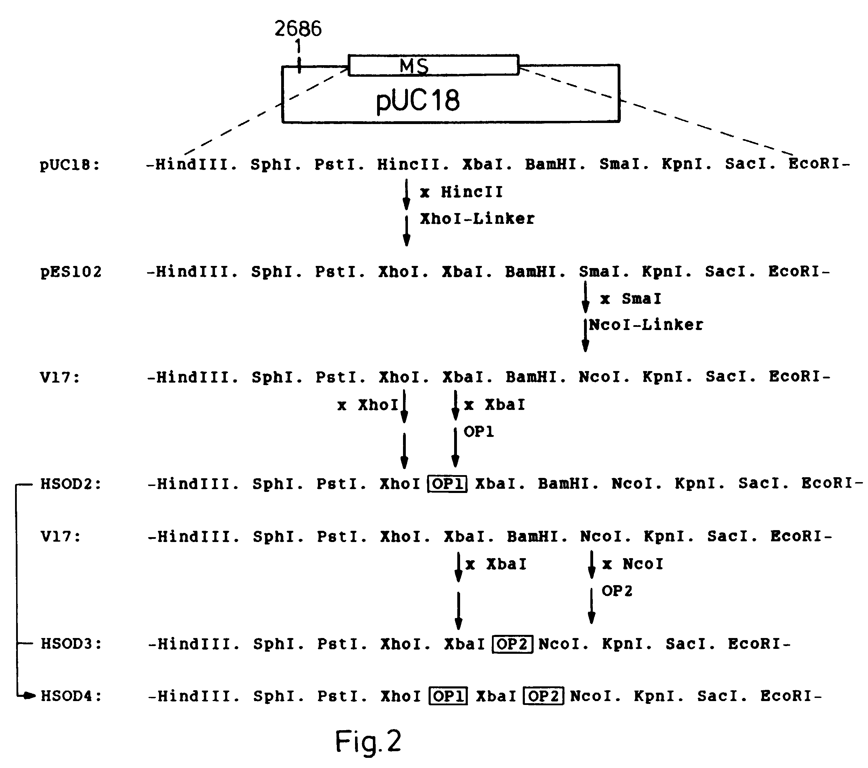

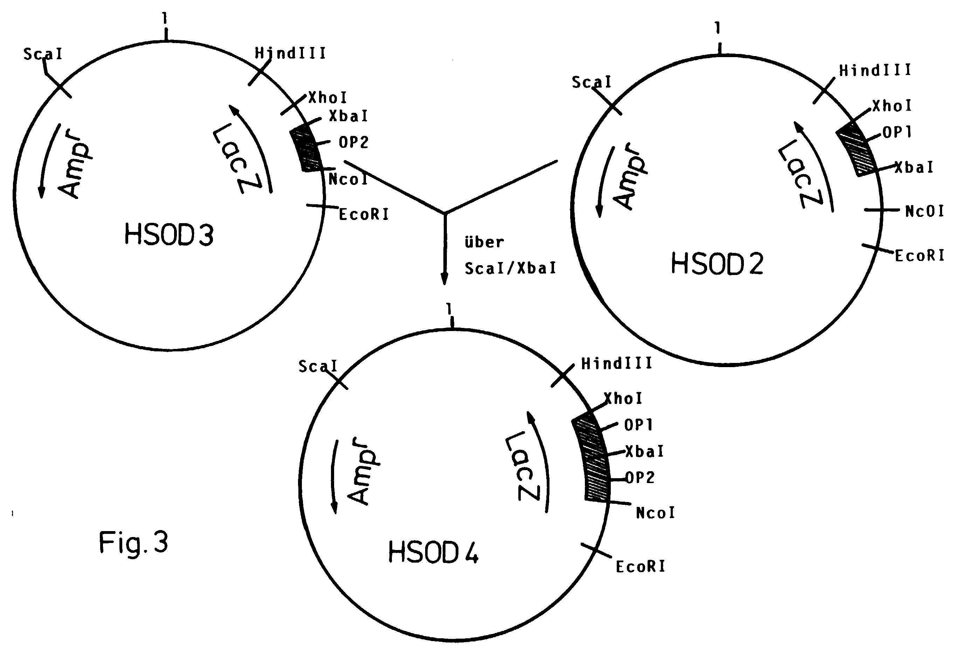

Da dem isolierten cDNA-Klon 8 am N-Terminus die den 21 Aminosäuren (AS) entsprechende Basenanzahl fehlt, wurden zur Vervollständigung des Gens entsprechend der berichteten Aminosäure-Sequenz (D. Barra et al., J.Biol.Chem.259, 12595-12601, 1984) unter Berücksichtigung der Hefe-Codon-Auswahl (P.M.Sharp et al., Nucl.Acids.Res.14, 5125-5143, 1986) 2 Oligonukleotidpaare als XhoI-XbaI-Fragment (OP1, entsprechend Formel VIa) bzw. XbaI-NcoI-Fragment (OP2, entsprechend Formel VIb) konstruiert und synthetisiert (381A DNA-Synthesizer, Applied Biosystems). OP1 wurde über XhoI/XbaI in das Plasmid V 17 (entstanden aus pUC18 (J. Vieira and J. Messing, Gene 19, 259, 1982) nach HincII Restriktion und Insertierung von XhoI-Linkern (New England Biolabs,d(CCTCGAGG) und SmaI-Restriktion des entstandenen Plasmids pES102 mit nachfolgender Insertierung von NcoI-Linkern (New England Biolabs, d(CCCATGGG)),insertiert(Fig. 2), OP2 über XbaI/NcoI: Dazu wurden je 4 ug V 17-DNA mit je 10 Einheiten XbaI und NcoI bzw. XhoI und XbaI in 40 ul CORE-Puffer 2 Stunden bei 37°C verdaut und durch Gelelektrophorese (0,7 % Agarose, s.o.) gereinigt. Je 5 µl der synthetisierten Einzelstränge von OP1 bzw. von OP2 (jeweils 10 pM/ul) wurden gemischt, 10 min bei 65°C inkubiert und langsam auf RT abgekühlt. Jeweils 1/10 davon wurden mit je 50 ng doppelt geschnittenem Vektor (XhoI/XbaI für OP1 und XbaI/NcoI für OP2) unter oben beschriebenen Bedingungen ligiert (Plasmide HSOD2 und HSOD3, Fig. 2). Schließlich wurden HSOD2 und HSOD3 über ScaI/XbaI (also nach doppelter Verdauung mit ScaI und XbaI in CORE-Puffer 2 Stunden bei 37°C) nach Reinigung und Isolierung der geschnitten Vektoren durch Gelelektrophorese und Ligation unter oben beschriebenen Bedingungen (Klonieren der Oligopaare OP1 und OP2) zu Plasmid HSOD4 kombiniert (Fig. 2, 3), dieses durch NcoI-Restriktion, darauf folgendem Klenow-Fill-in und EcoRI-Restriktion für die Aufnahme des ThaI/EcoRI-cDNA-Fragments vorbereitet: 5 ug DNA wurden in 50 ul High-Puffer mit 18 Einheiten NcoI bei 37°C mehrere Stunden inkubiert, die geschnittene DNA durch Gelelektrophorese gereinigt, isoliert und die Hälfte davon in 30 ul Klenow-Reaktionslösung 1 Stunde bei RT inkubiert.

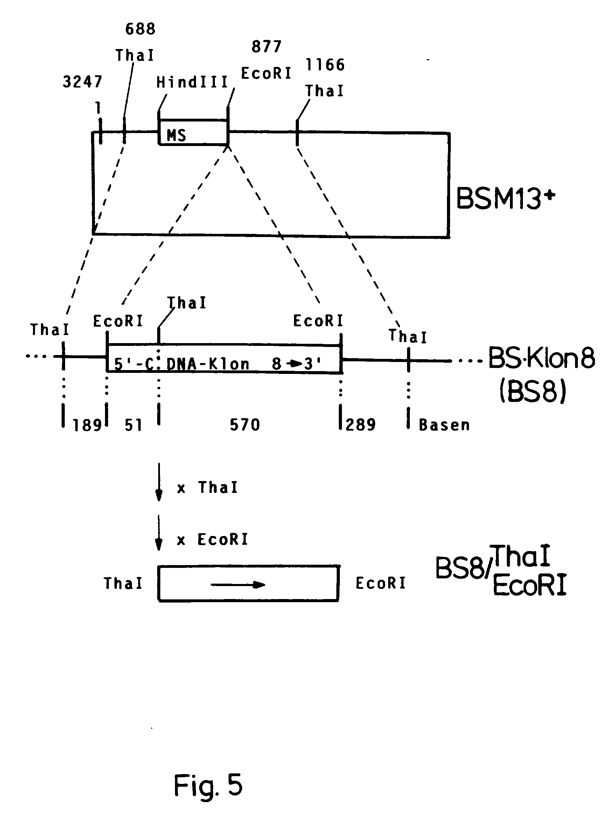

Nach Beendigung der Reaktion durch Zugabe von 2 ul 0,5 M EDTA und Inkubation der Reaktionslösung bei 70°C (10 min) wurde die DNA durch Gelelektrophorese gereinigt, isoliert und mit 7,5 Einheiten EcoRI in 20 ul HIGH-Puffer nachgeschnitten, nochmals gereinigt und isoliert. (Fig. 5)

Das ThaI/EcoRI-cDNA-Fragment wurde wie folgt präpariert:

Mit dem Plasmid BS8, das den isolierten cDNA-Klon8 (s.o.) enthält, wurden kompetente E.coli (Stamm JM 101) Wirtszellen transformiert, und das Plasmid unter geeigneten Bedingungen präpariert (T. Maniatis et al, 1982, p368).

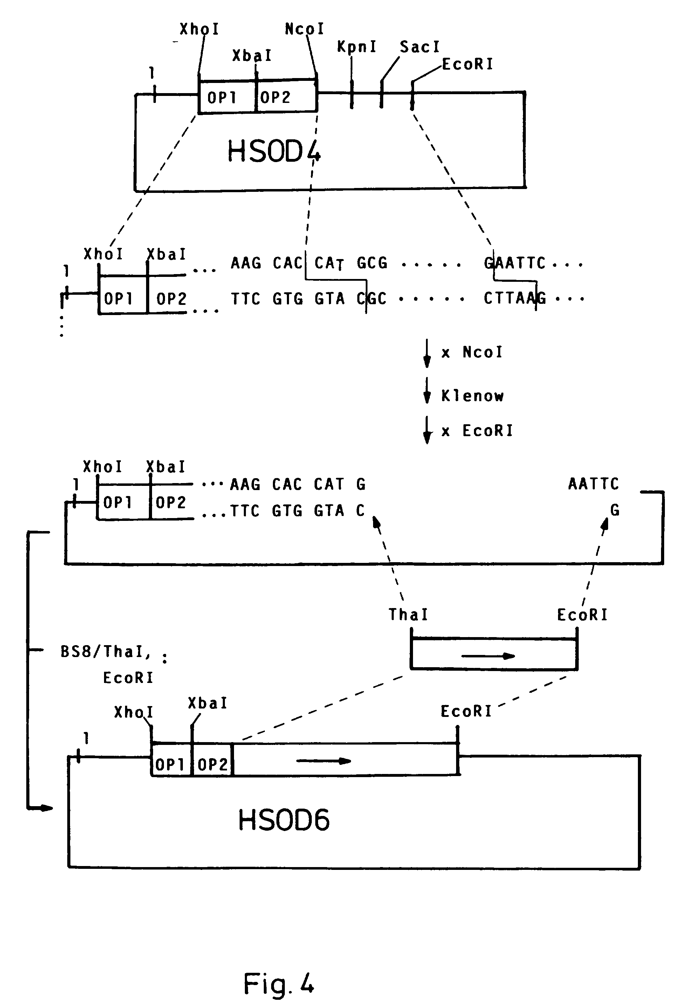

Nach Restriktion mit ThaI (10 ug Plasmid wurden in 40 ul ThaI-Puffer mit 25 Einheiten ThaI 8 Stunden bei 60°C verdaut), Nachschneiden des 759 bp Thal Fragments mit EcoRI (s.o.), mit jeweils nachfolgender gelelektrophoretischer Reinigung und Isolation des entsprechenden Fragments, wurde das so erhaltene Thal/ EcoRI-Fragment (Fig. 4) mit dem entsprechend vorbereitetem Plasmid HSOD4 (ca. 100 ng Fragment wurden mit 50 ng geschnittenem Vektor in 10 ul Ligationslösung ligiert. (s.o.)) zu HSOD6 (Fig. 5) kombiniert. Plasmid HSOD6 enthält somit die vollständige c-DNA für hMn-SOD inklusive Met. Der Leserahmen bleibt dabei erhalten.

b. Konstruktion der Expressionskassette

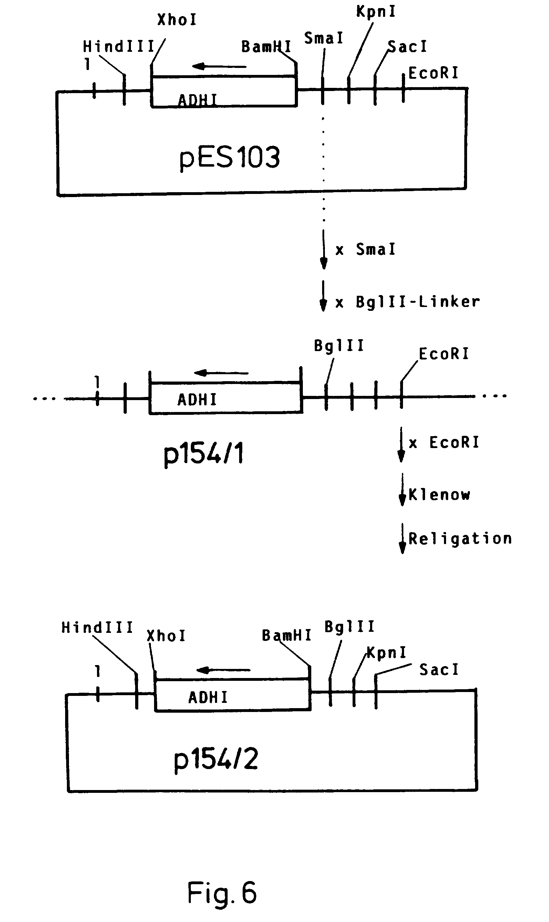

Plasmid HSOD6 wurde mit Xhol und EcoRI (je 5 Einheiten/ug DNA) in CORE-Puffer doppelt verdaut, das Xhol-Fragment (Gen) isoliert und in das Plasmid PKH1 bzw. PKH2 über Xhol/EcoRI insertiert. Die Plasmide PKH1 bzw. PKH2 wurden wie folgt präpariert (Fig.6, 7, 8): In Plasmid PES 103, das den ADHI-Promotor als 1500 bp BamHI-Xhol-Fragment in PES 102 (PES 102 ist ein pUC18 Derivat, welches in der HincII-Schnittstelle einen Xhol-Linker enthält, die Konstruktion des BamHI-Xhol-Fragments ist in Methods in Enzymology 101, 192-201 beschrieben) enthält, wurden nach Smal-Restriktion (1 ug Plasmid wurde mit 5 Einheiten Smal in Smal-Puffer für 2 Stunden bei 37°C verdaut), Reinigung und Isolation BglIII-Linker insertiert (T. Maniatis et al., 1982, p 396). Das so entstandene Plasmid (P154/1, Fig. 6) wurde durch EcoRI-Restriktion (s.o.), Klenow-Fill-in (s.o.) und Religation (lug DNA wurde in 40 ul Ligationslösung (s.o.) über Nacht bei 14°C inkubiert) zu Plasmid 154/2 verändert (Fig. 6).

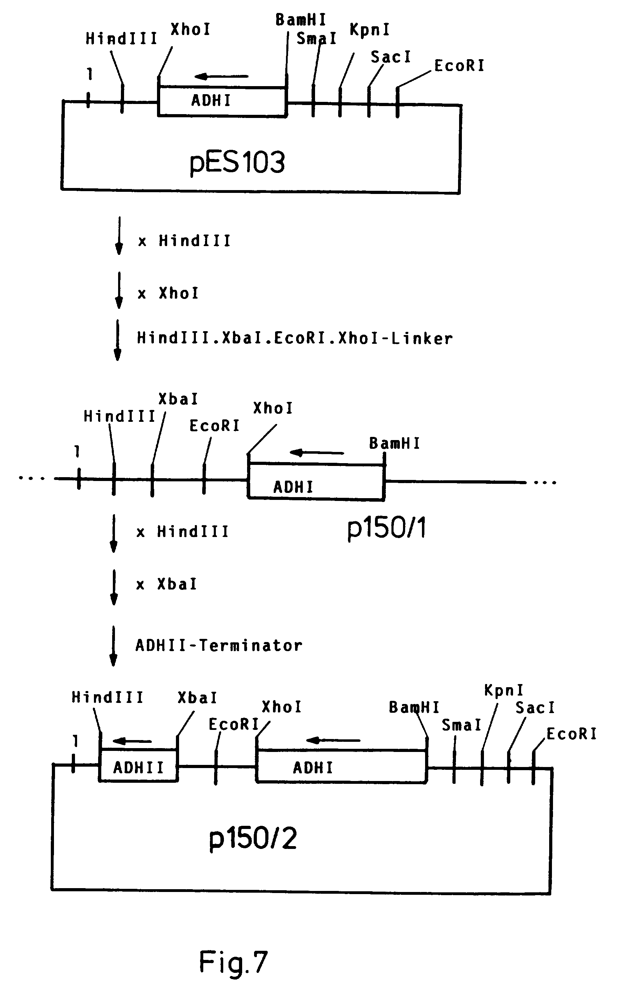

Ebenfalls ausgehend von Plasmid pES103 wurde nach doppelter Verdauung mit Xhol und HindIII in CORE-Puffer der Linker -Xhol.EcoRI.Xbal.HindIII- (Fig. 7, synthetisiert am 381A DNA-Synthesizer) insertiert. Dieser Linker enthält die Sequenz

**TCGAGGAATTCTCTAGAA**

**CCTTAAGAGATCTTTCGA.**

In das so erhaltene Plasmid 150/1 wurde über Xbal/HindIII (doppelte Verdauung in CORE-Puffer) der ADHII Terminator insertiert (Plasmid 150/2 (Fig.7)). Der ADHII-Terminator wurde wie folgt erhalten: Plasmid pMW5 ADHII (Washington Research Fundation) wurde mit HindIII (Core-Puffer), danach mit SphI (in SphI-Puffer) verdaut und das isolierte 605 bp Fragment in den Vektor V18 kloniert und in die HincII-Schnittstelle ein Xbal-Linker (Biolabs, CTCTAGAG) eingebaut (Ligation s.o.). Ein 335 bp langes Xbal/SphI Fragment wurde in pUC18 (Xbal/SphI) einligiert (pGD2).

Der Vektor V18 wurde dadurch erhalten, daß in pUC18 in die Smal-Stelle ein HindIII-Linker eingebaut wurde und die HindIII-Stelle am ursprünglichen Ort fehlt, so daß die Multiklonierstelle in V18 EcoRI.Sstl.KpnI.HindIII.BamHI.Xbal.Sall.Pstl.SphI lautet.

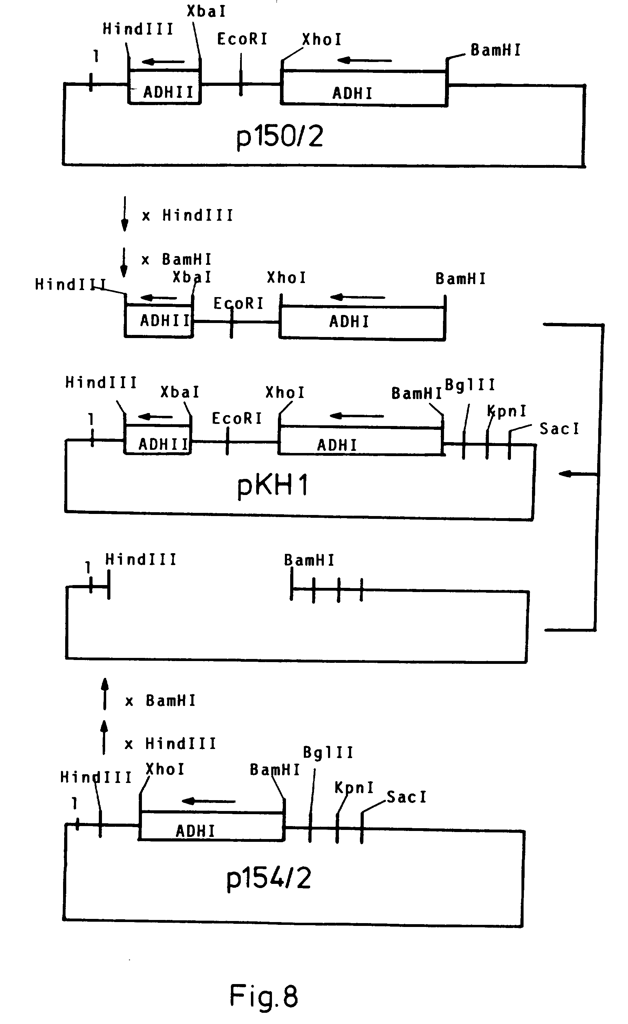

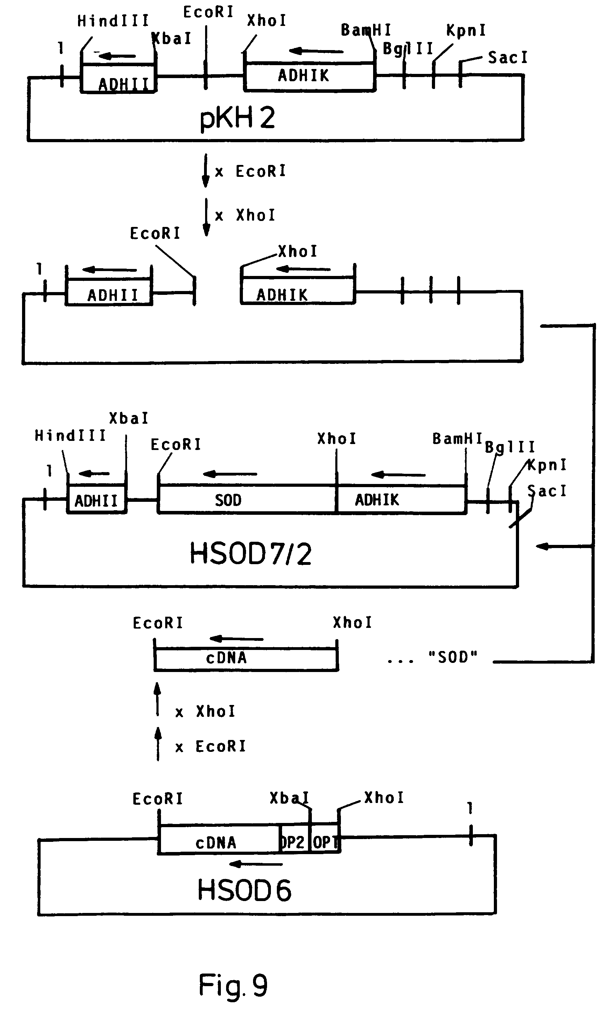

Der ADHII-Terminator wurde schließlich nach doppelter Verdauung mit Xbal/HindIII in CORE-Puffer nach den üblichen Methoden (s.o.) isoliert. Plasmid 150/2 enthält somit bis auf das über Xhol/EcoRI zu insertierende Gen die für eine Genexpression notwendigen Einheiten von ca. 1500 bp (Promotor), 7 bp (Xhol-Linker), 6 bp (EcoRI-Linker), 7 bp (Xbal-Linker), 329 bp (Terminator). Diese Einheiten wurden nun über BamHI/HindIII (doppelte Verdauung in CORE-Puffer) in den Vektor 154/2 (Fig. 8) inseriert. In dem so erhaltenen Plasmid PKHI (Fig.8) wurde in analoger Weise der ADHI-Promotor durch den verkürzten Promotor ADHIk als BamHI/Xhol-Fragment (412 bp) ersetzt (pKH2, Fig. 9).

In beide Plasmide wurde schließlich über Xhol/EcoRI (s.o.) das aus HSOD6 herausgeschnittene vollständige cDNA-Gen (s.o.) insertiert. Die so erhaltenen Plasmide HSOD7/1 bzw. HSOD7/2 (Fig. 9 zeigt nur HSOD7/2) unterscheiden sich voneinander nur durch die verschiedenen Promotoren ADHI und ADHIk (s.o.). Die so fertiggestellten Expressionskassetten wurden über BglIII/HindIII (nach doppelter Verdauung der Plasmide in CORE-Puffer und Isolation der herausgeschnittenen Expressionskassetten) in die entsprechend vorbereiteten und öffentlich erhältlichen Hefetransformationsvektoren YEp13 (J.R. Broach et al., Gene 8, 121-133, 1979, ATCC 37115), pJDB207 (DSM 3181, hinterlegt am 28.12.84), pEAS102 (siehe oben), YIp5 (K. Struhl et al., Proc. Natl.,Acad.Sci. USA 76, 1035-1039, 1979, ATCC 37061) über die Schnittstellen BamHI und HindIII insertiert.

Beispiel 7. Herstellung einer für die Expression geeigneten Hefe Mn-SOD-Mutante

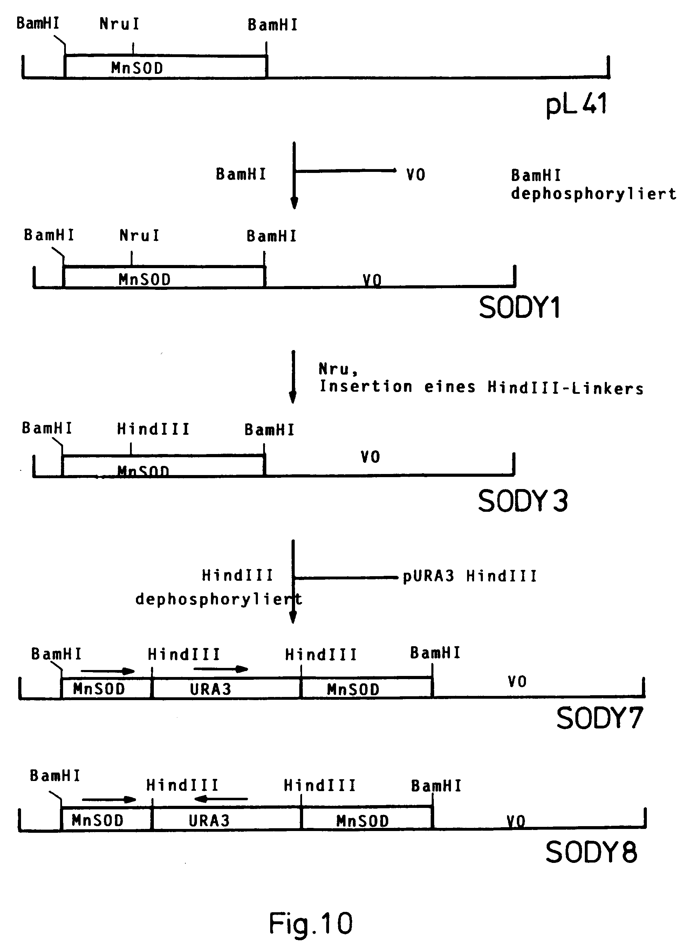

Das Gen für Hefe-Mn-SOD (A.P.G.M. van Loon et al., Gene 26, 261-272, 1983) ist als BamHI-Fragment im Vektor PL 41 (Fig.10) enthalten und die Sequenz vollständig publiziert (C.A.M. Marres et al. Eur.J.Biochem.147, 153 - 161, 1985). Nach Restriktion mit BamHI (2 ug Plasmid wurden mit 5 Einheiten in 150 mM NaCl, 6 mM Tris-HCl pH 7.9. 6 mM $MgCl_2$, 100 ug/ul Rinderserumalbumin 2 Stunden bei 36°C verdaut) wurde das 2045 bp lange BamHI-Fragment, das das Gen enthält, wie üblich durch Gelelektrophorese gereinigt und isoliert und über BamHI in den Vektor VO (pUC18, jedoch ohne HindIII-Schnittstelle) subkloniert.

Der Vektor VO wurde dadurch erhalten, indem 1 μg pUC18 mit HindIII geschnitten wurde (CORE-Puffer), das linearisierte Fragment aus dem Gel nach bekannten Methoden isoliert, die überstehenden Enden mit 2 U Klenow-Polymerase (Ligasepuffer + 0.2 mM dNTP) aufgefüllt und nach 30 min bei RT durch Zugabe von 2 U T4-DNA-Ligase über Nacht bei 14°C regligiert wurde.

Das entstandene Plasmid SODY1 (Fig.10) wurde durch NruI-Restriktion (1 ug Plasmid wurde mit 5 Einheiten NruI in NruI-Puffer 2 Stunden bei 36°C verdaut) durch Gelelektrophorese gereinigt und durch Insertieren eines HindIII-Linkers (CAAGCTTG) zu SODY3 (Fig. 10) verändert. Schließlich wurde in die HindIII-Schnittstelle das URA3-Gen (erhalten aus pURA3) insertiert: 4 ug SODY3 wurden mit 20 Einheiten HindIII 2 Stunden bei 37°C in CORE-Puffer verdaut und dephosphoryliert:

Zu 40 ul Verdauungsansatz wurden 40µl $H_2O$, 10µl 1 mM EDTA, 5µl 1M Tris-HCl pH9.5, 1 µl 100 mM Spermidin 1 ul Calf Intestinal Alkaline Phophatase (CIAP, 1 mg/ml H 2 O) zu gegeben und bei 36°C inkubiert.Nach 15 min wurde nochmals 1 ul CIAP zugegeben und weitere 15 min inkubiert. Der dephosphorylierte Vektor wurde ebenfalls durch Agarosegelelektrophorese gereinigt. 2 ug Plasmid pURA3 wurden mit HindIII geschnitten (siehe oben) und ein 1,2 kb Fragment, welches das Hefegen URA3 enthält, ebenfalls isoliert und in den vorbereiteten Vektor insertiert (s.o.).

Die so entstandenen Plasmide SODY7 und SODY8 enthalten das URA3-Gen innerhalb des Hefe-Mn-Gens und unterscheiden sich in der Orientierung des URA-Gens relativ zum Mn-SOD-Gen (Fig.10).

Die Orientierung des URA3-Gens relativ zum Mn-SOD-Gen ist feststellbar, da das URA3-Gen eine asymetrische PstI-Stelle enthält.

Mit dem Plasmid SODY7 und SODY8 wurden im Stamm DBY 747 (Genotyp a, leu2, his3, trpl, ura3, Yeast Genetic Stock Centre, Berkeley) ein "Gen-Transplacement" durchgeführt (Methods in Enzymology 101, 202-211 und 211-228). Der Stamm DBY 747 wurde mit dem BamHI-Fragment aus SODY7 und SODY8 transformiert (J.D. Beggs, Nature 275, 104, 1978). Dazu wurden 20 ug SODY7 bzw. SODY8 mit 50 U BamHI in 200 ul BamHI-Puffer (150 mM NaCl, 6 mM TrisHCl pH 7.9, 6 mM $MgCl_2$, 1 mM DTT) geschnitten und die gesamte Verdauungsmischung (ohne den pUC-Anteil abzutrennen) mit Phenol extrahiert (Maniatis, T. et al., Molecular Cloning, 1982, S. 458f) und durch Äthatnolfällung konzentriert (Zusatz von 20 ul 3 M Natriumacetat pH 5.5, 500 ul Äthanol). Die DNA wurde in 10 ul Wasser aufgenommen und direkt zur Transformation von Hefe eingesetzt.

Die Transformanten wurden auf Uracilprototrophie selektioniert.

Einzelne Transformanten wurden über Nacht in 5 ml SC-URA-Medium bei 28°C gezüchtet. Die Zellen wurden durch Zentrifugation geerntet, nach van Loon et al. aufgeschlossen (Proc.Natl.Acad.Sci.USA 83, 3820-3824, 1986) und auf ihren Gehalt an Mn-SOD untersucht. Zur gelelektrophoretischen Erfassung von Mn-SOD einerseits und Cu/Zn-SOD andererseits wurden bereits bestehende Arbeitsvorschriften (Ch. Beauchamp und I. Fridovich, Anal. Biochem.44, 276-287, 1971; H.P. Misra und I. Fridovich, Arch.Biochem.Biophys. 183, 511-515, 1977; B.J. Davis, Annals of the NY Academy of Sciences Vol. 121, 404-427, 1964) zur Anwendung gebracht. Bestens bewährt hat sich dabei die Auftrennung der Proteine mit anschließender Negativ-Färbung mit Nitroblautetrazolium (B.J. Davis, 1964; Ch. Beauchamp und I. Fridovich, 1971). Eine Erhöhung der Empfindlichkeit ist durch die Anfärbung mit Dianisidin möglich (H.P. Misra und I. Fridovich, 1977). Zur weiteren Charakterisierung wurde ein spektrophotometrischer Assay (Hyland, K. et al. Anal. Biochem. 135. 280-287, 1983) mit alkalischem Dimethylsulfoxid als $O_2^{\cdot}$ - erzeugendes System und mit Cytochrom c als "scavenger" eingesetzt.

Die Unterscheidung zwischen Mn-SOD einerseits und Cu/Zn-SOD andererseits erfolgt durch Zusatz von KCN (s.o. und M. Ysebaert-Vanneste and W.H. Vanneste, Anal.Biochem.107, 86-95, 1980). Die Stämme SODY7/2, SODY7/6, SODY7/8 und SODY7/10 enthielten keine Mn-SOD Aktivität.

Beispiel 8. Herstellung der Expressionsvektoren

Die unter Beispiel 6b beschriebenen Expressionskassetten wurden als BglII/HindIII-Fragmente aus den Plasmiden HSOD7/1 bzw. HSOD7/2 herausgeschnitten (jeweils 2 µg Plasmid-DNA im Core-Puffer, 2 Stunden bei 37°C mit je 10 U Enzym). Ebenso wurde je 1 µg YEp13, pJDB207 und pEAS102 mit HindIII-BamHI geschnitten (Verdauungsbedingungen wie oben beschrieben).

Je 50 µg Vektor-DNA und 200 ug Insert wurden in Ligasepuffer (wie beschrieben) mit 1 U Ligase über Nacht bei 14°C ligiert und zur Transformation des E.coli-Stammes HB101 eingesetzt. In der folgenden Tabelle ist die Bezeichnung der entsprechenden Plasmide angegeben. Tabelle 1: Benennung der Expressionsvektoren

| Vektor | Insert: HSOD7/1 | HSOD7/2 |
|--------|-----------------|---------|
| YEp13 | pWS550A | pWS371 A |
| pJDB207 | pWS490A | pWS372 A |

Fortsetzung der Tablelle auf der nächsten

(fortgesetzt)

| Vektor | Insert: HSOD7/1 | HSOD7/2 |
|--------|-----------------|---------|
| pEAS102 | pWS491A | pWS373 A |

Beispiel 9. Herstellung eines zur Transformation geeigneten Hefestammes (WS30-5g)

Es wurde ein Hefestamm hergestellt, der neben den für den Hefestamm SODY7/2 beschriebenen genetischen Markern zusätzlich eine Mutation in einer der lysosomalen Hauptproteasen (die durch ihre Aktivität andere lysosomale Proteasen aktivieren kann) enthält und somit beim Aufbrechen der Hefezellen weniger Proteasen freisetzt (Mutation pep4)(E.W. Jones et al., Genetics 102, 665-677, 1982).

Dazu wurde der MnSOD-defiziente Stamm SODY7/2 mit dem proteasedefizienten Stamm WS20-25 (α leu2 his3 trp1 ura3 pep4) gekreuzt und die daraus resultierenden Haploiden auf ihre genetischen Marker hin untersucht (F. Sherman et al., Methods in Yeast Genetics, Cold Spring Harbor, N.Y.,1972).

Der erhaltene Stamm WS30-5g (leu2 his3 trpl pep4 sodl) ist sehr gut transformierbar und erfüllt die gewünschten Bedingungen.

Derartige Kreuzungen können auch mit anderen gut bekannten und erhältlichen Hefestämmen, beispielsweise mit 20 B-12 (Yeast Genetic Stock Center, Berkeley) mit ähnlich guten Resultaten durchgeführt werden.

Beispiel 10. Hefetransformation und Expression in Hefe

Der Hefestamm SODY7/2 wurde mit den Plasmiden pWS371A, pWS372A bzw. pWS373A transformiert (J.D. Beggs, Nature 275, 104-109, 1978) und die Transformanten auf ihre Expression hin untersucht.

Dazu wurde eine Vorkultur der Transformanten in SC-leu-Flüssigmedium (analog dem beschriebenen SC-URA-Medium, enthält aber zusätzlich 2.4 g Uracil, jedoch ohne Leucin) hergestellt (Schütteln bei 300 rpm, 28°C, über Nacht). Davon wurden 100 ul in 4 ml YP5%D (1% Bacto Yeast Extract, 2% Bacto Pepton, 5% Glucose) inokuliert und über Nacht gezüchtet (wie die Vorkultur). Die Zellen wurden geerntet und aufgeschlossen wie unter Beispiel 7 bereits beschrieben. Auf das Aktivitätsgel wurde die Menge Zellrohsaft, die 1 ml Kultur entspricht, aufgetragen. Der Aktivitätstest wurde wie unter Beispiel 7 beschrieben, durchgeführt.

Der Hefestamm WS30-5g (leu2 his3 trpl pep4 sodl) wurde mit den Plasmiden pWS550A, pWS490A, pwS491A transformiert. Die Herstellung der Vorkultur, Kultur und die Messung der hMn-SOD-Aktivität erfolgten wie oben beschrieben.

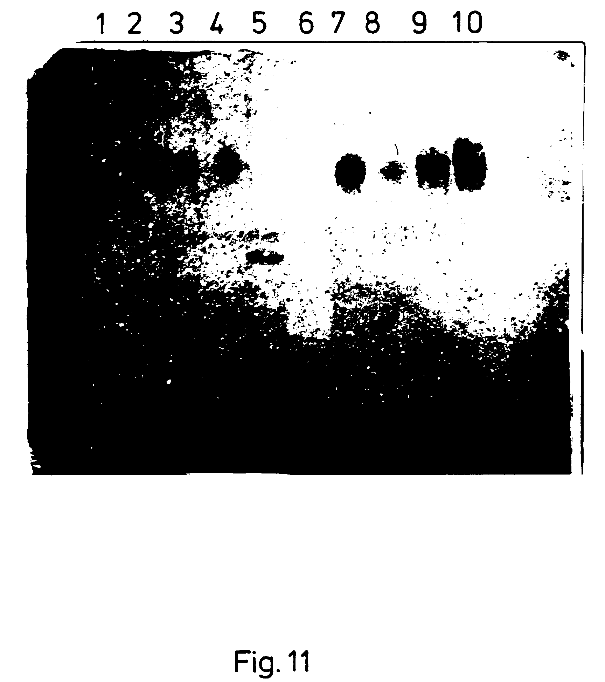

Die Expression der Plasmide pWS490A, pWS491A im Hefestamm WS30-5g dokumentiert Fig. 11.

Die in der Hefe unter diesen Bedingungen gemessenen MnSOD-Menge entsprach ungefähr 0,5 mg/Liter Kultur.

Beispiel 11. Synthese eines die Hefe-Leader DNA Sequenz enthaltenden Linkers

Es wurden sechs verschiedene Oligonukleotide EBI 656, EBI 636, EBI 643, EBI 646, EBI 660 und EBI 638 folgender Sequenzen und Längen

**EBI 656:**
5´ TCGAGTATACAATGTTCGCGAAAACAGCTGCAGCTAATTTA³     41bp

**EBI 636:**
5' TCTTGGTTAAATTAGCTGCAGCTGTTTTCGCGAACATTGTATAC³´    44bp

**EBI 643:**
5´ ACCAAGAAGGGTGGTTTGTCATTGCTCTCCACCACAGCAAGGAGAACC³  48bp

**EBI 646:**
5´ AGTGCTTGGTTCTCCTTGCTGTGGTGGAGAGCAATGACAAACCACCCT³ **48bp**

**EBI 660:**
5´ AAGCACTCTTTGCCAGACTTGCCATACGACTACGGTGCT³´ **39bp**

**EBI 638:**
5´ CTAGAGCACCGTAGTCGTATGGCAAGTCTGGCAAAG³´ **36bp**

über einen 381 A DNA-Synthesizer (Applied Biosystems), wie unter 3b. beschrieben, hergestellt.

Die Oligonukleotide EBI 636, EBI 643, EBI 646 und EBI 660 wurden für die anschließende Ligasereaktion jeweils an ihren 5'-Enden unter folgenden Bedingungen phosphoryliert:

| Reaktionsansatz Nr. 1 | 2μl EBI 636 (=l00pMol) |
| | 1μl 10 x Linker Kinase Puffer |
| | 3μl 10mM ATP |
| | 1μl T4 Polynukleotid Kinase, |
| | Biolabs 10U/μl |
| | 3μl Wasser |

Reaktionsansatz Nr.2 — analog zu Nr. 1, aber mit 2μl (100 p Mol) EBI 660

| Reaktionansatz Nr.3 | 2μl Oligonukleotid EBI 643 (=100 pMol) |
| | 2μl Oligonukleotid EBI 646 (=100 pMol) |
| | 1μl 10 x Linker Kinase Puffer |
| | 3μl 10mM ATP |
| | 1μl T4 Polynukleotid Kinase (10 Units) |
| | 1μl Wasser |

10 x Linker Kinase Puffer : 0,7 M Tris.Cl pH 7,6
0,1M $MgCl_2$
0.05M DTT (Dithiotreitol)

Die Reaktion erfolgte 30 Minuten lang bei 37°C. Anschließend wurde die T4 Polynukleotid Kinase durch Erhitzen auf 100°C inaktiviert.

Die Oligonukleotide EBI 656 und EBI 638, welche die 5'-Enden des fertigen 128bp langen DNA Inserts bilden sollen (Formel XI), wurden nicht phosphoryliert, um die Ausbildung multimerer DNA-Inserts in der anschließenden Ligasereaktion zu vermeiden.

Um eine Zusammensetzung des gewünschten Linkers aus den einzelnen Oligonukleotiden nach dem Schema

5´ **EBI656** P **EBI643** P **EBI660** 3´

3´ **EBI636** P **EBI646** P **EBI638** 5´

zu erreichen, wurden für die Annealingreaktion (Hybridisierung der komplementären Oligonukleotide miteinander) zum Reaktionsansatz Nr.1 2μl (=100pmol) EBI656 und zum Reaktionsansatz Nr.2 2μl EBI 638 (=100pMol) hinzugefügt. Im Reaktionsansatz Nr.3 sind bereits 2 komplementäre Oligonukleotide (EBI 643, EBI 646) enthalten. Alle 2 Reaktionsansätze wurden 2 Minuten lang bei 100°C erhitzt und langsam im Wasserbad abgekühlt.

Die in den Reaktionen Nr.1 bis Nr.3 entstandenen kurzen doppelsträngigen DNA Fragmente wurden wie folgend miteinander ligiert:

| 10µl | Reaktionsansatz | Nr.1 (EBI 636 + EBI 656) |
|---|---|---|
| 10µl | Reaktionsansatz | Nr.2 (EBI 660 + EBI 638) |
| 10µl | Reaktionsansatz | Nr.3 (EBI 643 + EBI 646) |
| 3µl | 10mM ATP | |
| 1µl | DNA Ligase, Boehringer Mannheim, 7Units/µl | |

Die Reaktion erfolgte 15 Stunden lang bei 4°C.

Die DNA wurde ihre Größe nach einem 1-prozentigen Agarosegel aufgetrennt und das gewünschte 128bp lange DNA Fragment gemäß Formel XI vom Gel eluiert (G.M. Dretzen et al., Anal. Biochem. 112, 295-298, 1981).

Beispiel 12. Konstruktion der die Leader-DNA Sequenz enthaltenden Expressionsvektoren

Plasmid HSOD6 wurde wie üblich mit XhoI und XbaI (je 5 Einheiten/µg DNA) im CORE-Puffer doppelt verdaut und der 128bp lange Linker (XhoI - mitochondrialer Leader - XbaI) nach bekannten Methoden darin insertiert (pEO22-A). Das nun mit der mitochondrialen Hefe Leader DNA Sequenz versehene hMn-SOD Gen wurde mit XhoI - EcoRI (je 5 Einheiten/µg DNA) im CORE-Puffer doppelt verdaut und über XhoI - EcoRI in pKHI (Beispiel 6b, Fig. 8) insertiert (pE023-A).

Die so fertiggestellte Expressionkassette wurde analog Beispiel 8 über BglII/HindIII (nach doppelter Verdauung der Plasmide in CORE-Puffer und Isolation der herausgeschnittenen Expressionskassette) in die entsprechend vorbereiteten Hefetransformationsvektoren YEp13, pJDB207 und pEAS102 über die Schnittstellen BamHI und HindIII insertiert. Die folgende Tabelle 2 bezeichnet die entsprechend erhaltenen Plasmide.

Tabelle 2: Benennung der Expressionsvektoren

| Vektor | Plasmidbezeichnung |
|---|---|
| PJDB207 | pEO24-AB |
| pEAS102 | pEO25-AC |
| YEp13 | pEO26-AD |

Beispiel 13. Hefetransformation und Expression in Hefe

Der Hefestamm WS30-5g (Beispiel 9) wurde mit den in Tabelle 2 angeführten Plasmiden transformiert und die Transformanten auf ihre Expression hin untersucht (Beispiel 10).

Für die Fermentation des transformierten Hefestamms WS30-5g wurde eine Vorkultur der Zusammensetzung: 6,7 g/l yeast nitrogen base w/o amino acids (Difco), 10g/l Glucose, 0,16g/l Arginin, 0,25g/l Lysin: 0,06 g/l Tryptophan, 0,08 g/l Methionin, 0,03g/l Cystein, 0,10g/l Histidin, 0,16 g/l Tyrosin, 0,17 g/l Phenylalanin, 0,16 g/l Threonin, 0,18 g/l Isoleucin, 0,21 g/l Valin, 0,40 g/l Glutaminsäure, 0,21 g/l Glycin, 0,02 g/l Cystin, 0,15 g/l Alanin, 0,20 g/l Asparaginsäure, 0,20g/l Prolin, 0,15g/l Serin, 0,10g/l Asparagin, 0,20g/1 Glutamin, 25mg/l Adenin, 50mg/l Uracil mit Magnetrührstab und unter Belüftung bis zur optischen Dichte $OD_{546} = 0.01$ vorgezüchtet.

Die anschließende Hauptkultur mit der Zusammensetzung:

8,0g/l $(NH_4)_2$ $SO_4$, 2,56g/l $(NH_4)_2 HPO_4$, 1,16g/l KCl, 0,60g/l $MgSO_4$ . 7 $H_2O$, 0,56g/l $CaCl_2$ . $2H_2O$, 0,04 mg/l Biotin, 80 mg/l m-Inosit, 40 mg/l Ca-Pantothenat, 8mg/l Thiamin, 2mg/l Pyridoxin, 3,1mg/l $CUSO_4$ . 5 $H_2O$, 19mg/l $FeCL_3$.6 $H_2O$, 12mg/l $ZnSO_4$.7 $H_2O$, 14mg/l $MnSO_4.H_2O$, 5mg/l $H_3BO_3$, 1mg/l KJ, 2mg/l $Na_2$ $MoO_4$.2 $H_2O$, lg/l Hefeextrakt, 0,2g/l Uracil, 0.1g/l Adenin, 0,5g/l Citronensäure, 15g/l Glutaminsäure, 0,2g/l Histidin, 0,5g/l Tryptophan, 100 g/l Glucose erfolgte im 20l Fermenter (CHEMAP). Für diesen Zweck wurde als Inoculum 5% der Menge der Vorkultur verwendet und unter Rühren (1000 rpm), Belüften (0.5 vvm) und konstantem ph (5.0) bei 28°C im 20l Fermenter gezüchtet.

Nach Absinken des Glucosegehalts auf 50g/l wurden nochmals 50g/l Glucose zugegeben und weiterfermentiert, bis der Glucosegehalt 10g/l betrug (was bei 45 Stunden der Fall war). Die Fermentationsbrühe wurde daraufhin abgekühlt. zentrifugiert und die Biomasse eingefroren. Die Ausbeute an Biomasse betrug 18g/l Zellnaßgewicht.

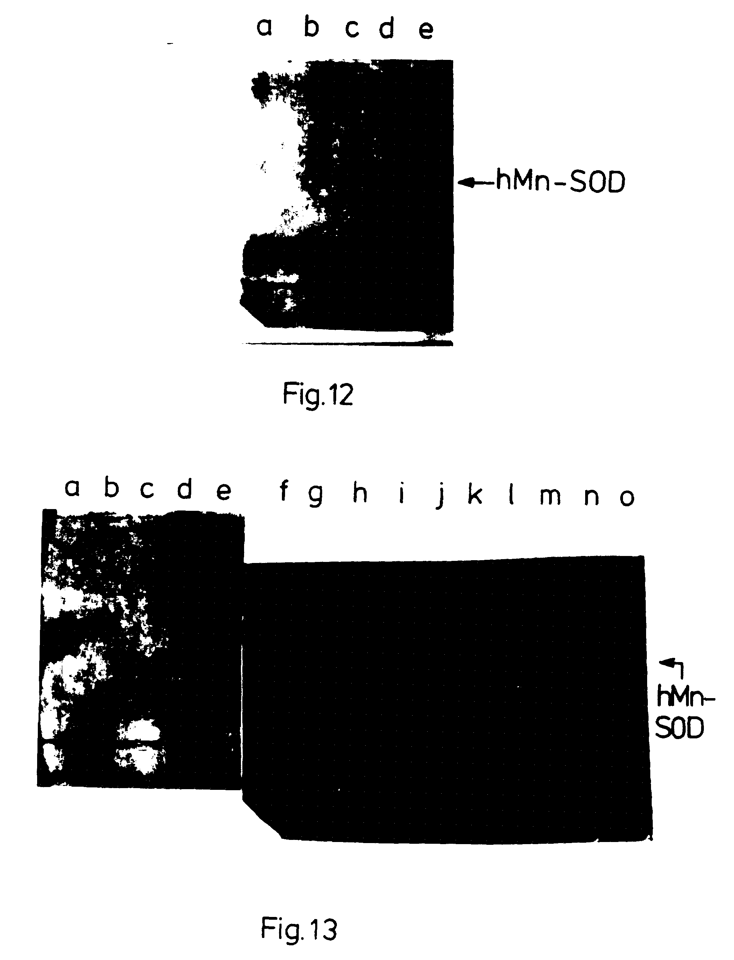

Die Expression der Plasmid pEO24-AB, pEO25-AC und pEO26-AD im Hefestamm WS30-5g dokumentiert Fig.12.

Beispiel 14. Hefe-Mitochondrien Präparation

Um festzustellen, ob das Einführen der Hefe-mitochondrialen Leader Sequenz vor das hMn-SOD Gen dazu führt, daß das Protein in die Mitochondrien importiert wird, wurden Hefe Mitochondrien präpariert und die Mn-SOD Aktivität in den Mitochondrien sowie im Cytoplasma analysiert.

Hefe Mitochondrien wurden nach einer abgeänderten Form der Methode von G. Daum et al., The Journal of Biol.

Chem., 257, 13028-13033, 1982, präpariert.

Eine Vorkultur der Transformanten in SC-leu-Flüssigmedium (Beispiel 10) wurde durch Schütteln (300rpm) bei 28°C über Nacht gezüchtet. Davon wurden 25ml in 225ml YPD-Medium inokuliert und wie die Vorkultur über Nacht gezüchtet. Die Zellen wurden im allgemeinen bei einer optischen Dichte 5 - 7, bei 600nm gemessen, durch Zentrifugation (Sorval, 6500rpm, 5 Min.) geerntet. Die Zellen wurden 1 Mal mit 100ml Wasser gewaschen.Das Zellpellet wurde in 1M Mannit,20mM $KP_i$ ($KH_2PO_4$/$K_2HPO_4$) pH 7,4 suspendiert (1ml pro 300 mg Zellgewicht) und 1mg/ml Zymolase (Miles, MG.500) hinzugefügt. Spheroplasten wurden durch zweistündiges langsames Schütteln (50 rpm) bei 28°C erzeugt. Die Spheroplasten wurden durch Zentrifugation geerntet (3000 rpm, 5 Min., Hereaus Christ Tischzentrifuge) und 1 Mal mit 1M Mannit, 20mM $KP_i$ pH 7,4, 1mM PMSF (Phenylmethylsulfonylfluorid) gewaschen. Der Überstand wurde verworfen und 1 - 2 Pelletvolumina an Glasperlen (Durchmesser 0,1 mm) hinzugefügt.

Die Zellen wurden durch 1-minütiges Rühren aufgebrochen und in 2,5 ml 0,65M Mannit, 1mM EDTA, 1mM PMSF suspendiert. Ganze Zellen und Zelltrümmer wurden bei 2000 rpm, 5 Minuten lang (Hereaus Christ Tischzentrifuge) abzentrifugiert. Die Mitochondrien wurden anschließend durch Zentrifugation (Sorval, J-21, 12000rpm, 10 Min.) aus dem Überstand gewonnen. Der Überstand enthält das Zytoplasma und wurde aufgehoben, um später auf hMn-SOD Aktivität untersucht zu werden. Das rotbraune Mitochondrienpellet wurde mit dem oben angeführten Puffer gewaschen (weisse zytoplasmatische Bestandteile wurden weggespült) und die Mitochondrien in 2,5ml des gleichen Puffers suspendiert. Verunreinigungen wurden nochmals durch Zentrifugation (Hereaus Christ, Tischzentrifuge, 4000rpm, 5Min.) entfernt und die Mitochondrien in einer zweiten Zentrifugation (Sorval, J-21, 12000rpm, 10Min.) aus dem Überstand pelletiert. Die Mitochondrien wurden mit Glasperlen aufgebrochen, analog der Methode, um Hefezellen aufzubrechen (van Loon et al., Proc.Natl. Acad.Sci.USA 83, 3820-3824, 1986), und im Aktivitätsgel auf ihren Gehalt an Mn-SOD untersucht (Fig. 13).

Beispiel 15. Reinigung der erfindungsgemäßen hMn-SOD

Die Isolierung der rekombinanten hMn-SOD erfolgte aus dem Stamm WS30-5g/pEO24-AB (Hefevektor PJDB207) über mehrere Schritte.

Schritt 1: Zellaufschluß

Die Zellmasse (Beispiel 13) wurde in 10 ml destilliertem Wasser pro Gramm Naßgewicht gewaschen und bei 16 000 x g, 15 Minuten zentrifugiert. Der Niederschlag wurde in Na, K-Phosphatpuffer (50 mM, pH 7,0) im Verhältnis 1:3 (w/v) resuspendiert. Die Zellen wurden dann in einer kontinuierlich arbeitenden Zellmühle (Dynomill KDL; Bachofer, Basel, Schweiz; 0,6 1-Mahlbehälter, Wasserkühlung) mit Hilfe von Glaskugeln (0,1 mm Durchmesser) bei einer Flußrate von 6 l/h aufgeschlossen. Der Zellextrakt wurde 15 Minuten zentrifugiert (16 000 x g, 4° C) und der Niederschlag verworfen.

Schritt 2: Polyethylenimin-Fällung

Zum Überstand aus Schritt 1 wurde eine 5 prozentige (w/v) wässrige Polyethyleniminlösung (pH 8,0) unter Rühren bis zu einer Endkonzentration von 0,5 % zugesetzt (Polyethylenimin, Serva, Heidelberg). Danach wurde noch 30 Minuten weitergerührt und dann der Niederschlag bei 16 000 x g (30 Minuten) abzentrifugiert.

Schritt 3: Hitzefällung

Der Überstand aus Schritt 2 wurde in Stahlbechern unter Rühren in einem 80° C heißen Wasserbad auf 60° C erhitzt und im Eisbad wieder auf Raumtemperatur abgekühlt. Ausgefälltes Protein wurde durch Zentrifugation (10 000 x g, 10 min, 4° C) entfernt.

Schritt 4: Ammoniumsulfat-Fällung

Der Überstand aus Schritt 3 wurde mit festem Ammoniumsulfat auf 20 % Sättigung gebracht und das Präzipitat durch Zentrifugation (10 000 x g, 15 min, 4° C) entfernt. Die Ammoniumsulfatkonzentration wurde dann auf 90 % erhöht und der Niederschlag durch Zentrifugation (10 000 x g, 15 min, 4° C) gewonnen. Das Sediment wurde in wenig MES-Puffer (Morpholinoethansulfonatpuffer, 50 mM, pH 6,0; 2-Morpholinoethansulfonsäure von Sigma, Deisenhofen) augenommen und über Nacht gegen den gleichen Puffer dialysiert.

Schritt 5: Kationenaustauschchromatographie

Eine Mono S Säule (Mono S HR 5/5, Pharmacia, Schweden) wurde mit 5 Säulenvolumen MES-Puffer äquilibriert. Nachdem die Säule mit dem Extrakt aus Schritt 4 beladen war, wurden nicht gebundene Proteine mit 5 Säulenvolumen MES-Puffer ausgewaschen. Die erfindungsgemäße hMn-SOD wurde dann in einem linearen Gradienten von 0 - 50 mM NaCl in MES-Puffer eluiert (20 Säulenvolumen). Fraktionen, die Mn-SOD-Aktivität enthielten, wurden vereinigt und gegen Na, K-Phosphatpuffer (5mM, pH 7,0) dialysiert.

Die hefeeigenen SOD-Enzyme (Mn-SOD, CuZn-SOD) können in diesem Reinigungsschritt abgetrennt werden. Ein Elutionsdiagramm ist in Fig.14 dargestellt.

Schritt 6: Adsorptionschromatographie an Hydroxylapatit

Eine mit Phosphatpuffer (5mM, pH 7,0) äquilibrierte Hydroxylapatitsäule (HA-Ultrogel, IBF, Villeneuve-la-Garenne, Frankreich) wurde mit dem Dialysat aus Schritt 5 beladen und die erfindungsgemäße hMn-SOD mit einem linearen Gradienten (20 Säulenvolumen) von 5 - 300 mM Na,K-Phosphat pH 7,0 eluiert.

Der in den einzelnen Reinigungsschritten erzielte Reinheitsgrad von hMn-SOD wurde durch reduzierende SDS-Polyacrylamidgelelektrophorese verfolgt (Fig. 15).

Beispiel 16. Charakterisierung der erfindungsgemäßen hMn-SOD

Die nach Beispiel 15 gereinigte erfindungsgemäße hMn-SOD wurde über Gelpermeations-HPLC, Reverse Phase HPLC, N-terminale Sequenzierung, SDS-Gelelektrophorese, native Gelelektrophorese und isoelektrische Fokussierung analysiert und mit der natürlichen hMn-SOD verglichen.

a. Gelpermeations-HPLC:

Säule : Waters Protein Pak I 125, 2 x (7,8 x 300 mm), 10 µm Teilchendurchmesser
Eluens: 0,5 M $Na_2SO_4$, 0,02 M $NaH_2PO_4$, pH 7,0, 0,04% Tween 20, 25% Propylenglycol
Fluß: 0,5 ml/min
Detektion: UV-Absorption, 214 nm

Natürliche bzw. erfindungsgemäße hMn-SOD zeigen den Hauptpeak des SOD-Tetrameren bei einem Molekulargewicht von 70.000 bzw. 76.000, wobei die Eichung über vier Standardproteinen erfolgte. Innerhalb des experimentellen Fehlers dieser Methode sind diese Werte als identisch anzusehen.

b. Reverse Phase HPLC

Säule: Bakerbond WP $C_{18}$, 4,6 x 250 mm, 5µm Teilchendurchmesser, 30 nm Porendurchmesser
Eluens A : 0,1% Trifluoressigsäure in Wasser
Eluens B : 0,1% Trifluoressigsäure in Acetonitril
Gradient : 20% B für 2 min, 20 - 68% B in 24 min, 68% B für 10 min, 68- 20% B in 1 min
Fluß: 1,0 ml/min
Detektion: UV-Absocption, 214 nm und 280 nm

Natürliche und erfindungsgemäße hMn-SOD zeigen eine Retentionszeit von knapp 21 min (20.7 bzw 20.9 min).

c. N-terminale Sequenzierung

Sequenziert wurde ein über Reverse Phase HPLC entsalzter Peak von erfindungsgmäßer hMn-SOD. Die Sequenzierung erfolgte mit einem Gasphasensequenator der Firma Applied Biosystems (Modell 470 A) mit dem Programm 02RPTH. Mit einer Ausgangsmenge von 0,8 nM konnte bis zur Aminosäure 20 sequenziert werden. Es wurde 100%ige Übereinstimmung mit der erwarteten Sequenz (aus natürlichem Protein und cDNA) gefunden. Die Leader-Sequenz zum Transport in die Mitochondrien war vollständig abgespalten.

d. SDS-Gelelektrophorese

| | |
|---|---|
| Trenngel: | 15% Acrylamid |
| Stackinggel: | 4% Acrylamid |
| Färbung : | Silberfärbung nach B.R. Oakley et al. (Analyt. Biochem. 105, 361-363, 1980). |
| Gelmaße : | 0.75 mm (8 x 10cm) |
| Laufbedingungen : | 60 min, 150 V |

Die SDS-Gelelektrophorese wurde weitgehend nach der Originalvorschrift von U.K. Lämmli (Nature 227, 680-685, 1970) durchgeführt. Bei der Probenvorbereitung für hMn-SOD wurden die Proben mit DTT als Reduktionsmittel versetzt und aufgekocht. Am SDS-Gel tritt hMn-SOD hauptsächlich als Monomeres mit M etwa 25.000 auf. Je nach Vollständigkeit der Reduktion ist auch das Tetramere bei M etwa 90.000 nachweisbar. Fig. 15 zeigt ein 15%iges SDS Polyacrylamidgel nach Silberfärbung.

e. Native Gelelektrophorese

| | |
|---|---|
| Trenngel: | 7,5% native PAGE nach Davis, B.J. (Ann. NY Acad. Sci. 121, 404-427, 1964) |
| Stackinggel: | 2% Acrylamid + Saccharose |
| Gelmaße: | 0.75mm (8x10cm) |
| Laufbedingungen: | 75 min, 150V (const.) |
| Färbung: | Coomassie Blue nach bekannten Methoden und Aktivitätsfärbung mit o-Dianisidin nach Misra, H.P., Fridovich, I.(Arch. Biochem. Biophys. 183, 511-515, 1977) |

Die nach der Hydroxylapatit-Chromatographie erhaltene erfindungsgemäße hMn-SOD zeigte nach der Elektrophorese sowohl mit Coomassie Blue Anfärbung (aufgetragene Menge an hMn-SOD: 0.3µg) als auch nach Aktivitätsfärbung mit o-Dianisidin (aufgetragene Menge an hMn-SOD: 75, 30 bzw. 15 ng) eine einheitliche und in gleicher Position liegende Bande.

f. Isoelektrische Fokussierung

| | |
|---|---|
| pH-Bereich: | 3.5-9.5 |
| Gelplatten: | LKB, PAGplate (1mm x (9 x 10 cm)) |
| Elektrodenlösungen: | 1 M Phosphorsäure (Anode) |
| | 1 M Natronlauge (Kathode) |
| Kühltemperatur : | 7°C |
| Probenmenge: | 4.0 bzw. 6.5 µg |
| Laufbedingungen: | Präfokussierung 500 Vh |
| | Fokussierung 3000 Vh insgesamt |
| Färbung: | Coomassie Blue, Aktivitätsfärbung mit o-Dianisidin |

Als isolelektrischer Punkt wurde pT= 8.15 ermittelt.

**Patentansprüche**

1. Verfahren zur Herstellung einer rekombinanten, tetrameren, reifen human Mangan Superoxiddismutase (hMnSOD), welche am N-Terminus kein Methionin aufweist, **dadurch gekennzeichnet**, daß

   a) eine DNA mit der Codierungsfunktion für hMnSOD oder Teilsequenzen davon mit einer für eine aminoterminalen Leadersequenz codierenden DNA versehen wird, die zwischen dem Start-ATG und dem ersten für hMnSOD kodierenden Codon positioniert wird,
   b) mit dieser aus Schritt a) erhaltenen Einheit ein für die Expression der hMnSOD geeignetes Expressionsplasmid konstruiert wird,

c) eine geeignete Wirtszelle mit dem aus Schritt b) erhaltenen Expressionsplasmid transformiert wird, sodaß diese die gewünschte hMnSOD intrazellulär zum tetrameren Enzym prozessiert und akkumuliert und

d) die durch die transformierte Wirtszelle synthetisierte und korrekt prozessierte, tertamere hMnSOD isoliert und gereinigt wird.

2. Verfahren nach Anspruch 1, **dadurch gekennzeichnet**, daß die für eine hMnSOD codierende DNA mit einer DNA Sequenz der Formeln Ia, Ib, II, IIIa, IIIb, Va, Vb, VIa, VIb, VIIa, VIIb oder IX unter stringenden Bedingungen hybridisiert, wobei diese DNA-Sequenzen synthetischen, halbsynthetischen oder natürlichen Ursprungs sein können.

3. Verfahren nach Anspruch 2, **dadurch gekennzeichnet**, daß die für eine hMnSOD codierende DNA Sequenz oder Teilen davon ausgewählt sind aus den Formeln Ia, Ib, II, IIIa, IIIb, Va, Vb, VIa, VIb, VIIa, VIIb oder IX oder degenerierte Variationen davon oder Mutationen dieser Sequenzen mit der Codierungsfunktion von hMnSOD.

4. Verfahren nach einem der Ansprüche 1 bis 3, **dadurch gekennzeichnet**, daß die Leadersequenz eine Mikroorganismen spezifische Leadersequenz ist.

5. Verfahren nach Anspruch 4, **dadurch gekennzeichnet**, daß die Leadersequenz eine hefespezifische Leaderseqenz ist.

6. Verfahren nach Ansprüchen 1 bis 4, **dadurch gekennzeichnet**, daß die Leadersequenz eine bakterielle Leadersequenz ist.

7. Verfahren nach Ansprüchen 1 bis 5, **dadurch gekennzeichnet**, daß die Leadersequenz eine mitochondriale Leadersequenz ist.

8. Verfahren nach Anspruch 7, **dadurch gekennzeichnet**, daß die mitochondriale Leadersequenz hefespezifisch ist.

9. Verfahren nach einem der Ansprüche 1 bis 8, **dadurch gekennzeichnet**, daß das Expressionsplasmid im wesentlichen aus den Komponenten Promotor, Initiationssignal, hMnSOD Gen mit Leadersequenz, Stop-Codon, Terminator besteht.

10. Verfahren nach einem der Ansprüche 1 bis 8, **dadurch gekennzeichnet**, daß die transformierte Wirtszelle eine prokaryotische oder eukaryotische Wirtszelle ist.

11. Verfahren nach Anspruch 10, **dadurch gekennzeichnet**, daß die transformierte Wirtszelle eine Hefezelle ist.

12. Verfahren nach einem der Ansprüche 1 bis 11, **dadurch gekennzeichnet**, daß die gewünschte hMnSOD in das Wachstumsmedium oder den periplasmatischen Raum oder in das Mitochondrium sezerniert wird, wobei die gewünschte hMnSOD korrekt zum tetrameren Molekül prozessiert wird.

13. Verfahren zur Herstellung einer rekombinanten, tetrameren, reifen human Mangan Superoxiddismutase (hMnSOD), welche am **N**-Terminus kein Methionin aufweist, **dadurch gekennzeichnet**, daß

a) eine mRNA aus menschlichen Gewebe oder Gewebekulturzellen isoliert und die poly(A)$^+$-RNA präpariert wird,

b) von dieser poly(A)$^+$-RNA eine doppelsträngige cDNA synthetisiert wird und davon eine cDNA-Bank konstruiert wird,

c) aus besagter cDNA-Bank mittels mindestens einer von der Aminosäuresequenz der hMnSOD abgeleiteten DNA-Sonde eine für hMnSOD codierende DNA - Gesamt- oder Teilsequenz identifiziert und isoliert wird,

d) diese DNA-Sequenz gegebenenfalls bis zum Start- oder Stoppcodon komplettiert wird,

e) eine aminoterminale Leadersequenz, vorzugsweise eine Mikroorganismen spezifische, direkt nach dem Startcodon (ATG) des hMnSOD Gens und vor dem ersten für hMnSOD codierenden Codon positioniert wird,

f) mit dieser kompletten, mit einer Leadersequenz ausgestatteten hMnSOD codierenden DNA Sequenz eine geeignete Expressionskassette konstruiert wird,

g) diese Expressionskassette in ein Expressionsplasmid, vorzugsweise in einen für eukaryotische Zellen geeigneten Expressionsvektor bzw. geeignetes Expressionsplasmid, inkorporiert wird,

h) mit diesem Expressionsplasmid, welches das mit einer Leadersequenz ausgestattete hMnSOD Gen enthält, geeignete Wirtszellen, vorzugsweise eukaryotische Wirtszellen, transformiert werden, und
i) die durch diesen transformierten Wirt synthetisierte und korrekt prozessierte hMnSOD aus Mitochondrien extrahiert und anschließend gereinigt wird.

14. Verfahren nach Anspruch 13, **dadurch gekennzeichnet**, daß die abgeleitete DNA-Sonde ausgewählt ist aus den Formeln Va und Vb.

15. Verfahren nach Ansprüchen 13 und 14, **dadurch gekennzeichnet**, daß die aminoterminale Leadersequenz eine hefespezifische Leadersequenz ist.

16. Verfahren nach Ansprüchen 13 bis 15, **dadurch gekennzeichnet**, daß die aminoterminale Leadersequenz eine mitochondriale Leadersequenz ist.

17. Verfahren nach Ansprüchen 13 bis 16, **dadurch gekennzeichnet**, daß die Expressionskassette aus Promotor, Initiationssignal, hMnSOD Gen mit aminoterminaler Leadersequenz, Stopcodon, Terminator besteht.

18. Verfahren nach Ansprüchen 1 bis 17, **dadurch gekennzeichnet**, daß das Expressionsplasmid oder der Expressionsvektor ein Hefevektor ist.

19. Verfahren nach Ansprüchen 13 bis 18, **dadurch gekennzeichnet**, daß die transformierte Wirtszelle eine Hefezelle ist.

20. Verfahren nach einem der Ansprüche 1 bis 19, **dadurch gekennzeichnet**, daß die zu verwendenden Vektoren und Plasmide eine für hMnSOD codierende DNA Sequenzen enthaltende Expressionskassette tragen, prokaryotische und eukaryotische Wirtszellen stabil transformieren können, in einer dieser Wirtszellen replizierbar sind und die darin enthaltende genetische Information für hMnSOD korrekt transkribiert und translatiert wird, wobei die Expressionskassette die Komponenten Promotor, Initiationscodon, aminoterminale Leadersequenz, hMnSOD Strukturgen, Stopcodon und gegebenenfalls Terminator in korrekter Orientierung zur Leserichtung enthält.

21. Verfahren nach einem der Ansprüche 1 bis 20, dadurch gekennzeichnet, daß die für hMnSOD codierende DNA ausgewählt ist aus den Formeln IIIa und IIIb, sodaß eine korrekt prozessierte, tetramere, reife hMnSOD gemäß Formeln IVa und IVb hergestellt wird.

## Claims

1. Process for preparing a recombinant, tetrameric, mature human manganese superoxide dismutase (hMnSOD) which has no methionine at the N-terminus, characterised in that

   a) a DNA with the coding function for hMnSOD or partial sequences thereof is provided with a DNA coding for an aminoterminal leader sequence which is positioned between the start-ATG and the first codon coding for hMnSOD,
   b) using this unit obtained from step a) an expression plasmid suitable for the expression of hMnSOD is constructed,
   c) a suitable host cell is transformed with the expression plasmid obtained in step b), so that this host cell processes the desired hMnSOD intracellularly into the tetrameric enzyme and accumulates it and
   d) the tetrameric hMnSOD synthesised and correctly processed by the transformed host cell is isolated and purified.

2. Process according to claim 1, characterised in that the DNA coding for an hMnSOD is hybridised with a DNA sequence of formulae Ia, Ib, II, IIIa, IIIb, Va, Vb, VIa, VIb, VIIa, VIIb or IX under stringent conditions, whilst these DNA sequences may be of synthetic, semisynthetic or natural origin.

3. Process according to claim 2, characterised in that the DNA sequence coding for hMnSOD, or parts of said sequence, are selected from the formulae Ia, Ib, II, IIIa, IIIb, Va, Vb, VIa, VIb, VIIa, VIIb or IX or degenerate variations thereof or mutations of these sequences with the coding function of hMnSOD.

4. Process according to one of claims 1 to 3, characterised in that the leader sequence is a leader sequence specific to microorganisms.

5. Process according to claim 4, characterised in that the leader sequence is a yeast-specific leader sequence.

6. Process according to claims 1 to 4, characterised in that the leader sequence is a bacterial leader sequence.

7. Process according to claims 1 to 5, characterised in that the leader sequence is a mitochondrial leader sequence.

8. Process according to claim 7, characterised in that the mitochondrial leader sequence is yeast-specific.

9. Process according to one of claims 1 to 8, characterised in that the expression plasmid consists essentially of the components promoter, initiation signal, hMnSOD gene with leader sequence, stop codon and terminator.

10. Process according to one of claims 1 to 8, characterised in that the transformed host cell is a prokaryotic or eukaryotic host cell.

11. Process according to claim 10, characterised in that the transformed host cell is a yeast cell.

12. Process according to one of claim 1 to 11, characterised in that the desired hMnSOD is secreted into the growth medium or the periplasmatic space or into the mitochondrion, the desired hMnSOD being correctly processed into the tetrameric molecule.

13. Process for preparing a recombinant, tetrameric, mature human manganese superoxide dismutase (hMnSOD) which has no methionine at the N-terminus, characterised in that

a) an mRNA is isolated from human tissue or tissue culture cells and the poly(A)$^+$-RNA is prepared,
b) from this poly(A)$^+$-RNA a double-stranded cDNA is synthesised and from this a cDNA bank is constructed,
c) from said cDNA bank a total or partial DNA sequence coding for hMnSOD is identified and isolated by means of at least one DNA probe derived from the amino acid sequence of the hMnSOD,
d) this DNA sequence is optionally completed up to the start or stop codon,
e) an amino-terminal leader sequence, preferably one which is specific to microorganisms, is positioned directly after the start codon (ATG) of the hMnSOD gene and in front of the first codon coding for hMnSOD,
f) using this complete DNA sequence equipped with a leader sequence and coding for hMnSOD, a suitable expression cassette is constructed,
g) this expression cassette is incorporated in an expression plasmid, preferably an expression vector or expression plasmid suitable for eukaryotic cells,
h) using this expression plasmid, which contains the hMnSOD gene equipped with a leader sequence, suitable host cells, preferably eukaryotic host cells, are transformed, and
i) the hMnSOD synthesised and correctly processed by this transformed host is extracted from mitochondria and then purified.

14. Process according to claim 14, characterised in that the derived DNA probe is selected from formulae Va and Vb.

15. Process according to claims 13 and 14, characterised in that the aminoterminal leader sequence is a yeast-specific leader sequence.

16. Process according to claims 13 to 15, characterised in that the aminoterminal leader sequence is a mitochondrial leader sequence.

17. Process according to claims 13 to 16, characterised in that the expression cassette consists of a promoter, initiation signal, hMnSOD gene with aminoterminal leader sequence, stop codon and terminator.

18. Process according to claims 1 to 17, characterised in that the expression plasmid or the expression vector is a yeast vector.

19. Process according to claims 13 to 18, characterised in that the transformed host cell is a yeast cell.

20. Process according to one of claims 1 to 19, characterised in that the vectors and plasmids to be used carry an expression cassette containing DNA sequences coding for hMnSOD, are capable of stably transforming prokaryotic and eukaryotic host cells, are replicable in one of these host cells and the genetic information contained therein for hMnSOD is correctly transcribed and translated, the expression cassette containing the components promoter, initiation codon, aminoterminal leader sequence, hMnSOD structural gene, stop codon and optionally terminator in the correct orientation relative to the reading direction.

21. Process according to one of claims 1 to 20, characterised in that the DNA coding for hMnSOD is selected from formulae IIIa and IIIb, so as to prepare a correctly processed, tetrameric, mature hMnSOD according to formulae IVa and IVb.

## Revendications

1. Procédé de préparation d'une manganèse superoxyde dismutase humaine (hMnSOD) mature, tétramérique, recombinée, qui ne présente pas de méthionine à l'extrémité N-terminale, caractérisé en ce que

   a) un ADN à fonction de codage de la hMnSOD, ou des séquences partielles de celui-ci, est (sont) muni(es) d'un ADN codant une séquence signal aminoterminale qui est positionné entre l'ATG d'initiation et le premier codon codant la hMnSOD,

   b) un plasmide d'expression convenant pour l'expression de la hMnSOD est construit avec cette unité obtenue dans l'étape a),

   c) une cellule hôte appropriée est transformée avec le plasmide d'expression obtenu dans l'étape b), de sorte que celle-ci mature et accumule de manière intracellulaire la hMnSOD voulue en l'enzyme tétramérique et

   d) la hMnSOD tétramérique synthétisée et maturée correctement par la cellule hôte transformée est isolée et purifiée.

2. Procédé selon la revendication 1, caractérisé en ce que l'ADN codant une hMnSOD s'hybride avec une séquence d'ADN selon les formules Ia, Ib, II, IIIa, IIIb, Va, Vb, VIa, VIb, VIIA, VIIb ou IX dans des conditions strictes, ces séquences d'ADN pouvant être d'origine synthétique, semisynthétique Ou naturelle.

3. Procédé selon la revendication 2, caractérisé en ce que la séquence d'ADN codant une hMnSOD, ou des parties de celle-ci, est (sont) choisie(s) parmi les formules Ia, Ib, II, IIIa, IIIb, Va, Vb, VIa, VIb, VIIa, VIIb ou IX ou parmi des variantes dégénérées de celles-ci ou des mutations de ces séquences à fonction de codage de la hMnSOD.

4. Procédé selon l'une des revendications 1 à 3, caractérisé en ce que la séquence signal est une séquence signal spécifique de micro-organismes.

5. Procédé selon la revendication 4, caractérisé en ce que la séquence signal est une séquence signal spécifique des levures.

6. Procédé selon les revendications 1 à 4, caractérisé en ce que la séquence signal est une séquence signal bactérienne.

7. Procédé selon les revendications 1 à 5, caractérisé en ce que la séquence signal est une séquence signal mitochondriale.

8. Procédé selon la revendication 7, caractérisé en ce que la séquence signal mitochondriale est spécifique des levures.

9. Procédé selon l'une des revendications 1 à 8, caractérisé en ce que le plasmide d'expression consiste essentiellement en les composants promoteur, signal d'initiation, gène de la hMnSOD muni d'une séquence signal, codon d'arrêt, terminateur.

10. Procédé selon l'une des revendications 1 à 8, caractérisé en ce que la cellule hôte transformée est une cellule hôte procaryotique ou eucaryotique.

**11.** Procédé selon la revendication 10, caractérisé en ce que la cellule hôte transformée est une cellule de levure.

**12.** Procédé selon l'une des revendications 1 à 11, caractérisé en ce que la hMnSOD voulue est sécrétée dans le milieu de culture ou dans l'espace périplasmique ou dans la mitochondrie, la hMnSOD voulue étant maturée correctement en la molécule tétramérique.

**13.** Procédé de préparation d'une manganèse superoxyde dismutase humaine (hMnSOD) mature, tétramérique recombinée qui ne présente pas de méthionine à l'extrémité N-terminale, caractérisé en ce que

a) un ARNm est isolé à partir de tissu humain ou de cellules de culture de tissu et l'ARN-poly(A)$^+$ est préparé,

b) à partir de cet ARN-poly(A)$^+$ un ADNc double brin est synthétisé et une banque d'ADNc est construite à partir de celui-ci,

c) à partir de ladite banque d'ADNc une séquence totale ou partielle d'ADN codant la hMnSOD est identifiée et isolée au moyen d'au moins une sonde d'ADN dérivée de la séquence d'acides aminés de la hMnSOD,

d) cette séquence d'ADN est éventuellement complétée jusqu'au codon d'initiation ou d'arrêt,

e) une séquence signal aminoterminale, de préférence une séquence signal spécifique de micro-organismes, est positionnée juste après le codon d'initiation (ATG) du gène de la hMnSOD et avant le premier codon codant la hMnSOD,

f) une cassette d'expression appropriée est construite avec cette séquence d'ADN complète, munie d'une séquence signal, codant la hMnSOD,

g) cette cassette d'expression est incorporée dans un plasmide d'expression, de préférence dans un vecteur d'expression ou plasmide d'expression approprié pour les cellules eucaryotiques,

h) des cellules hôtes appropriées, de préférence des cellules hôtes eucaryotiques, sont transformées avec ce plasmide d'expression qui contient le gène de la hMnSOD muni d'une séquence signal, et

i) la hMnSOD synthétisée et maturée correctement par cet hôte transformé est extraite des mitochondries puis purifiée.

**14.** Procédé selon la revendication 13, caractérisé en ce que la sonde d'ADN dérivée est choisie parmi les formules Va et Vb.

**15.** Procédé selon les revendications 13 et 14, caractérisé en ce que la séquence signal aminoterminale est une séquence signal spécifique des levures.

**16.** Procédé selon les revendications 13 à 15, caractérisé en ce que la séquence signal aminoterminale est une séquence signal mitochondriale.

**17.** Procédé selon les revendications 13 à 16, caractérisé en ce que la cassette d'expression consiste en un promoteur, un signal d'initiation, un gène de la hMnSOD muni d'une séquence signal aminoterminale, un codon d'arrêt, un terminateur.

**18.** Procédé selon les revendications 1 à 17, caractérisé en ce que le plasmide d'expression ou le vecteur d'expression est un vecteur de levure.

**19.** Procédé selon les revendications 13 à 18, caractérisé en ce que la cellule hôte transformée est une cellule de levure.

**20.** Procédé selon l'une des revendications 1 à 19, caractérisé en ce que les vecteurs et plasmides à utiliser portent une cassette d'expression contenant des séquences d'ADN codant la hMnSOD, peuvent transformer de manière stable des cellules hôtes procaryotiques ou eucaryotiques, sont réplicables dans l'une de ces cellules hôtes et l'information génétique pour la hMnSOD qui y est contenue est transcrite et traduite correctement, la cassette d'expression contenant les composants promoteur, codon d'initiation, séquence signal aminoterminale, gène de structure de la hMnSOD, codon d'arrêt et éventuellement terminateur dans l'orientation correcte par rapport au sens de lecture.

21. Procédé selon l'une des revendications 1 à 20, caractérisé en ce que l'ADN codant la hMnSOD est choisi parmi les formules IIIa et IIIb de sorte qu'une hMnSOD mature, tétramérique, maturée correctement, selon les formules IVa et IVb, est préparée.

GAATTCGCATGGTCGACTAC

```
      I   M   Q   L   H   H   S   K   H   H   A   A   Y
GATCATGCAGCTGCACCACAGCAAGCACCACGCGGCCTAC

      V   N   N   L   N   V   T   E   E   K   Y   Q   E   A
GTGAACAACCTGAACGTCACCGAGGAGAAGTACCAGGAGGCG

      L   A   K   G   D   V   T   A   Q   I   A   L   Q   P   A   L
TTGGCCAAGGGAGATGTTACAGCCCAGATAGCTCTTCAGCCTGCACTG

      K   F   N   G   G   G   H   I   N   H   S   I   F   W   T   N
AAGTTCAATGGTGGTGGTCATATCAATCATAGCATTTTCTGGACAAAC

      L   S   P   N   G   G   G   E   P   K   G   E   L   L   E   A
CTCAGCCCTAACGGTGGTGGAGAACCCAAAGGGGAGTTGCTGGAAGCC

      I   K   R   D   F   G   S   F   D   K   F   K   E   K   L   T
ATCAAACGTGACTTTGGTTCCTTTGACAAGTTTAAGGAGAAGCTGACG

      A   A   S   V   G   V   Q   G   S   G   W   G   W   L   G   F
GCTGCATCTGTTGGTGTCCAAGGCTCAGGTTGGGGTTGGCTTGGTTTC

      N   K   E   R   G   H   L   Q   I   A   A   C   P   N   Q   D
AATAAGGAACGGGGACACTTACAAATTGCTGCTTGTCCAAATCAGGAT

      P   L   Q   G   T   T   G   L   I   P   L   L   G   I   D   V
CCACTGCAAGGAACAACAGGCCTTATTCCACTGCTGGGGATTGATGTG

      W   E   H   A   Y   Y   L   Q   Y   K   N   V   R   P   D   Y
TGGGAGCACGCTTACTACCTTCAGTATAAAAATGTCAGGCCTGATTAT

      L   K   A   I   W   N   V   I   N   W   E   N   V   T   E   R
CTAAAAGCTATTTGGAATGTAATCAACTGGGAGAATGTAACTGAAAGA

      Y   M   A   C   K   K   *
TACATGGCTTGCAAAAAGTAAACCACGATCGTTATGCTGAAAAAAAAA
```

AAAAAAAAAAAAAAAAAAAAAAAGTAGTCGACCATGCGAATTC

# Fig.1

EP 0 282 899 B1

2686
1

| | MS | |
| pUC18 |

pUC18:  -HindIII. SphI. PstI. HincII. XbaI. BamHI. SmaI. KpnI. SacI. EcoRI-

x HincII

XhoI-Linker

pES102  -HindIII. SphI. PstI. XhoI. XbaI. BamHI. SmaI. KpnI. SacI. EcoRI-

x SmaI

NcoI-Linker

V17:  -HindIII. SphI. PstI. XhoI. XbaI. BamHI. NcoI. KpnI. SacI. EcoRI-

x XhoI          x XbaI

OP1

HSOD2:  -HindIII. SphI. PstI. XhoI [OP1] XbaI. BamHI. NcoI. KpnI. SacI. EcoRI-

V17:  -HindIII. SphI. PstI. XhoI. XbaI. BamHI. NcoI. KpnI. SacI. EcoRI-

x XbaI          x NcoI

OP2

HSOD3:  -HindIII. SphI. PstI. XhoI. XbaI [OP2] NcoI. KpnI. SacI. EcoRI-

HSOD4:  -HindIII. SphI. PstI. XhoI [OP1] XbaI [OP2] NcoI. KpnI. SacI. EcoRI-

Fig.2

48

Fig. 3

Fig. 4

Fig. 5

Fig. 6

Fig.7

Fig.8

Fig. 9

Fig.10

Fig. 11

a b c d e

←hMn-SOD

Fig.12

a b c d e    f g h i j k l m n o

hMn-
SOD

Fig.13

Fig.14

Fig.15